# EUROPEAN PATENT SPECIFICATION

(11) **EP 2 391 722 B1**
(45) Date of publication and mention of the grant of the patent: **11.06.2014**
(21) Application number: 10704194.9
(22) Date of filing: 02.02.2010
(51) Int. Cl.: C12N 15/861, A61K 35/76

(54) **NON-AD5 ADENOVIRAL VECTORS AND METHODS AND USES RELATED THERETO**
NICHT-AD5-ADENOVIRUSVEKTOREN SOWIE VERFAHREN UND VERWENDUNGEN IN VERBINDUNG DAMIT
VECTEURS ADÉNOVIRAUX NON AD5 ET PROCÉDÉS ET UTILISATIONS ASSOCIÉS

(30) Priority: 02.02.2009 FI 20090030; 02.07.2009 FI 20095751
(43) Date of publication of application: 07.12.2011
(73) Proprietor: Oncos Therapeutics Oy, 00180 Helsinki (FI)
(72) Inventor: HEMMINKI, Otto, FI-00270 Helsinki (FI); BAUERSCHMITZ, Gerd, 50935 Köln (DE); CERULLO, Vincenzo, FI-00330 Helsinki (FI); PESONEN, Sari, FI-00570 Helsinki (FI); HEMMINKI, Akseli, FI-00260 Helsinki (FI)
(74) Representative: Kolster Oy Ab
(86) International application number: PCT/IB2010/050452
(87) International publication number: WO 2010/086838

(56) References cited:
- WO-A1-2005/030261
- WO-A2-2005/010149
- US-A1- 2005 214 923
- SHEN B ET AL: "The effect of hypoxia on the uptake, replication and lytic potential of group B adenovirus type 3 (Ad3) and type 11p (Ad11p)" GENE THERAPY, vol. 13, no. 12, June 2006 (2006-06), pages 986-990, XP002582335 ISSN: 0969-7128 cited in the application
- LEI N ET AL: "An oncolytic adenovirus expressing granulocyte macrophage colony-stimulating factor shows improved specificity and efficacy for treating human solid tumors" CANCER GENE THERAPY,, vol. 16, no. 1, 1 August 2008 (2008-08-01) , pages 33-43, XP002571569
- RAJECKI ET AL: "betahCG Expressing CRAd for Prostate Cancer" MOLECULAR THERAPY, ACADEMIC PRESS, SAN DIEGO, CA, US LNKD- DOI:10.1016/J.YMTHE.2005.10.009, vol. 13, 1 January 2006 (2006-01-01), pages S366-S367, XP005676056 ISSN: 1525-0016
- BARTON K N ET AL: "Polyethylene Glycol (Molecular Weight 400 DA) Vehicle Improves Gene Expression of Adenovirus Mediated Gene Therapy" JOURNAL OF UROLOGY, LIPPINCOTT WILLIAMS & WILKINS, BALTIMORE, MD, US LNKD- DOI:10.1016/S0022-5347(05)00918-3, vol. 175, no. 5, 1 May 2006 (2006-05-01), pages 1921-1925, XP024990991 ISSN: 0022-5347 [retrieved on 2006-05-01]

### SEQUENCE LISTING

<110> Oncos Therapeutics
<120> Non-Ad5 adenoviral vectors and methods and uses related thereto
<130> 2090771PC
<160> 21
<170> PatentIn version 3.5
<210> 1
   <211> 29
   <212> DNA
   <213> Artificial
<220>
   <223> primer
<400> 1
   catggtaccc aagtgtgtcg ctgtcgagt 29
<210> 2
   <211> 29
   <212> DNA
   <213> Artificial
<220>
   <223> primer
<400> 2
   catgatatca gcgatcagct gacacctac 29
<210> 3
   <211> 29
   <212> DNA
   <213> Artificial
<220>
   <223> primer
<400> 3
   catgatatcg tgccagcgag aagagtttt 29
<210> 4
   <211> 30
   <212> DNA
   <213> Artificial
<220>
   <223> primer
<400> 4
   cattctagag cgagcacaat agttctttca 30
<210> 5
   <211> 20
   <212> DNA
   <213> Artificial
<220>
   <223> primer
<400> 5
   ggttatgcca gggtggagta 20
<210> 6
   <211> 18
   <212> DNA
   <213> Artificial
<220>
   <223> primer
<400> 6
   aaggtgaagg ggcaggac 18
<210> 7
   <211> 18
   <212> DNA
   <213> Artificial
<220>
   <223> primer
<400> 7
   agcccctccc cttccttt 18
<210> 8
   <211> 20
   <212> DNA
   <213> Artificial
<220>
   <223> primer
<400> 8
   cccggtctca ctggagataa 20
<210> 9
   <211> 719
   <212> DNA
   <213> Artificial
<220>
   <223> Ad3-hTERT-E1
<400> 9
   ggcggatgca agtgaaaatt ctccattttc gcgcgaaaac taaatgagga agtgaatttc 60
   tgagtcattt cgcggttatg ccagggtgga gtatttgccg agggccgagt agactttgac 120
   cgtttacgtg gaggtttcga ttaccgtgtt tttccctaaa tttccgcgta cggtgtcaaa 180
   gttctgtgtt tttacgtagg tgtcagctga tcgctgatcg ctgcgctgtc ggggccaggc 240
   cgggctccca gtggattcgc gggcacagac gcccaggacc gcgcttccca cgtggcggag 300
   ggactgggga cccgggcacc cgtcctgccc cttcaccttc cagctccgcc tcctccgcgc 360
   ggaccccgcc ccgtcccgac ccctcccggg tccccggccc agccccctcc gggccctccc 420
   agcccctccc cttcctttcc gcggccccgc cctctcctcg cggcgcgagt ttcaggcagc 480
   gctgcgtcct gctgcgcacg tgggaagccc tggccgggca tcgtgccagc gagaagagtt 540
   ttctcctccg cgccgcaagt cagttctgcg ctttgaaaat gagacacctg cgcttcctgc 600
   cacaggaggt tatctccagt gagaccggga tcgaaatact ggagtttgtg gtaaataccc 660
   taatgggaga cgacccggaa ccgccagtgc agcctttcga tccacctacg ctgcacgat 719
<210> 10
   <211> 20
   <212> DNA
   <213> Artificial
<220>
   <223> primer
<400> 10
   cttctgaact cggtcctcac 20
<210> 11
   <211> 20
   <212> DNA
   <213> Artificial
<220>
   <223> primer
<400> 11
   tccagaatgt atagcggctc 20
<210> 12
   <211> 19
   <212> DNA
   <213> Artificial
<220>
   <223> primer
<400> 12
   cgaggcccag agcaagaca 19
<210> 13
   <211> 23
<212> DNA
   <213> Artificial
<220>
   <223> primer
<400> 13
   cacagcttct ccttaatgtc acg 23
<210> 14
   <211> 38084
   <212> DNA
   <213> Artificial Sequence
<220>
   <223> Ad3-hTERT-hNIS-E1A
<400> 14
   aaatctatct atataatata ccttatagat ggaatggtgc caacatgtaa atgaggtaat 60
   ttaaaaaagt gcgcgctgtg tggtgattgg ctgcggggtt aacggctaaa aggggcggcg 120
   cgaccgtggg aaaatgacgt gacttatgtg ggaggagtta tgttgcaagt tattacggta 180
   aatgtgacgt aaaacgaggt gtggtttgaa cacggaagta gacagttttc ccacgcttac 240
   tgacaggata tgaggtagtt ttgggcggat gcaagtgaaa attctccatt ttcgcgcgaa 300
   aactaaatga ggaagtgaat ttctgagtca tttcgcggtt atgccagggt ggagtatttg 360
   ccgagggccg agtagacttt gaccgtttac gtggaggttt cgattaccgt gtttttcacc 420
   taaatttccg cgtacggtgt caaagttctg tgtttttacg taggtgtcag ctgatcgctg 480
   atcggacgtc ctgcgctgtc ggggccaggc cgggctccca gtggattcgc gggcacagac 540
   gcccaggacc gcgcttccca cgtggcggag ggactgggga cccgggcacc cgtcctgccc 600
   cttcaccttc cagctccgcc tcctccgcgc ggaccccgcc ccgtcccgac ccctcccggg 660
   tccccggccc agccccctcc gggccctccc agcccctccc cttcctttcc gcggccccgc 720
   cctctcctcg cggcgcgagt ttcaggcagc gctgcgtcct gctgcgcacg tgggaagccc 780
   tggcccaatt gatggaggcc gtggagaccg gggaacggcc caccttcgga gcctgggact 840
   acggggtctt tgccctcatg ctcctggtgt ccactggcat cgggctgtgg gtcgggctgg 900
   ctcggggcgg gcagcgcagc gctgaggact tcttcaccgg gggccggcgc ctggcggccc 960
   tgcccgtggg cctgtcgctg tctgccagct tcatgtcggc cgtgcaggtg ctgggcgtgc 1020
   cgtcggaggc ctatcgctat ggcctcaagt tcctctggat gtgcctgggc cagcttctga 1080
   actcggtcct caccgccctg ctcttcatgc ccgtcttcta ccgcctgggc ctcaccagca 1140
   cctacgagta cctggagatg cgcttcagcc gcgcagtgcg gctctgcggg actttgcagt 1200
   acattgtagc cacgatgctg tacaccggca tcgtaatcta cgcaccggcc ctcatcctga 1260
   accaagtgac cgggctggac atctgggcgt cgctcctgtc caccggaatt atctgcacct 1320
   tctacacggc tgtgggcggc atgaaggctg tggtctggac tgatgtgttc caggtcgtgg 1380
   tgatgctaag tggcttctgg gttgtcctgg cacgcggtgt catgcttgtg ggcgggcccc 1440
   gccaggtgct cacgctggcc cagaaccact cccggatcaa cctcatggac tttaaccctg 1500
   acccgaggag ccgctataca ttctggactt ttgtggtggg tggcacgttg gtgtggctct 1560
   ccatgtatgg cgtgaaccag gcgcaggtgc agcgctacgt ggcttgccgc acagagaagc 1620
   aggccaagct ggccctgctc atcaaccagg tcggcctgtt cctgatcgtg tccagcgctg 1680
   cctgctgtgg catcgtcatg tttgtgttct acactgactg cgaccctctc ctcctggggc 1740
   gcatctctgc cccagaccag tacatgcctc tgctggtgct ggacatcttc gaagatctgc 1800
   ctggagtccc cgggcttttc ctggcctgtg cttacagtgg caccctcagc acagcatcca 1860
   ccagcatcaa tgctatggct gcagtcactg tagaagacct catcaaacct cggctgcgga 1920
   gcctggcacc caggaaactc gtgattatct ccaaggggct ctcactcatc tacggatcgg 1980
   cctgtctcac cgtggcagcc ctgtcctcac tgctcggagg aggtgtcctt cagggctcct 2040
   tcaccgtcat gggagtcatc agcggccccc tgctgggagc cttcatcttg ggaatgttcc 2100
   tgccggcctg caacacaccg ggcgtcctcg cgggactagg cgcgggcttg gcgctgtcgc 2160
   tgtgggtggc cttgggcgcc acgctgtacc cacccagcga gcagaccatg agggtcctgc 2220
   catcgtcggc tgcccgctgc gtggctctct cagtcaacgc ctctggcctc ctggacccgg 2280
   ctctcctccc tgctaacgac tccagcaggg cccccagctc aggaatggac gccagccgac 2340
   ccgccttagc tgacagcttc tatgccatct cctatctcta ttacggtgcc ctgggcacgc 2400
   tgaccactgt gctgtgcgga gccctcatca gctgcctgac aggccccacc aagcgcagca 2460
   ccctggcccc gggattgttg tggtgggacc tcgcacggca gacagcatca gtggccccca 2520
   aggaagaagt ggccatcctg gatgacaact tggtcaaggg tcctgaagaa ctccccactg 2580
   gaaacaagaa gccccctggc ttcctgccca ccaatgagga tcgtctgttt ttcttggggc 2640
   agaaggagct ggagggggct ggctcttgga ccccctgtgt tggacatgat ggtggtcgag 2700
   accagcagga gacaaacctc tgaattcgcc cctctccctc ccccccccct aacgttactg 2760
   gccgaagccg cttggaataa ggccggtgtg cgtttgtcta tatgttattt tccaccatat 2820
   tgccgtcttt tggcaatgtg agggcccgga aacctggccc tgtcttcttg acgagcattc 2880
   ctaggggtct ttcccctctc gccaaaggaa tgcaaggtct gttgaatgtc gtgaaggaag 2940
   cagttcctct ggaagcttct tgaagacaaa caacgtctgt agcgaccctt tgcaggcagc 3000
   ggaacccccc acctggcgac aggtgcctct gcggccaaaa gccacgtgta taagatacac 3060
   ctgcaaaggc ggcacaaccc cagtgccacg ttgtgagttg gatagttgtg gaaagagtca 3120
   aatggctctc ctcaagcgta ttcaacaagg ggctgaagga tgcccagaag gtaccccatt 3180
   gtatgggatc tgatctgggg cctcggtgca catgctttac atgtgtttag tcgaggttaa 3240
   aaaaacgtct aggccccccg aaccacgggg acgtggtttt cctttgaaaa acacgatgat 3300
   aatatggcca caattaatga tgggctcgaa tcgtgccagc gagaagagtt ttctcctccg 3360
   cgccgcaagt cagttctgcg ctttgaaaat gagacacctg cgcttcctgc cacaggaggt 3420
   tatctccagt gagaccggga tcgaaatact ggagtttgtg gtaaataccc taatgggaga 3480
   cgacccggaa ccgccagtgc agcctttcga tccacctacg ctgcacgatc tgtatgattt 3540
   agagatagac gggccggagg atcccaatga ggaagctgtg aatgggtttt ttactgattc 3600
   tatgctgcta gctgctgatg aaggattgga cataaaccct cctcctgaga cacttgttac 3660
   cccaggggtg gttgtggaaa gcggcatagg tgggaaaaaa ttgcctgatc tgggagcagc 3720
   tgaaatggac ttgcgttgtt atgaagaggg ttttcctccc agtgatgatg aagatgggga 3780
   aactgagcag tccatccata ccgcagtaaa tgagggagta aaagctgcca gcgatgtttt 3840
   taagttggac tgtccggagc tgcctggaca tggctgtaag tcttgtgaat ttcacaggaa 3900
   taacactgga atgaaagaac tattgtgctc gctttgctat atgagaatgc actgccactt 3960
   tatttacagt aagtgtattt aagtgaaatt taaaggaata gtgtagctat ttaataactg 4020
   ttgaatggta gatttatgtt tttttcttgc gattttttgt aggtcctgtg tctgatgatg 4080
   agtcaccttc tcctgattca actacctcac ctcctgaaat tcaggcgccc gcacctgcaa 4140
   acgtatgcaa gcccattcct gtgaagccta agcctgggaa acgccctgct gtggataagc 4200
   ttgaggactt gttggagggt ggggatggac ctttggacct tagtacccgg aaactgccaa 4260
   ggcaatgagt gccctgcagc tgtgtttatt taatgtgacg tcatgtaata aaattatgtc 4320
   agctgctgag tgttttatta cttcttgggt ggggacttgg atatataagt aggagcagat 4380
   ctgtgtggtt agctcacagc aacctgctgc catccatgga ggtttgggct atcttggaag 4440
   acctcagaca gactaagcta ctgctagaaa acgcctcgga cggagtctct ggcctttgga 4500
   gattctggtt cggtggtgat ctagctaggc tagtgtttag gataaaacag gactacaggg 4560
   aagaatttga aaagttattg gacgatagtc cgggactttt tgaagctctt aacttgggtc 4620
   atcaggctca ttttaaggag aaggttttat cagttttaga tttttctact cctggtagaa 4680
   ctgctgctgc tgtagctttt cttactttta tattggataa atggatccgc caaactcact 4740
   tcagcaaggg atacgttttg gatttcatag cagcagcttt gtggagaaca tggaaggctc 4800
   gcaggatgag gacaatctta gattactggc cagtgcagcc tctgggagta gcagggatac 4860
   tgagacaccc accgaccatg ccagcggttc tgcaggagga gcagcaggag gacaatccga 4920
   gagccggcct ggaccctccg gtggaggagt agctgacctg tttcctgaac tgcgacgggt 4980
   gcttactagg tctacgacca gtggacagaa caggggaatt aagagggaga ggaatcctag 5040
   tgggaataat tcaagaaccg agttggcttt aagtttaatg agccgcaggc gtcctgaaac 5100
   tgtttggtgg catgaggttc agagcgaagg cagggatgaa gtttcaatat tgcaggagaa 5160
   atattcacta gaacaactta agacctgttg gttggaacct gaggatgatt gggaggtggc 5220
   cattaggaat tatgctaaga tatctctgag gcctgataaa caatatagaa ttactaagaa 5280
   gattaatatt agaaatgcat gctacatatc agggaatggg gcagaggtta taatagatac 5340
   acaagataaa gcagttttta gatgttgtat gatgggtatg tggccagggg ttgtcggcat 5400
   ggaagcagta acacttatga atattaggtt taaaggggat gggtataatg gcattgtatt 5460
   tatggctaac actaagctga ttctacatgg ttgtagcttt tttgggttta ataatacgtg 5520
   tgtagaagct tgggggcaag ttagtgtgag gggttgtagt ttttatgcat gctggattgc 5580
   aacatcaggt agggtcaaga gtcagttgtc tgtgaagaaa tgcatgtttg agagatgtaa 5640
   tcttggcata ctgaatgaag gtgaagcaag ggtccgccac tgcgcagcta cagaaactgg 5700
   ctgcttcatt ctaataaagg gaaatgccag tgtgaagcat aatatgatct gtggacattc 5760
   ggatgagagg ccttatcaga tgctgacctg cgctggtgga cattgcaata ttcttgctac 5820
   cgtgcatatc gtttcacatg cacgcaaaaa atggcctgta tttgaacata atgtgattac 5880
   caagtgcacc atgcacatag gtggtcgcag gggaatgttt atgccttacc agtgtaacat 5940
   gaatcatgtg aaggtaatgt tggaaccaga tgccttttcc agagtgagct taacaggaat 6000
   ctttgatatg aatattcaac tatggaagat cctgagatat gatgacacta aaccaagggt 6060
   gcgcgcatgc gaatgcggag gcaagcatgc tagattccag ccggtgtgcg tggatgtgac 6120
   tgaagacttg agacccgatc atttggtgct tgcctgcact ggagcggagt tcggttctag 6180
   tggtgaagaa actgactaaa gtgagtagtg gggcaagatg tggatgggga ctttcaggtt 6240
   ggtaaggtgg gcagattggg taaattttgt taatttctgt cttgcagctg ccatgagtgg 6300
   aagcgcttct tttgaggggg gagtatttag cccttatctg acgggcaggc tcccaccatg 6360
   ggcaggagtt cgtcagaatg tcatgggatc cactgtggat gggagacccg tccagcccgc 6420
   caattcctca acgctgacct atgccacttt gagttcgtca ccattggatg cagctgcagc 6480
   cgccgccgct actgctgccg ccaacaccat ccttggaatg ggctattatg gaagcatcgt 6540
   tgccaattcc agttcctcta ataacccttc aaccctggct gaggacaagc tacttgttct 6600
   cttggcgcag ctcgaggcct taacccaacg cttaggcgaa ctgtctaagc aggtggccca 6660
   gttgcgtgag caaactgagt ctgctgttgc cacagcaaag tctaaataaa gatctcaaat 6720
   caataaataa agaaatactt gatataaaac aaatgaatgt ttatttgatt tttcgcgcgc 6780
   ggtatgccct ggaccatcgg tctcgatcat tgagaacgcg gtggatcttt tccagtaccc 6840
   tgtaaaggtg ggattgaatg tttagataca tgggcattag tccgtctcgg gggtggagat 6900
   agctccattg aagagcctct tgctccgggg tagtgttata aatcacccag tcatagcaag 6960
   gtcggagtgc atggtgttgc acaatatctt ttaggagcag actaattgca acggggaggc 7020
   ccttagtgta ggtgtttaca aatctgttga gctgggacgg gtgcatccgg ggtgaaatta 7080
   tatgcatttt ggactggatc ttgaggttgg caatgttgcc gcctagatcc cgtctcgggt 7140
   tcatattgtg caggaccacc aagacagtgt atccggtgca cttgggaaat ttatcatgca 7200
   gcttagaggg aaaagcatga aaaaatttgg agacgccttt gtgacccccc agattctcca 7260
   tgcactcatc cataatgata gcgatggggc cgtgggcagc ggcacgggcg aacacgttcc 7320
   gggggtctga aacatcatag ttatgctcct gagtcaggtc atcataagcc attttaataa 7380
   actttgggcg gagggtgcca gattggggga tgaaagttcc ctctggcccg ggagcatagt 7440
   ttccctcaca tatttgcatt tcccaggctt tcagttcaga gggggggatc atgtccacct 7500
   gcggggctat aaaaaatacc gtttctggag ccggggtgat taactgggac gagagcaaat 7560
   tcctaagcag ctgagacttg ccgcacccag tgggaccgta aatgacccca attacgggtt 7620
   gcagatggta gtttagggaa cgacagctgc cgtcctcccg gagcaggggg gccacttcgt 7680
   tcatcatttc ccttacatgg atattttccc gcaccaagtc cgttaggagg cgctctcccc 7740
   caagggatag aagctcctgg agcgaggaga agtttttcag cggcttcagc ccgtcagcca 7800
   tgggcatttt ggaaagagtc tgttgcaaga gctcgagccg gtcccagagc tcggtgatgt 7860
   gctctatggc atctcgatcc agcagacctc ctcgtttcgc gggttgggac ggctcctgga 7920
   gtagggaatc agacgatggg cgtccagcgc tgccagggtc cgatccttcc atggtcgcag 7980
   cgtccgagtc agggttgttt ccgtcacggt gaaggggtgc gcgcctggtt gggcgcttgc 8040
   gagggtgcgc ttcagactca tcctgctggt cgagaaccgc tgccgattgg cgccctgcat 8100
   gtcggccagg tagcagttta ccatgagttc gtagttgagc gcctcggccg cgtggccttt 8160
   ggcacggagc ttacctttgg aagttttatg gcaggcgggg cagtagatac atttgagggc 8220
   atacaacttg ggcgcgagga aaatggattc gggggagtat gcatccgcac cgcaggaggc 8280
   gcagacggtt tcgcactcca cgagccaggt cagatccggc tcatcggggt caaaaacaag 8340
   ttttccgcca tgttttttga tgcgtttctt acctttggtt tccatgagtt cgtgtccccg 8400
   ctgggtgaca aagaggctgt ccgtgtcccc gtagaccgac tttatgggcc tgtcctcgag 8460
   cggagtgcct cggtcctctt cgtagaggaa cccagcccac tctgatacaa aagcgcgtgt 8520
   ccaggccagc acaaaggagg ccacgtggga ggggtagcgg tcgttgtcaa ccagggggtc 8580
   caccttctct acggtatgta aacacatgtc cccctcctcc acatccaaga atgtgattgg 8640
   cttgtaagtg taggccacgt gaccaggggt ccccgccggg ggggtataaa agggggcggg 8700
   cctctgttcg tcctcactgt cttccggatc gctgtccagg agcgccagct gttggggtag 8760
   gtattccctc tcgaaggcgg gcatgacctc tgcactcagg ttgtcagttt ctaggaacga 8820
   ggaggatttg atattgacag taccagcaga gatgcctttc ataagactct cgtccatctg 8880
   gtcagaaaac acaatcttct tgttgtccag cttggtggca aatgatccat agagggcatt 8940
   ggatagaagc ttggcgatgg agcgcatggt ttggttcttt tccttgtccg cgcgctcctt 9000
   ggcggtgatg ttaagctgga cgtactcgcg cgccacacat ttccattcag gaaagatggt 9060
   tgtcagttca tccggaacta ttctgattcg ccatccccta ttgtgcaggg ttatcagatc 9120
   cacactggtg gccacctcgc ctcggagggg ctcattggtc cagcagagtc gacctccttt 9180
   tcttgaacag aaagggggga gggggtctag catgaactca tcaggggggt ccgcatctat 9240
   ggtaaatatt cccggtagca aatctttgtc aaaatagctg atggtggcgg gatcatccaa 9300
   ggtcatctgc cattctcgaa ctgccagcgc gcgctcatag gggttaagag gggtgcccca 9360
   gggcatgggg tgggtgagcg cggaggcata catgccacag atatcgtaga catagagggg 9420
   ctcttcgagg atgccgatgt aagtgggata acatcgcccc cctctgatgc ttgctcgcac 9480
   atagtcatag agttcatgtg agggggcaag aagacccggg cccagattgg tgcggttggg 9540
   tttttccgcc ctgtaaacga tctggcgaaa gatggcatgg gaattggaag agatagtagg 9600
   tctctggaat atgttaaaat gggcatgagg taagcctaca gagtccctta tgaagtgggc 9660
   atatgactct tgcagcttgg ctaccagctc ggcggtgatg agtacatcca gggcacagta 9720
   gtcgagagtt tcctggatga tgtcataacg cggttggctt ttcttttccc acagctcgcg 9780
   gttgagaagg tattcttcgt gatccttcca gtactcttcg aggggaaacc cgtctttttc 9840
   tgcacggtaa gagcccaaca tgtagaactg attgactgcc ttgtagggac agcatccctt 9900
   ctccactggg agagagtatg cttgggctgc attgcgcagc gaggtatgag tgagggcaaa 9960
   agtgtccctg accatgactt tgaggaattg atacttgaag tcgatgtcat cacaggcccc 10020
   ctgttcccag agttggaagt ccacccgctt cttgtaggcg gggttgggca aagcgaaagt 10080
   aacatcattg aagaggatct tgccggccct gggcatgaaa tttcgggtga ttttgaaagg 10140
   ctgaggaacc tctgctcggt tattgataac ctgagcggcc aagacgatct catcaaagcc 10200
   attgatgttg tgccccacta tgtacagttc taagaatcga ggggtgcccc tgacatgagg 10260
   cagcttcttg agttcttcaa aagtgagatc tgtagggtca gtgagagcat agtgttcgag 10320
   ggcccattcg tgcacgtgag ggttcgcttt aaggaaggag gaccagaggt ccactgccag 10380
   tgctgtttgt aactggtccc ggtactgacg aaaatgctgt ccgactgcca tcttttctgg 10440
   ggtgacgcaa tagaaggttt gggggtcctg ccgccagcga tcccacttga gttttatggc 10500
   gaggtcatag gcgatgttga cgagccgctg gtctccagag agtttcatga ccagcatgaa 10560
   ggggattagc tgcttgccaa aggaccccat ccaggtgtag gtttccacat cgtaggtgag 10620
   aaagagcctt tctgtgcgag gatgagagcc aatcgggaag aactggatct cctgccacca 10680
   gttggaggaa tggctgttga tgtgatggaa gtagaactcc ctgcgacgcg ccgagcattc 10740
   atgcttgtgc ttgtacagac ggccgcagta ctcgcagcga ttcacgggat gcaccttatg 10800
   aatgagttgt acctgacttc ctttgacgag aaatttcagt ggaaaattga ggcctggcgc 10860
   ttgtacctcg cgctttacta tgttgtctgc atcggcatga ccatcttctg tctcgatggt 10920
   ggtcatgctg acgagccctc gcgggaggca agtccagacc tcggcgcggc aggggcggag 10980
   ctcgaggacg agagcgcgca ggccggagct gtccagggtc ctgagacgct gcggagtcag 11040
   gttagtaggc agtgtcagga gattaacttg catgatcttt tggagggcgt gagggaggtt 11100
   cagatagtac ttgatctcaa cgggtccgtt ggtggagatg tcgatggctt gcagggttcc 11160
   gtgtcccttg ggcgctacca ccgtgccctt gtttttcatt ttggacggcg gtggctctgt 11220
   tgcttcttgc atgtttagaa gcggtgtcga gggcgcgcac cgggcggcag gggcggctcg 11280
   ggacccggcg gcatggctgg cagtggtacg tcggcgccgc gcgcgggtag gttctggtac 11340
   tgcgccctga gaagactcgc atgcgcgacg acgcggcggt tgacatcctg gatctgacgc 11400
   ctctgggtga aagctaccgg ccccgtgagc ttgaacctga aagagagttc aacagaatca 11460
   atctcggtat cgttgacggc ggcttgccta aggatttctt gcacgtcgcc agagttgtcc 11520
   tggtaggcga tctcggccat gaactgctcg atctcttcct cttgaagatc tccgcggccc 11580
   gctctctcga cggtggccgc gaggtcgttg gagatgcgcc caatgagttg agagaatgca 11640
   ttcatgcccg cctcgttcca gacgcggctg tagaccacag cccccacggg atctctcgcg 11700
   cgcatgacca cctgggcgag gttgagctcc acgtggcggg tgaagaccgc atagttgcat 11760
   aggcgctgga aaaggtagtt gagtgtggtg gcgatgtgct cggtgacgaa gaaatacatg 11820
   atccatcgtc tcagcggcat ctcgctgaca tcgcccagcg cttccaagcg ctccatggcc 11880
   tcgtagaagt ccacggcaaa gttaaaaaac tgggagttac gcgcggacac ggtcaactcc 11940
   tcttccagaa gacggataag ttcggcgatg gtggtgcgca cctcgcgctc gaaagcccct 12000
   gggatttctt cctcaatctc ttcttcttcc actaacatct cttcctcttc aggtggggct 12060
   gcaggaggag ggggaacgcg gcgacgccgg cggcgcacgg gcagacggtc gatgaatctt 12120
   tcaatgacct ctccgcggcg gcggcgcatg gtctcggtga cggcacgacc gttctccctg 12180
   ggtctcagag tgaagacgcc tccgcgcatc tccctgaagt ggtgactggg aggctctccg 12240
   ttgggcaggg acaccgcgct gattatgcat tttatcaatt gccccgtagg tactccgcgc 12300
   aaggacctga tcgtctcaag atccacggga tctgaaaacc tttcgacgaa agcgtctaac 12360
   cagtcgcaat cgcaaggtag gctgagcact gtttcttgcg ggcgggggcg gctagacgct 12420
   cggtcggggt tctctctttc ttctccttcc tcctcttggg agggtgagac gatgctgctg 12480
   gtgatgaaat taaaataggc agttttgaga cggcggatgg tggcgaggag caccaggtct 12540
   ttgggtccgg cttgttggat acgcaggcga tgagccattc cccaagcatt atcctgacat 12600
   ctggccagat ctttatagta gtcttgcatg agtcgttcca cgggcacttc ttcttcgccc 12660
   gctctgccat gcatgcgagt gatcccgaac ccgcgcatgg gctggacaag tgccaggtcc 12720
   gctacaaccc tttcggcgag gatggcttgc tgcacctggg tgagggtggc ttggaagtcg 12780
   tcaaagtcca cgaagcggtg gtaggccccg gtgttgattg tgtaggagca gttggccatg 12840
   actgaccagt tgactgtctg gtgcccaggg cgcacgagct cggtgtactt gaggcgcgag 12900
   tatgcgcggg tgtcaaagat gtaatcgttg caggtgcgca ccaggtactg gtagccaatg 12960
   agaaagtgtg gcggtggctg gcggtacagg ggccatcgct ctgtagccgg ggctccgggg 13020
   gcgaggtctt ccagcatgag gcggtggtag ccgtagatgt acctggacat ccaggtgata 13080
   ccggaggcgg tggtggatgc acgtgggaac tcgcgcacgc ggttccagat gttgcgcagc 13140
   ggcatgaagt agttcatggt aggcacggtc tggccagtga ggcgcgcgca gtcattgacg 13200
   ctctgtagac acggagaaaa cgaaagcgat gagcggctcg actccgtggt ctgggggaac 13260
   gtgaacgggt tgggtcgcgg tgtaccccgg ttcgagtcca aagctaagcg atcacgctcg 13320
   gatcggccgg agccgcggct aacgtggtat tggctatccc gtctcgaccc agccgacgaa 13380
   tatccagggt acggagtaga gtcgtttttg ctgctttttc ctggacgtgt gccattgcca 13440
   cgtcaagctt tagaacgctc agttctcggg ccgtgagtgg ctcgcgcccg tagtctggag 13500
   aatcagtcgc cagggttgcg ttgcggtatg ccccggttgg agcctaagcg cggctcgtat 13560
   cggccggttt ccgcgacaag cgagggtttg gcagccccgt tatttccaag accccgccag 13620
   ccgacttctc cagtttacgg gagcgagccc tttttttttt ttttttgttt ttgtcgccca 13680
   gatgcatcca gtgctgcgac agatgcgccc ccagcaacag gccccttctc agcaacagcc 13740
   acaaaaggct cttcttgctc ctgcaactac tgcagctgca gccgtgagcg gcgcgggaca 13800
   gcccgcctat gatctggact tggaagaggg cgagggattg gcgcgcctgg gggctccatc 13860
   gcccgagcgg cacccgcggg tgcaactaaa aaaggactct cgcgaggcgt acgtgcccca 13920
   gcagaacctg ttcagggaca ggagcggcga ggagccagag gagatgcgag catctcgatt 13980
   taacgcgggt cgcgagctgc gccacggtct ggatcgaaga cgggtgctgc aagacgagga 14040
   ttttgaggtc gatgaagtca cagggatcag cccagctagg gcacatgtgg ccgcggccaa 14100
   cctagtctcg gcctacgagc agaccgtgaa ggaggagcgc aacttccaaa aatcttttaa 14160
   caaccatgtg cgcaccctga tcgcccgcga ggaagtgacc ctgggtctga tgcatctgtg 14220
   ggacctgatg gaggctatcg cccaaaaccc cactagcaaa ccactgacag ctcagctgtt 14280
   tctggtggtt caacatagca gggacaacga ggcattcagg gaggcgttgt tgaacatcac 14340
   cgagcctgat gggagatggc tgtatgatct gatcaacatc ctgcaaagta ttatagtgca 14400
   ggaacgtagc ctgggtttgg ctgagaaagt ggcagctatc aactactcgg tcttgagcct 14460
   gggcaaatac tacgctcgca agatctacaa gaccccctac gtacccatag acaaggaggt 14520
   gaagatagat gggttttaca tgcgcatgac tctgaaggtg ctgactctga gcgacgatct 14580
   gggggtgtat cgcaatgaca ggatgcaccg cgcggtgagc gccagcagga ggcgcgagct 14640
   gagcgacaga gaacttatgc acagcttgca aagagctcta acgggggccg ggactgatgg 14700
   ggagaactac tttgacatgg gagcggattt gcaatggcaa cccagtcgca gggccatgga 14760
   ggctgcaggg tgtgagcttc cttacataga agaggtggat gaagtcgagg acgaggaggg 14820
   cgagtacttg gaagactgat ggcgcgaccc gtatttttgc tagatggaac agcagcaggc 14880
   accggacccc gcaatgcggg cggcgctgca gagccagccg tccggcatta actcctcgga 14940
   cgattggacc caggccatgc aacgcataat ggcgctgacg acccgcaacc ccgaagcctt 15000
   tagacagcaa ccccaggcca accgcctttc tgccatactg gaggccgtag tgccctcccg 15060
   ctccaacccc acccacgaga aggtcctggc tatcgtgaac gcgctggtgg agaacaaggc 15120
   catccgtccc gatgaggccg ggctggtata caatgctctc ttggagcgcg tggcccgtta 15180
   caacagcagc aacgtgcaaa ccaacctgga ccggatggtg accgatgtgc gcgaggccgt 15240
   gtctcagcgc gagcgattcc agcgcgacgc caacttgggg tcgttggtag cgctaaacgc 15300
   tttcctcagc acccagcccg ccaacgtgcc ccgtggtcag caagactata caaacttttt 15360
   gagtgcattg agactcatgg tagctgaggt gccccagagc gaggtgtacc agtccgggcc 15420
   agattacttc ttccagacca gcagacaggg cttgcagaca gtgaacctga ctcaggcttt 15480
   caagaacctg aagggtctgt ggggagtgca cgccccagta ggggatcgcg cgaccgtgtc 15540
   tagcttgctg actcccaact cccgcctgct gctgctgctg gtatccccct ttactgacag 15600
   cggtagcatt gaccgcaact cgtacttggg ctacctgctt aacctgtatc gcgaggccat 15660
   aggacagagc caggtggacg agcagaccta tcaagaaatc acccaagtga gccgcgccct 15720
   gggtcaggaa gacacgggca gtttggaagc caccctgaac ttcttgctaa ccaaccggtc 15780
   acagaagatc cctcctcagt atgcgcttac cgctgaggag gagcggatcc tcagatacgt 15840
   gcaacagagc gttggactgt tcctgatgca ggagggggcg acacctaccg ccgcgctgga 15900
   catgacagct cgaaacatgg agcccagcat gtatgctagt aacaggcctt tcattaacaa 15960
   actgctggac tacctgcaca gggcggccgc catgaactct gattatttca ccaatgctat 16020
   cctgaaccca cactggctgc ccccacctgg tttctacact ggcgagtacg acatgcccga 16080
   ccccaatgac gggttcctgt gggacgatgt ggacagcagc atattttccc cgcctcccgg 16140
   ttatacagtt tggaagaagg aagggggcga tagaagacac tcttccgtgt cgctatccgg 16200
   aacggctggt gctgccgcga ccgtgcccga agctgcaagt cctttcccta gcttgccctt 16260
   ttcactaaac agcgttcgca gcagtgaact ggggagaata acccgcccgc gcttgatggg 16320
   cgaggatgag tacttgaatg actctttgct gaggccagag agggaaaaga acttccccaa 16380
   caatggaata gagagtctgg tggataagat gagtagatgg aagacctatg cgcaggatca 16440
   cagagacgag cccaggatct tgggggctac aagcagaccg atccgtagac gccagcgcca 16500
   cgacaggcag atgggtcttg tgtgggacga tgaggactct gccgatgata gcagcgtgtt 16560
   ggacttgggt ggaagaggag ggggcaaccc gttcgctcat ctgcgtccca gattcgggcg 16620
   catgttgtaa aagtgaaagt aaaataaaaa ggcaactcac caaggccatg gcgaccgagc 16680
   gtgcgttcgt tcttttttgt tatctgtgtc tagtacgatg aggagacgag ccgtgctagg 16740
   cggagcggtg gtgtatccgg agggtcctcc tccttcttac gagagcgtga tgcagcaaca 16800
   ggcggcgatg atacagcccc cactggaggc tcccttcgta cccccacggt acctggcgcc 16860
   tacggaaggg agaaacagca ttcgttactc ggagctgtcg cccctgtacg ataccaccaa 16920
   gttgtatctg gtggacaaca agtcggcgga catcgcctcc ctgaactatc agaacgacca 16980
   cagcaacttc ctgaccacgg tggtgcagaa caatgacttt acccccacgg aggctagcac 17040
   ccagaccatc aactttgacg agcggtcgcg atggggcggt cagctgaaga ccatcatgca 17100
   caccaacatg cccaacgtga acgagtacat gttcagcaac aagttcaagg cgagggtgat 17160
   ggtgtccaga aaagctcctg aaggtgttac agtaaatgac acctatgatc ataaagagga 17220
   tatcttgaag tatgagtggt ttgagttcat tttaccagaa ggcaactttt cagccaccat 17280
   gacgatcgac ctgatgaaca atgccatcat tgacaactac ctggaaattg gcagacagaa 17340
   tggagtgctg gaaagtgaca ttggtgttaa gtttgacact agaaatttca ggctcgggtg 17400
   ggaccccgaa actaagttga ttatgccagg tgtctacact tatgaggcat tccatcctga 17460
   cattgtattg ctgcctggtt gcggggtaga ctttactgaa agccgactta gcaacttgct 17520
   tggcatcagg aagagacatc cattccagga gggtttcaaa atcatgtatg aagatcttga 17580
   agggggtaat attcctgccc ttttggatgt cactgcctat gaggaaagca aaaaggatac 17640
   cactactgaa acaaccacac tggctgttgc agaggaaact agtgaagatg atgatataac 17700
   tagaggagat acctatataa ctgaaaaaca aaaacgtgaa gctgcagctg ctgaagttaa 17760
   aaaagagtta aagatccaac ctctagaaaa agacagcaag agtagaagct acaatgtctt 17820
   ggaagacaaa atcaacacag cctaccgcag ttggtacctg tcctacaatt acggtaaccc 17880
   tgagaaagga ataaggtctt ggacactgct caccacttca gatgtcacct gtggggcaga 17940
   gcaggtctac tggtcgctcc ctgacatgat gcaagaccca gtcaccttcc gctccacaag 18000
   acaagtcaac aactacccag tggtgggtgc agagcttatg cccgtcttct caaagagttt 18060
   ctacaatgag caagccgtgt actctcagca gctccgacag gccacttcgc tcacgcacgt 18120
   cttcaaccgc ttccctgaga accagatcct catccgcccg ccggcgccca caattaccac 18180
   cgtcagtgaa aacgttcctg ctctcacaga tcacgggacc ctgccgttac gcagcagtat 18240
   ccggggagtc cagcgcgtga ccgttactga cgccagacgc cgcacctgtc cctacgttta 18300
   caaggccctg ggcatagtcg cgccgcgcgt tctttcaagc cgcactttct aaaaaaaaaa 18360
   aaaatgtcca ttctcatctc gcccagtaat aataccggtt ggggactgta tgcgcccacc 18420
   aagatgtatg gaggcgcccg caagcgctct acccagcatc ctgtgcgcgt tcgcggtcat 18480
   ttccgcgctc cctggggcgc actcaagggt cgtacccgca ctcggaccac ggtcgatgat 18540
   gtgatcgacc aggtggtcgc cgatgctcgt aattatactc ctactgcgcc tacatctact 18600
   gtggatgcag ttattgacag tgtggtggca gacgcccgcg cctatgctcg ccggaagagc 18660
   cgaaggaggc gcatcgccag gcgccacagg gctactcccg ccatgcgagc cgcaaaagct 18720
   attctgcgga gggccaaacg tgtggggcga agagccatgc ttagagcggc cagacgcgcg 18780
   gcttcaggtg ccagcagcgg caggtcccgc aggcgcgcgg ccacggcggc agcagcggcc 18840
   attgccaaca tggcccaacc gcgaagaggc aatgtgtact gggtgcgtga tgccactacc 18900
   ggccagcgcg tgcccgtgcg cacccgcccc cctcgcactt agaagatact gagcagtctc 18960
   cgatgttgtg tcccagcggc aagtatgtcc aagcgcaaat acaaggaaga gatgctccag 19020
   gtcatcgcgc ctgaaatcta cggtccgccg gtgaaggatg aaaaaaagcc ccgcaaaatc 19080
   aagcgggtca aaaataacaa aaaggaagaa gatgacgatg atgggctggt ggagtttgtg 19140
   cgcgagttcg ccccaagacg gcgcgtgcag tggcgcgggc gcaaagtgcg tcaagtgctc 19200
   agacccggga ccactgtggt ttttacacct ggcgagcgtt ccagcactac ttttaaacgg 19260
   tcctatgatg aggtgtacgg ggatgacgat attcttgagc aggcggcaga ccgccttgac 19320
   gagtttgctt atggcaagcg cactagatcc agtcccaaag aggaggctgt gtccattcct 19380
   ttggatcatg gaaatcccac ccccagcctc aaaccagtca ccctgcagca agtgctgccc 19440
   gtgcctgcgc ggagaggcgt aaagcgcgag ggtgaggacc tatatcccac catgcagcta 19500
   atggtgccca agcgccagag gctagaagac gtactggaga aaatgaaagt ggatgccgat 19560
   atccagcctg aggtcaaagt aagacctatc aaggaagtgg cgccaggttt gggagtacaa 19620
   accttcgaca tcaagattcc caccgagtcc atggaagtgc agaccgaacc tgcaaaaccc 19680
   accacctcaa ttgaggtgca aacggaaccc tggatgcccg cgcccgttgc cgcccccagc 19740
   accactcgaa gatcacgacg aaagtacggc ccagcaagtc tgctaatgcc caactatgct 19800
   ctgcacccat ccatcattcc cactccgggt tacagaggca ctcgctacta tcgaaaccgg 19860
   agcaacacct ctcgccgccg caaaccacct gcaagtcgca ctcgccgtcg ccgccgccgc 19920
   aacactgcca gcaaattgac tcccgccgcc ctggtgcgga gagtgtaccg cgatggtcgc 19980
   gctgaacctc tgacgctgcc gcgcgcgcgc taccatccaa gcatcaccac ttaatgactg 20040
   ttgacgctgc ctccttgcag atatggccct cacttgccgc cttcgcgtcc ccattactgg 20100
   ctaccgagga agaaactcgc gccgtagaag gatgttgggg cgagggatgc gccgccacag 20160
   acgaaggcgc gctatcagca gacgattagg gggtggcttt ttgccagctc ttatacccat 20220
   catcgccgca gcgatcgggg cgataccagg catagcttca gtggcggttc aggcctcgca 20280
   gcgccactaa cattggaaaa aacttataaa taaaaaatag aatggactct gacgctcctg 20340
   gtcctgtgac tatgtttttg tagagatgga agacatcaat ttttcatccc tggctccgcg 20400
   acacggcacg aggccgtaca tgggcacctg gagcgacatc ggcacgagcc aactgaacgg 20460
   gggcgccttc aattggagca gtatctggag cgggcttaaa aattttggct cgaccgtaaa 20520
   aacctatggg aacaaagctt ggaacagcag cacagggcag gctctgagaa ataagcttaa 20580
   ggaacaaaac ttccaacaga aggtggtcga tgggatcgcc tctggtatta acggcgtagt 20640
   ggatctggcc aaccaggctg tacaaaaaca gataaacagc cgcctggacc cgccgcccgc 20700
   aacccctggt gaaatggaag tggaggaaga acttcctccg ctggaaaagc ggggcgacaa 20760
   gcgtccgcga cccgagctag agcagacgct ggtgacgcgc gcagacgagc ccccttcata 20820
   tgaggaggca gtaaagctcg gaatgcccac taccaggcct gtagctcaca tggctaccgg 20880
   ggtgatgaaa ccttctcagt cacatcgacc cgccaccttg gacttgcctc ctccccctgc 20940
   ttctgcggcg cctgttccca aacctgtcgc taccagaaag cccaccgccg tacagcccgt 21000
   cgccgtagcc agaccgcgtc ctgggggcac accgcgcccg aaagcaaact ggcaaagtac 21060
   tctgaacagc atcgtgggtc tgggcgtgca gagtgtaaag cgccgtcgct gctattaatt 21120
   aaatatggag tagcgcttaa cttgcttgtc tgtgtgtatg tatcatcacc acgccgccgc 21180
   agcagaggag aaaggaagag gtcgcgcgcc gaggctgagt tgctttcaag atggccaccc 21240
   catcgatgat gccccaatgg gcatacatgc acatcgccgg acaggatgct tcggagtacc 21300
   tcagtccggg tctggtgcag ttcgcccgtg caacagacac ctacttcagt atggggaaca 21360
   aatttagaaa ccccacagtg gcgcccaccc acgatgtgac caccgaccgt agccagcgcc 21420
   tgatgctgcg cttcgtgccc gttgaccggg aagacaatac ctactcttac aaagttcgct 21480
   acacgctggc tgtaggcgac aacagagtgc ttgacatggc cagcacattc tttgacattc 21540
   ggggggtgct tgatagaggt cctagcttca agccatattc cggcacagct tacaattcac 21600
   tcgctcctaa gggcgcgccc aatacatctc agtggatagt tacaacaaat ggggacaatg 21660
   cagtaactac caccacaaac acatttggca ttgcttccat gaagggagac aatattacta 21720
   aagaaggttt gcaaattggg aaagacatta ccactactga aggagaagaa aagcccattt 21780
   atgccgataa aacatatcag ccagagcctc aagttggaga agaatcatgg actgatactg 21840
   atggaacaaa tgaaaagttt ggtggaagag cccttaaacc agctaccaac atgaagccat 21900
   gctacgggtc ttttgcaaga cctacaaaca taaaaggggg ccaagctaaa aacagaaaag 21960
   taaaaccaac aaccgaagga ggggttgaaa ctgaggaacc agatattgat atggaatttt 22020
   tcgatggtag agatgctgtt gcaggagctt tagcgcctga aattgtgctt tatacggaaa 22080
   atgtaaattt ggaaactcca gacagtcatg tggtatataa accagaaacg tctaataact 22140
   ctcatgcaaa tttgggtcaa caagccatgc ctaacagacc caattacatt ggcttcaggg 22200
   ataacttcgt aggcctaatg tactacaaca gtactggaaa tatgggagtt ttggctggcc 22260
   aagcatcaca actgaatgca gtggttgact tgcaggacag aaatactgaa ctgtcatatc 22320
   agcttttgct tgattctctg ggagacagaa ccagatactt cagcatgtgg aatcaggctg 22380
   tggacagtta cgatcccgat gttcgcatta ttgaaaatca tggcatcgag gatgaactgc 22440
   ctaattactg ttttcctctg aatggcatag gaccagggca cacatatcaa ggcattaaag 22500
   ttaaaaccga tgacactaat ggatgggaaa aagatgctaa tgttgctcca gctaatgaaa 22560
   taaccatagg caacaacctg gctatggaaa ttaatatcca agctaacctt tggagaagtt 22620
   ttctgtactc taatgtggct ttgtaccttc cagatgttta caagtacacg ccacctaaca 22680
   ttactttgcc cactaacacc aacacctatg agtacatgaa cgggcgagtg gtatccccat 22740
   ccctggttga ttcatacatc aacattggcg ccaggtggtc tcttgaccca atggacaatg 22800
   tgaatccatt caaccaccac cgcaatgctg gtctgcgcta caggtccatg cttctgggaa 22860
   atggtcgtta tgtgcctttc cacatacaag tgcctcagaa attctttgct gtcaagaacc 22920
   tacttcttct acctggctcc tacacctacg agtggaactt ccgaaaggat gtgaacatgg 22980
   tcctgcaaag ttcccttgga aatgacctca gaacggatgg tgctaccata agtttcacca 23040
   gcatcaatct ctatgccacc ttcttcccca tggctcacaa cacagcttcc acccttgaag 23100
   ccatgctgcg caacgatacc aatgatcagt catttaacga ctacctctct gcagctaaca 23160
   tgctttaccc cattcctgcc aatgcaacca acattccaat ttccatccca tctcgcaact 23220
   gggcagcctt caggggctgg tccttcacca gactcaaaac caaggagact ccatctcttg 23280
   gatcagggtt cgatccctac ttcgtatatt ctggatctat tccctacctg gatggcacct 23340
   tttaccttaa ccacactttc aagaaggtct ccatcatgtt tgactcctca gtcagctggc 23400
   ctggcaatga caggctgttg agtccaaatg agtttgaaat caagcgcact gtggacgggg 23460
   aaggatacaa cgtggcacaa tgcaacatga ccaaagactg gttcctggtt cagatgcttg 23520
   ccaattacaa cattggctac cagggctttt acatccctga gggatacaag gatcgcatgt 23580
   actccttttt cagaaacttc cagcctatga gcaggcaggt ggttgatgag gttaattaca 23640
   ctgactacaa agccgtcacc ttaccatacc aacacaacaa ctctggcttt gtagggtacc 23700
   ttgcacctac tatgagacaa ggggaacctt acccagccaa ttatccatac ccgctcatcg 23760
   gaactactgc cgttaagagt gtcacccaga aaaagttcct gtgcgacagg accatgtggc 23820
   gcattccctt ctccagcaac ttcatgtcca tgggggccct taccgacctg ggacagaaca 23880
   tgctctatgc caactcagcc catgcgctgg acatgacttt tgaggtggat cccatggatg 23940
   agcccaccct gctttatctt cttttcgaag tcttcgacgt ggtcagagtg caccagccac 24000
   accgcggcgt catcgaggcc gtctacctgc gcacaccgtt ctcggccggc aacgccacca 24060
   cataagaagc ctcttgcttc ttgcaagcag cagctgcagc catgacatgc gggtccggaa 24120
   acggctccag cgagcaagag ctcaaagcca tcgtccgaga cctgggctgc ggaccctatt 24180
   tcctgggaac ctttgacaag cgtttcccgg ggttcatggc ccccgacaag ctcgcctgcg 24240
   ccatagtcaa cactgccgga cgcgagacgg ggggagagca ctggctggct tttggttgga 24300
   acccgcgcta caacacctgc tacctttttg atccttttgg gttctcggat gagcggctca 24360
   aacagattta ccagtttgag tacgaggggc tcctgcgtcg cagtgccctt gctaccaaag 24420
   accgctgcat caccctggag aagtctaccc aaagcgtgca gggtccgcgc tcagccgcct 24480
   gtggactttt ttgctgtatg ttccttcatg cctttgtgca ctggcccgac cgccccatgg 24540
   acggaaaccc caccatgaag ttgctgactg gggtgtccaa cagcatgctc caatcacccc 24600
   aagtccagcc caccctgcgc cgcaaccagg aggtgctata ccgcttccta aacacccact 24660
   catcttactt tcgttctcac cgcgcgcgca ttgaaagggc caccgcgttt gaccgtatgg 24720
   atatgcaata agtcatgtaa aaccgtgttc aataaacagc actttatttt tacatgcact 24780
   gaggctctgg ttttgctcat ttgtttcatc atttactcag aagtcgaatg ggttctggcg 24840
   ggagtcagag tgacccgcgg gcagggatac gttgcggaac tgtaacctgt tctgccactt 24900
   gaactcgggg attaccagct tgggaactgg aatctcggga aaggtgtctt gccacaactt 24960
   tctggtcagt tgcatagcgc caagcaggtc aggagcagag atcttgaaat cacagttggg 25020
   gccggcattc tggacacggg agttgcgata cactgggttg caacactgga acactatcaa 25080
   cgctgggtgt cttacgcttg ccaacacggt tgggtcactg atggtagtca catccaagtc 25140
   ttcagcattg gccatcccaa agggggtcat cttacatgtc tgcctgccca tcacgggagc 25200
   gcagcctggc ttgtggttgc aatcacaatg aatggggatc agcatcatcc tggcttggtc 25260
   gggagttatc cctgggtaca cagccttcat gaaggcttcg tactgcttaa aagcttcctg 25320
   ggccttactt ccctcggtgt agaacatccc acaggacttg ctggaaaatt gattagtagt 25380
   acagttggca tcattcacac aacagcgggc atcgttgttg gccaactgaa ccacatttct 25440
   gccccagcgg ttttgggtga tcttggctct gtctggattc tccttcatag cgcgctgccc 25500
   gttctcgctc gccacatcca tctcgataat gtggtccttc tggatcatga tagtgccatg 25560
   caggcatttc accttgcctt cataatcggt gcatccatga gcccacagag cgcacccggt 25620
   gcactcccaa ttattgtggg cgatctcaga ataataatgt accaatccct gcatgaatct 25680
   tcccatcatt gttgtcaagg tcttcatgct ggtaaatgtc agcgggatgc cacggtgctc 25740
   ctcgttcaca tactggtggc agatacgctt gtattgctcg tgctgctctg gcatcagctt 25800
   gaaagaggtt ctcagatcat tatccagcct gtacctttcc attagcacag ccatcacttc 25860
   catgcccttc tcccaggcag ataccagggg cagactcaaa ggattcctaa cagcaataaa 25920
   agtagctcct ttagctatag ggtcattctt gtcgatcttc tcaacacttc tcttgccatc 25980
   cttctcaatg atgcgcaccg gggggtagct gaagcccacg gccaccaact gagcctgttc 26040
   tctttcttct tcactatcct ggctgatgtc ttgcagaggg acatgcttgg tcttcctggg 26100
   cttcttcttg ggagggatcg ggggaggact gttgctccgc tccggagaca gggatgactg 26160
   cgaagtttcg cttaccaata ccacctggct ctcggtagaa gaaccggacc ccacacgacg 26220
   gtaggtgttc ctcttcgggg gcagaggtgg aggcgactga gatgggctgc ggtctggcct 26280
   tggaggcgga tggctggcag agctcattcc gcgttcgggg gtgtgctccc ggtggcggtc 26340
   gcttgactga tttcctccgc ggctggccat tgtgttctcc taggcagaga aacaacagac 26400
   atggaaactc agccatcact gccaacatcg ctgcaagcac catcacacct cgcccccagc 26460
   agcgacgagg aggagagctt aaccacccca ccacccagtc ccgctaccac cacctctacc 26520
   ctcgatgatg aggaggaggt cgacgcagcc caggagatgc aggcgcagga taatgtgaaa 26580
   acggaagaga ttgaggcaga tgtcgagcag gacccgggct atgtgacgcc ggcggagcac 26640
   caggaggagc tgaaacgctt tctagacaga gaggatgacg accgcccaga gcatcaagca 26700
   gatggcgttt accaggaggc tgggatcagg gatcatgtcg ccgactacct caccggcctt 26760
   ggtggggagg acgtgctcct caaacatcta gcaaggcagt cgatcatagt taaagacgca 26820
   ttgctcgatc tcactgaagt gcccatcagt gtggaagagc ttagccgcgc ctacgagctg 26880
   aacctctttt cgcctcaggt accccccaag cggcagccaa acggcacctg cgaggccaac 26940
   cctcgactca acttctatcc agcttttact atccccgaag tgttggccac ctaccacatc 27000
   tttttcaaga accaaaagat tccagtctcc tgccgcgcca accgcacccg cgccgatgcc 27060
   ctgctcaact tgggtccggg agctcgctta cctgatatag cttccttgga agaggttcca 27120
   aagatctttg agggtctggg aagtgatgag acacgggccg caaatgctct gcaacaggga 27180
   gagaatgaca tggatgaaca ccacagcgct ctggtggaac tggagggtga caatgcccgg 27240
   attgcagtgc tcaagcgcag tatcgtggtc acccattttg cctaccccgc tgttaacctg 27300
   ccccccaaag ttatgagcgc tgtcatggac catctgctca tcaaacgagc aagacctctt 27360
   tcagaaaacc agaacatgca ggatccagac gcctcggacg agggcaagcc ggtagtcagt 27420
   gacgagcagc tatctcgctg gctgggtacc aactcccccc gagatttgga agagaggcgc 27480
   aagcttatga tggctgtagt gctagtaact gtggagctgg agtgtctgcg ccgctttttc 27540
   accgaccctg agaccctgcg caagctagag gagaacctgc actacacctt tagacatggc 27600
   ttcgtgcggc aggcatgcaa gatctccaac gtggagctta ccaacctggt ttcttacatg 27660
   ggcattttgc atgagaaccg actagggcag agcgtcctgc acaccaccct taaaggggag 27720
   gcccgccgtg actacatccg agactgtgtc tacctctacc tctgccatac ctggcaaact 27780
   ggtatgggtg tgtggcaaca gtgtttggaa gagcagaacc taaaagagct ggacaagctc 27840
   ttgcagagat ccctcaaagc cctgtggaca ggttttgatg agcgcaccgt cgcctcggac 27900
   ctggcagaca tcatcttccc cgagcgtctc agggttactc tgcgaaacgg cctgccagac 27960
   tttatgagcc agagcatgct taacaacttt cgctctttca tcctggaacg ctccggtatc 28020
   ctgcctgcca cctgctgtgc gctgccctcc gactttgtgc ctctcaccta ccgcgagtgc 28080
   ccaccgccgc tatggagcca ctgctacctg ttccgcctgg ccaactacct ctcctaccac 28140
   tcggatgtta tagaggatgt gagcggagac ggcctgctgg aatgccactg ccgctgcaat 28200
   ctttgcacac cccaccgctc ccttgcctgc aacccccagt tgctgagcga gacccagatt 28260
   atcggcacct tcgagctgca gggtcccaga agtaaaggcg aggggtcttc tccggggcag 28320
   agtttgaaac tgacaccggg gctgtggacc tccgcctacc tgcgcaagtt tcaccccgag 28380
   gactaccatc cctatgagat caggttctat gaggaccaat cacatcctcc caaagtcgag 28440
   ctctcagcct gcgtcatcac ccagggagca attctggccc aattgcaagc catccaaaaa 28500
   tctcgccaag aatttctgct aaaaaaggga aacggggtct accttgaccc tcagaccggt 28560
   gaggagctca acacaaggtt cccccaggat gtcccatcgc cgaggaagca agaagttgaa 28620
   ggtgcagctg tcgcccccag aggatatgaa ggaagactgg gacagtcagg cagaggagga 28680
   gatggaagat tgggacagcc aggcagagga ggtggacagc ctggaggaag acagtttgga 28740
   ggaggaagac gaggaggcag aggaggtgga agaagcaacc gccgccaaac agttgtcatc 28800
   ggcggcggag acaagcaagt ccccagacag cagcacggct accatctccg ctccgggtcg 28860
   gggggcccag cggcggccca acagtagatg ggacgagacc gggcgattcc caaacccgac 28920
   caccgcttcc aagaccggta agaaggagcg acagggatac aagtcctggc gtggacataa 28980
   aaacgctatc atctcctgct tgcatgaatg cgggggcaac atatccttca cccggcgata 29040
   cctgctcttc caccacggtg taaacttccc ccgcaatatc ttgcattact accgtcacct 29100
   ccacagcccc tactgcagtc agcaagtccc ggcaaccccg acagaaaaat acagcagcga 29160
   caacggtgac cagaaaacca gcagttagaa aatccacaac aagtgcacca ggaggaggac 29220
   tgaggatcac agcgaacgag ccagcgcaga ccagagagct gaggaaccgg atctttccaa 29280
   ccctctatgc cattttccag cagagtcggg ggcaagagca ggaactgaaa gtaaaaaacc 29340
   gatctctgcg ctcgctcacc agaagttgtt tgtatcacaa gagcgaagac caacttcagc 29400
   gcactctcga ggacgccgag gctctcttca acaagtactg cgcgctgact cttaaagagt 29460
   agcccttgcc cgcgctcatt ttgaaaacgg cgggaatcac gtcacccttg gcacctgtcc 29520
   tttgcccttg tcatgagtaa agagattccc acgccttaca tgtggagcta tcagccccaa 29580
   atggggttgg cagcaggcgc ttcccaggac tactccaccc gcatgaattg gcttagcgcc 29640
   gggccctcaa tgatatcacg ggttaatgat atacgagctt atcgaaacca gttactccta 29700
   gaacagtcag ctctcaccac cacaccccgt caacacctta atccccgaaa ttggcccgcc 29760
   accctggtgt accaggaaaa tcccgctccc accaccgtac tacttcctcg agacgcccag 29820
   gccgaagttc agatgactaa cgcaggtgta cagctggcgg gcggttccgc cctatgtcgt 29880
   caccgacctc aacagagtat aaaacgcctg gtgattagag gccgaggtat ccagctcaac 29940
   gacgagtcgg ttagctcttc gcttggtctg cgaccagacg gagtcttcca aatcgccggc 30000
   tgtgggagat cttccttcac tcctcgtcag gctgtgctga ctttggagag ttcgtcctcg 30060
   cagccccgct cgggcggcat tggaactctc cagtttgtgg aggagtttac tccctctgtc 30120
   tacttcaacc ccttctccgg ctctcctggc cagtacccgg acgagttcat accaaacttc 30180
   gacgcaatca gcgagtcagt ggatggctat gattgatgtc taatggtggt gcggctgagc 30240
   tagctcgact gcgacaccta gaccactgcc gccgctttcg ctgcttcgcc cgggaactca 30300
   ccgagttcat ctacttcgaa ctctccgagg agcaccctca gggtccggcc cacggagtgc 30360
   ggattaccat cgaaggggga atagactctc gcctgcatcg catcttctcc cagcggcccg 30420
   tgctaattga acgcgaccag ggaaatacaa ccatctccat ctactgcatc tgtaaccacc 30480
   ccggattgca tgaaagcctt tgctgtcttg tttgtgctga gtttaataaa aactgagtta 30540
   agaccctcct acggactacc gcttcttcaa tcaggacttt acaacaccaa ccagatcttc 30600
   cagaagaccc agacccttcc tcctttcatc caggactcta actctacctt accagcaccc 30660
   tccactacta accttcccga aacaaacaag cttgcatctc atctgcaaca ccgcctttca 30720
   cgaagccttc tttctgccaa tactaccact cccaaaaccg gaggtgagct ccgcggtctt 30780
   cctactgacg acccctgggt ggtagcgggt tttgtaacgt taggagtagt tgcgggtggg 30840
   cttgtgctga tcctttgcta cctatacaca ccttgctgtg catatttagt catattgtgc 30900
   tgttggttta agaaatgggg gccatactag tcgtgcttgc tttactttcg cttttgggtc 30960
   tgggctctgc taatctcaat cctctcgatc acgatccatg tttagacttc gacccagaaa 31020
   actgcacact tacttttgca cccgacacaa gccgtctctg tggagttctt attaagtgcg 31080
   gatgggactg caggtccgtt gaaattacac ataataacaa aacatggaac aataccttat 31140
   ccaccacatg ggagccagga gttccccagt ggtatactgt ctctgtccga ggtcctgacg 31200
   gttccatccg cattagtaac aacactttca ttttttctga aatgtgcgat ctggccatgt 31260
   tcatgagcag acagtatgac ctatggcctc ccagcaaaga gaacattgtg gcattttcca 31320
   ttgcttattg cttggtaaca tgcatcatca ctgctatcat ttgtgtgtgc atacacttgc 31380
   ttatagttat tcgccctaga caaagcaatg aggaaaaaga gaaaatgcct taaccttttt 31440
   cctcatacct tttctttaca gcatggcttc tgttacagct ctaattattg ccagcattgt 31500
   cactgtcgct cacgggcaaa caattgtcca tattacctta ggacataatc acactcttgt 31560
   agggccccca attacttcag aggttatttg gaccaaactt ggaagtgttg attattttga 31620
   tataatttgc aacaaaacta aaccaatatt tgtaatctgt aacagacaaa atctcacgtt 31680
   aattaatgtt agcaaaattt ataacggtta ctattatggt tatgacagat ccagtagtca 31740
   atataaaaat tacttagttc gcataactca gcccaaatta acagtgccaa ctatgacaat 31800
   aattaaaatg gctaataaag cattagaaaa ttttacatca ccaacaacac ccaatgaaaa 31860
   aaacattcca aattcaatga ttgcaattat tgcggcggtg gcattgggaa tggcactaat 31920
   aataatatgc atgctcctat atgcttgtta ctataaaaag tttcaacata aacaggatcc 31980
   actactaaat tttaacattt aattttttat acagatgatt tccactacaa tttttatcat 32040
   tactagcctt gcagctgtaa cttatggccg ttcacaccta actgtacctg ttggctcaac 32100
   atgtacacta caaggacccc aagaaggcta tgtcacttgg tggagaatat atgataatgg 32160
   agggttcgct agaccatgtg atcagcctgg tacaaaattt tcatgcaacg gaagagactt 32220
   gaccattatt aacataacat caaatgagca aggcttctat tatggaacca actataaaaa 32280
   tagtttagat tacaacatta ttgtagtgcc agccaccact tctgctcccc gcaaatccac 32340
   tttctctagc agcagtgcca aagcaagcac aattcctaaa acagcttctg ctatgttaaa 32400
   gcttccaaaa atcgctttaa gtaattccac agccgctccc aatacaattc ctaaatcaac 32460
   aattggcatc attactgccg tggtagtggg attaatgatt atatttttgt gtataatgta 32520
   ctacgcctgc tgctatagaa aacatgaaca aaaaggtgat gcattactaa attttgatat 32580
   ttaatttttt atagaattat gatattgttt caatcaaata ccactacctc ctatgcatac 32640
   acaaacattc agcctaaata cgctatgcaa ctagaaatca caatactaat tgtaattgga 32700
   attcttatac tatctgttat tctttatttt atattctgcc gtcaaatacc caatgttcat 32760
   agaaattcta aaagacgtcc catctattct cctatgatta gtcgtcccca tatggctctg 32820
   aatgaaatct aagatctttt tttttttctc ttacagtatg gtgaacatca atcatgatcc 32880
   ctagaaattt cttcttcacc atactcatct gtgcttttaa tgtctgtgct actttcacag 32940
   cagtagccac tgcaagccca gactgtatag gaccatttgc ttcctatgca ctttttgcct 33000
   tcgttacttg catctgcgtg tgtagcatag tctgcctggt tattaatttt ttccaactgg 33060
   tagactggat ctttgtgcga attgcctacc tacgtcacca tcccgaatac cgcaatcaaa 33120
   atgttgcggc acttcttagg cttatttaaa accatgcagg ctatgctacc agtcatttta 33180
   attttgctac taccctgcat tcccctagct tccaccgcca ctcgcgctac acctgaacaa 33240
   cttagaaaat gcaaatttca acaaccatgg tcatttcttg attgctacca tgaaaaatct 33300
   gattttccca catactggat agtgattgtt ggaataatta acatactttc atgtaccttt 33360
   ttctcaatca caatataccc cacatttaat tttgggtgga attctcccaa tgcactgggt 33420
   tacccacaag aaccagatga acatattcca ctacaacaca tacaacaacc actagcactg 33480
   gtacagtatg aaaatgagcc acaaccttca ctgccccctg ccattagtta cttcaaccta 33540
   accggcggag atgactgacc caatcgccac atcatccacc gctgccaagg agctgctgga 33600
   catggacgga cgtgcctcag aacagcgact catccaacta cgcattcgtc agcagcagga 33660
   acgagcagta aaagagctaa gggatgccat tgggattcac cagtgcaaaa aaggcatatt 33720
   ctgcttagta aaacaatcca aaatctccta cgagatcacc gctactgacc atcgtctctc 33780
   atacgagctc ggtccgcagc gacaaaaatt cacctgcatg gtgggaatca accccatagt 33840
   tatcacccag cagtctggag atactaaggg ttgtatccag tgttcctgtg attccaccga 33900
   gtgcatctac acactgctga agaccctctg cggccttcga gacctcctac ccatgaacta 33960
   atcattgccc ctaccttacc caatcaaaat attaataaag acacttactt gaaatcagca 34020
   atacagtctt tgtcaaaact ttctaccagc agcacctcac cctcttccca actctggtac 34080
   tctaaacgtc ggagggtggc atactttctc cacactttga aagggatgtc aaattttatt 34140
   tcctcttctt tgcccacaat cttcatttct ttatccccag atggccaagc gagctcggct 34200
   aagcacttcc ttcaacccgg tgtaccctta tgaagatgaa agcagctcac aacacccatt 34260
   tataaatcct ggtttcattt cccctgacgg gttcacacaa agtccaaacg gggttttaag 34320
   tcttaaatgt gttaatccac ttaccactgc aagcggctcc ctccaactta aagtgggaag 34380
   tggtcttaca gtagacacta ctgatggatc cttagaagaa aacatcaaag ttaacacccc 34440
   cctaacaaag tcaaaccatt ctataaattt accaatagga aacggtttgc aaatagaaca 34500
   aaacaaactt tgcagtaaac tcggaaatgg tcttacattt gactcttcca attctattgc 34560
   actgaaaaat aacactttat ggacaggtcc aaaaccagaa gccaactgca taattgaata 34620
   cgggaaacaa aacccagata gcaaactaac tttaatcctt gtaaaaaatg gaggaattgt 34680
   taatggatat gtaacgctaa tgggagcctc agactacgtt aacaccttat ttaaaaacaa 34740
   aaatgtctcc attaatgtag aactatactt tgatgccact ggtcatatat taccagactc 34800
   atcttctctt aaaacagatc tagaactaaa atacaagcaa accgctgact ttagtgcaag 34860
   aggttttatg ccaagtacta cagcgtatcc atttgtcctt cctaatgcgg gaacacataa 34920
   tgaaaattat atttttggtc aatgctacta caaagcaagc gatggtgccc tttttccgtt 34980
   ggaagttact gttatgctta ataaacgcct gccagatagt cgcacatcct atgttatgac 35040
   ttttttatgg tccttgaatg ctggtctagc tccagaaact actcaggcaa ccctcataac 35100
   ctccccattt accttttcct atattagaga agatgactga caacaaaaat aaagttcaac 35160
   attttttatt gaaattcctt ttacagtatt cgagtagtta ttttgcctcc cccttcccat 35220
   ttaacagaat acaccaatct ctccccacgc acagctttaa acatttggat accattagag 35280
   atagacatag ttttagattc cacattccaa acagtttcag agcgagccaa tctggggtca 35340
   gtaatacata aaaatgcatc gggatagtct tttaaagcgc tttcacagtc caactgttgc 35400
   ggatgcgact ccggagtctg aatcacggtc atctggaaga agaacgatgg gaatcataat 35460
   ccgaaaacgg aatcgggcga ttgtgtctca tcaaacccac aagcaaccgc tgtctgcgtc 35520
   gctccgtgcg actgctgttt atgggatcgg ggtccgcagt gtcctgaagc atgattttaa 35580
   tagcccttaa cattaacttt ctggtgcgat gcgcgcagca acgcattctg atttcactta 35640
   gattactaca gtaggtacag cacattatca caatattgtt taataaacca taattaaaag 35700
   cgctccagcc aaaactcata tctgatataa tcgcccctgc atgaccatca taccaaagtt 35760
   taatataaat taaatgtcgt tccctcaaaa acacactacc cacatacatg atctcttttg 35820
   gcatgtgcat attaacaatt tgtctgtacc atggacaacg ttggttaatc atgcaaccca 35880
   atataacctt ccggaaccac actgccaaca ccgctccccc agccatgcat tgaagtgaac 35940
   cctgctgatt acaatgacaa tgaagaaccc aattctctcg accatgaatc acttgagact 36000
   gaaaaatatc tatagtagca caacaaagac ataaatgcat gcatcttctc ataattttta 36060
   actcctctgg atttaaaaac atatcccaag gaatgggaaa ctcttgcaga acagtaaagc 36120
   tggcagaaca aggaagacca cgaacacaac ttacactatg catagtcata gtatcacaat 36180
   ctggcaacag cgggtggtct tcagtcatag aagctcgggt ttcattttcc tcacatcgtg 36240
   gtaattgggc tctggtgtaa gggtgatgtc tggcgcatga tgtggagcgt gcgcgcaacc 36300
   ttgtcataat ggagttgctt cctgacattc tcgtattttg tatagcaaaa cgctgccctg 36360
   gcacaacaca ctcttcttcg tcttctatcc tgccgcttag tgtgttccgt ctgataattc 36420
   aagtacagcc acactcttaa gttggtcaaa agaatgctgg cttcagttgt aatcaaaact 36480
   ccatcatatt taattgttct aaggaaatca tccacggtag catatgcaaa tcccaaccaa 36540
   gcaatgcaac tggattgcgt ttcaagcagc agaggagagg gaagagacgg aagaatcatg 36600
   ttaattttta ttccaaacga tctcgcagta cttcaaattg tagatcgcgc agatggcatc 36660
   tatcgccccc actgtgttgg tgaaaaagca cagctaaatc aaaagaaatg cgattttcaa 36720
   ggtgctcaac ggtggcttcc aacaaagcct ccacgcgcac atccaaaaac aaaagaatac 36780
   caaaagaagg agcattttct aactcctcaa acatcatatt acattcctgc accattccca 36840
   gataattttc agctttccag ccttgaatta ttcgtgtcag ttcttgtggt aaatccaaac 36900
   cacacattac aaacaggtcc cggagggcgc cctccaccac cattcttaaa cacaccctca 36960
   taatgacaaa atatcttgct cctgtgtcac ctgtagcaaa ttaagaatgg catcatcaat 37020
   tgacatgccc ttggctctaa gttcttctct aagttctagt tgtagatact ctctcatatt 37080
   atcaccaaac tgcttagcca gaagcccccc gggaacaata gcaggggacg ctacagtgca 37140
   gtacaagcgc agacctcccc aattggctcc agcaaaaaca agattagaat aagcatactg 37200
   ggaaccacca gtaatatcat caaagttgct ggaaatataa tcaggcagag tttcttgtaa 37260
   aaattgaata aaagaaaaat tttccaaaga aacattcaaa accgttggga tgcaaataca    37320
   ataggttacc gcgctgcgct ccaacattgt tagttttgaa ttagtctgca aaataaaaga    37380
   aacaagcgtc atatcatagt agcctgtcga acaggtggaa aaatcagtct ttccatcaca    37440
   agacaagcca cagggtctcc agctcgaccc tcgtaaaacc tgtcattgtg attaaacaac    37500
   agcaccgaaa gttcctcgcg gtggccagca tgaataattc ttgatgaagc atacaatcca    37560
   gacatgttag catcagttaa agagaaaaaa cagccaacat agcctctggg tataattatg    37620
   cttaatttta agtatagcaa agccacccct cgcggataca aagtaaaagg cacaggagaa    37680
   taaaaaatat aattatttct ctgctgctgt tcaggcaacg ttgctcccgg tccctctaaa    37740
   tagacataca aagcctcatc agccatggct taccaggcaa agtacagcgg gcgcacaaag    37800
   cacaagctct aaagaagctc taaaaacact ctccaacctc tccacaatat atacacaagc    37860
   cctaaactga cgtaatggga gtaaagtgaa aaaaaaatac cgccaagccc aacacacacc    37920
   ccgaaactgc gtcagcagga aaaagtacag tttcacttcc gcattcccaa caagcgtaac    37980
   ttcctctttc tcatggtacg tcacatccga ttaacttgca acgtcatttt cccacggtcg    38040
   cgccgcccct tttagccgtt aaccccgcag ccaatcacca attt                     38084
<210> 15
   <211> 1932
   <212> DNA
   <213> Artificial Sequence
<220>
   <223> hNIS
<400> 15
   atggaggccg tggagaccgg ggaacggccc accttcggag cctgggacta cggggtcttt      60
   gccctcatgc tcctggtgtc cactggcatc gggctgtggg tcgggctggc tcggggcggg     120
   cagcgcagcg ctgaggactt cttcaccggg ggccggcgcc tggcggccct gcccgtgggc     180
   ctgtcgctgt ctgccagctt catgtcggcc gtgcaggtgc tgggcgtgcc gtcggaggcc     240
   tatcgctatg gcctcaagtt cctctggatg tgcctgggcc agcttctgaa ctcggtcctc     300
   accgccctgc tcttcatgcc cgtcttctac cgcctgggcc tcaccagcac ctacgagtac     360
   ctggagatgc gcttcagccg cgcagtgcgg ctctgcggga ctttgcagta cattgtagcc     420
   acgatgctgt acaccggcat cgtaatctac gcaccggccc tcatcctgaa ccaagtgacc     480
   gggctggaca tctgggcgtc gctcctgtcc accggaatta tctgcacctt ctacacggct     540
   gtgggcggca tgaaggctgt ggtctggact gatgtgttcc aggtcgtggt gatgctaagt     600
   ggcttctggg ttgtcctggc acgcggtgtc atgcttgtgg gcgggccccg ccaggtgctc     660
   acgctggccc agaaccactc ccggatcaac ctcatggact ttaaccctga cccgaggagc      720
   cgctatacat tctggacttt tgtggtgggt ggcacgttgg tgtggctctc catgtatggc      780
   gtgaaccagg cgcaggtgca gcgctacgtg gcttgccgca cagagaagca ggccaagctg      840
   gccctgctca tcaaccaggt cggcctgttc ctgatcgtgt ccagcgctgc ctgctgtggc      900
   atcgtcatgt ttgtgttcta cactgactgc gaccctctcc tcctggggcg catctctgcc      960
   ccagaccagt acatgcctct gctggtgctg gacatcttcg aagatctgcc tggagtcccc     1020
   gggcttttcc tggcctgtgc ttacagtggc accctcagca cagcatccac cagcatcaat     1080
   gctatggctg cagtcactgt agaagacctc atcaaacctc ggctgcggag cctggcaccc     1140
   aggaaactcg tgattatctc caaggggctc tcactcatct acggatcggc ctgtctcacc     1200
   gtggcagccc tgtcctcact gctcggagga ggtgtccttc agggctcctt caccgtcatg     1260
   ggagtcatca gcggccccct gctgggagcc ttcatcttgg gaatgttcct gccggcctgc     1320
   aacacaccgg gcgtcctcgc gggactaggc gcgggcttgg cgctgtcgct gtgggtggcc     1380
   ttgggcgcca cgctgtaccc acccagcgag cagaccatga gggtcctgcc atcgtcggct     1440
   gcccgctgcg tggctctctc agtcaacgcc tctggcctcc tggacccggc tctcctccct     1500
   gctaacgact ccagcagggc ccccagctca ggaatggacg ccagccgacc cgccttagct     1560
   gacagcttct atgccatctc ctatctctat tacggtgccc tgggcacgct gaccactgtg     1620
   ctgtgcggag ccctcatcag ctgcctgaca ggccccacca agcgcagcac cctggccccg     1680
   ggattgttgt ggtgggacct cgcacggcag acagcatcag tggcccccaa ggaagaagtg     1740
   gccatcctgg atgacaactt ggtcaagggt cctgaagaac tccccactgg aaacaagaag     1800
   ccccctggct tcctgcccac caatgaggat cgtctgtttt tcttggggca gaaggagctg     1860
   gagggggctg gctcttggac cccctgtgtt ggacatgatg gtggtcgaga ccagcaggag     1920
   acaaacctct ga                                                         1932
<210> 16
   <211> 36587
   <212> DNA
   <213> Artificial Sequence
<220>
   <223> Ad3-hTERT-GM-CSF-E1A
<400> 16
<210> 17
   <211> 435
   <212> DNA
   <213> Artificial Sequence
<220>
   <223> GM-CSF
<400> 17
   atgtggctgc agagcctgct gctcttgggc actgtggcct gcagcatctc tgcacccgcc 60
   cgctcgccca gccccagcac gcagccctgg gagcatgtga atgccatcca ggaggcccgg 120
   cgtctcctga acctgagtag agacactgct gctgagatga atgaaacagt agaagtcatc 180
   tcagaaatgt ttgacctcca ggagccgacc tgcctacaga cccgcctgga gctgtacaag 240
   cagggcctgc ggggcagcct caccaagctc aagggcccct tgaccatgat ggccagccac 300
   tacaagcagc actgccctcc aaccccggaa acttcctgtg caacccagac tatcaccttt 360
   gaaagtttca aagagaacct gaaggacttt ctgcttgtca tcccctttga ctgctgggag 420
   ccagtccagg agtga 435
<210> 18
   <211> 35613
   <212> DNA
   <213> Artificial Sequence
<220>
   <223> Ad3-hTERT-E1A
<400> 18
   ctatctatat aatatacctt atagatggaa tggtgccaac atgtaaatga ggtaatttaa 60
   aaaagtgcgc gctgtgtggt gattggctgc ggggttaacg gctaaaaggg gcggcgcgac 120
   cgtgggaaaa tgacgtgact tatgtgggag gagttatgtt gcaagttatt acggtaaatg 180
   tgacgtaaaa cgaggtgtgg tttgaacacg gaagtagaca gttttcccac gcttactgac 240
   aggatatgag gtagttttgg gcggatgcaa gtgaaaattc tccattttcg cgcgaaaact 300
   aaatgaggaa gtgaatttct gagtcatttc gcggttatgc cagggtggag tatttgccga 360
   gggccgagta gactttgacc gtttacgtgg aggtttcgat taccgtgttt ttcacctaaa 420
   tttccgcgta cggtgtcaaa gttctgtgtt tttacgtagg tgtcagctga tcgctgatcg 480
   ctgcgctgtc ggggccaggc cgggctccca gtggattcgc gggcacagac gcccaggacc 540
   gcgcttccca cgtggcggag ggactgggga cccgggcacc cgtcctgccc cttcaccttc 600
   cagctccgcc tcctccgcgc ggaccccgcc ccgtcccgac ccctcccggg tccccggccc 660
   agccccctcc gggccctccc agcccctccc cttcctttcc gcggccccgc cctctcctcg 720
   cggcgcgagt ttcaggcagc gctgcgtcct gctgcgcacg tgggaagccc tggccgatgg 780
   gctcgaatcg tgccagcgag aagagttttc tcctccgcgc cgcaagtcag ttctgcgctt 840
   tgaaaatgag acacctgcgc ttcctgccac aggaggttat ctccagtgag accgggatcg 900
   aaatactgga gtttgtggta aataccctaa tgggagacga cccggaaccg ccagtgcagc 960
   ctttcgatcc acctacgctg cacgatctgt atgatttaga gatagacggg ccggaggatc 1020
   ccaatgagga agctgtgaat gggtttttta ctgattctat gctgctagct gctgatgaag 1080
   gattggacat aaaccctcct cctgagacac ttgttacccc aggggtggtt gtggaaagcg 1140
   gcataggtgg gaaaaaattg cctgatctgg gagcagctga aatggacttg cgttgttatg 1200
   aagagggttt tcctcccagt gatgatgaag atggggaaac tgagcagtcc atccataccg 1260
   cagtaaatga gggagtaaaa gctgccagcg atgtttttaa gttggactgt ccggagctgc 1320
   ctggacatgg ctgtaagtct tgtgaatttc acaggaataa cactggaatg aaagaactat 1380
   tgtgctcgct ttgctatatg agaatgcact gccactttat ttacagtaag tgtatttaag 1440
   tgaaatttaa aggaatagtg tagctattta ataactgttg aatggtagat ttatgttttt 1500
   ttcttgcgat tttttgtagg tcctgtgtct gatgatgagt caccttctcc tgattcaact 1560
   acctcacctc ctgaaattca ggcgcccgca cctgcaaacg tatgcaagcc cattcctgtg 1620
   aagcctaagc ctgggaaacg ccctgctgtg gataagcttg aggacttgtt ggagggtggg 1680
   gatggacctt tggaccttag tacccggaaa ctgccaaggc aatgagtgcc ctgcagctgt 1740
   gtttatttaa tgtgacgtca tgtaataaaa ttatgtcagc tgctgagtgt tttattactt 1800
   cttgggtggg gacttggata tataagtagg agcagatctg tgtggttagc tcacagcaac 1860
   ctgctgccat ccatggaggt ttgggctatc ttggaagacc tcagacagac taagctactg 1920
   ctagaaaacg cctcggacgg agtctctggc ctttggagat tctggttcgg tggtgatcta 1980
   gctaggctag tgtttaggat aaaacaggac tacagggaag aatttgaaaa gttattggac 2040
   gatagtccgg gactttttga agctcttaac ttgggtcatc aggctcattt taaggagaag 2100
   gttttatcag ttttagattt ttctactcct ggtagaactg ctgctgctgt agcttttctt 2160
   acttttatat tggataaatg gatccgccaa actcacttca gcaagggata cgttttggat 2220
   ttcatagcag cagctttgtg gagaacatgg aaggctcgca ggatgaggac aatcttagat 2280
   tactggccag tgcagcctct gggagtagca gggatactga gacacccacc gaccatgcca 2340
   gcggttctgc aggaggagca gcaggaggac aatccgagag ccggcctgga ccctccggtg 2400
   gaggagtagc tgacctgttt cctgaactgc gacgggtgct tactaggtct acgaccagtg 2460
   gacagaacag gggaattaag agggagagga atcctagtgg gaataattca agaaccgagt 2520
   tggctttaag tttaatgagc cgcaggcgtc ctgaaactgt ttggtggcat gaggttcaga 2580
   gcgaaggcag ggatgaagtt tcaatattgc aggagaaata ttcactagaa caacttaaga 2640
   cctgttggtt ggaacctgag gatgattggg aggtggccat taggaattat gctaagatat 2700
   ctctgaggcc tgataaacaa tatagaatta ctaagaagat taatattaga aatgcatgct 2760
   acatatcagg gaatggggca gaggttataa tagatacaca agataaagca gtttttagat 2820
   gttgtatgat gggtatgtgg ccaggggttg tcggcatgga agcagtaaca cttatgaata 2880
   ttaggtttaa aggggatggg tataatggca ttgtatttat ggctaacact aagctgattc 2940
   tacatggttg tagctttttt gggtttaata atacgtgtgt agaagcttgg gggcaagtta 3000
   gtgtgagggg ttgtagtttt tatgcatgct ggattgcaac atcaggtagg gtcaagagtc 3060
   agttgtctgt gaagaaatgc atgtttgaga gatgtaatct tggcatactg aatgaaggtg 3120
   aagcaagggt ccgccactgc gcagctacag aaactggctg cttcattcta ataaagggaa 3180
   atgccagtgt gaagcataat atgatctgtg gacattcgga tgagaggcct tatcagatgc 3240
   tgacctgcgc tggtggacat tgcaatattc ttgctaccgt gcatatcgtt tcacatgcac 3300
   gcaaaaaatg gcctgtattt gaacataatg tgattaccaa gtgcaccatg cacataggtg 3360
   gtcgcagggg aatgtttatg ccttaccagt gtaacatgaa tcatgtgaag gtaatgttgg 3420
   aaccagatgc cttttccaga gtgagcttaa caggaatctt tgatatgaat attcaactat 3480
   ggaagatcct gagatatgat gacactaaac caagggtgcg cgcatgcgaa tgcggaggca 3540
   agcatgctag attccagccg gtgtgcgtgg atgtgactga agacttgaga cccgatcatt 3600
   tggtgcttgc ctgcactgga gcggagttcg gttctagtgg tgaagaaact gactaaagtg 3660
   agtagtgggg caagatgtgg atggggactt tcaggttggt aaggtgggca gattgggtaa 3720
   attttgttaa tttctgtctt gcagctgcca tgagtggaag cgcttctttt gaggggggag 3780
   tatttagccc ttatctgacg ggcaggctcc caccatgggc aggagttcgt cagaatgtca 3840
   tgggatccac tgtggatggg agacccgtcc agcccgccaa ttcctcaacg ctgacctatg 3900
   ccactttgag ttcgtcacca ttggatgcag ctgcagccgc cgccgctact gctgccgcca 3960
   acaccatcct tggaatgggc tattatggaa gcatcgttgc caattccagt tcctctaata 4020
   acccttcaac cctggctgag gacaagctac ttgttctctt ggcgcagctc gaggccttaa 4080
   cccaacgctt aggcgaactg tctaagcagg tggcccagtt gcgtgagcaa actgagtctg 4140
   ctgttgccac agcaaagtct aaataaagat ctcaaatcaa taaataaaga aatacttgat 4200
   ataaaacaaa tgaatgttta tttgattttt cgcgcgcggt atgccctgga ccatcggtct 4260
   cgatcattga gaacgcggtg gatcttttcc agtaccctgt aaaggtggga ttgaatgttt 4320
   agatacatgg gcattagtcc gtctcggggg tggagatagc tccattgaag agcctcttgc 4380
   tccggggtag tgttataaat cacccagtca tagcaaggtc ggagtgcatg gtgttgcaca 4440
   atatctttta ggagcagact aattgcaacg gggaggccct tagtgtaggt gtttacaaat 4500
   ctgttgagct gggacgggtg catccggggt gaaattatat gcattttgga ctggatcttg 4560
   aggttggcaa tgttgccgcc tagatcccgt ctcgggttca tattgtgcag gaccaccaag 4620
   acagtgtatc cggtgcactt gggaaattta tcatgcagct tagagggaaa agcatgaaaa 4680
   aatttggaga cgcctttgtg accccccaga ttctccatgc actcatccat aatgatagcg 4740
   atggggccgt gggcagcggc acgggcgaac acgttccggg ggtctgaaac atcatagtta 4800
   tgctcctgag tcaggtcatc ataagccatt ttaataaact ttgggcggag ggtgccagat 4860
   tgggggatga aagttccctc tggcccggga gcatagtttc cctcacatat ttgcatttcc 4920
   caggctttca gttcagaggg ggggatcatg tccacctgcg gggctataaa aaataccgtt 4980
   tctggagccg gggtgattaa ctgggacgag agcaaattcc taagcagctg agacttgccg 5040
   cacccagtgg gaccgtaaat gaccccaatt acgggttgca gatggtagtt tagggaacga 5100
   cagctgccgt cctcccggag caggggggcc acttcgttca tcatttccct tacatggata 5160
   ttttcccgca ccaagtccgt taggaggcgc tctcccccaa gggatagaag ctcctggagc 5220
   gaggagaagt ttttcagcgg cttcagcccg tcagccatgg gcattttgga aagagtctgt 5280
   tgcaagagct cgagccggtc ccagagctcg gtgatgtgct ctatggcatc tcgatccagc 5340
   agacctcctc gtttcgcggg ttgggacggc tcctggagta gggaatcaga cgatgggcgt 5400
   ccagcgctgc cagggtccga tccttccatg gtcgcagcgt ccgagtcagg gttgtttccg 5460
   tcacggtgaa ggggtgcgcg cctggttggg cgcttgcgag ggtgcgcttc agactcatcc 5520
   tgctggtcga gaaccgctgc cgattggcgc cctgcatgtc ggccaggtag cagtttacca 5580
   tgagttcgta gttgagcgcc tcggccgcgt ggcctttggc acggagctta cctttggaag 5640
   ttttatggca ggcggggcag tagatacatt tgagggcata caacttgggc gcgaggaaaa 5700
   tggattcggg ggagtatgca tccgcaccgc aggaggcgca gacggtttcg cactccacga 5760
   gccaggtcag atccggctca tcggggtcaa aaacaagttt tccgccatgt tttttgatgc 5820
   gtttcttacc tttggtttcc atgagttcgt gtccccgctg ggtgacaaag aggctgtccg 5880
   tgtccccgta gaccgacttt atgggcctgt cctcgagcgg agtgcctcgg tcctcttcgt 5940
   agaggaaccc agcccactct gatacaaaag cgcgtgtcca ggccagcaca aaggaggcca 6000
   cgtgggaggg gtagcggtcg ttgtcaacca gggggtccac cttctctacg gtatgtaaac 6060
   acatgtcccc ctcctccaca tccaagaatg tgattggctt gtaagtgtag gccacgtgac 6120
   caggggtccc cgccgggggg gtataaaagg gggcgggcct ctgttcgtcc tcactgtctt 6180
   ccggatcgct gtccaggagc gccagctgtt ggggtaggta ttccctctcg aaggcgggca 6240
   tgacctctgc actcaggttg tcagtttcta ggaacgagga ggatttgata ttgacagtac 6300
   cagcagagat gcctttcata agactctcgt ccatctggtc agaaaacaca atcttcttgt 6360
   tgtccagctt ggtggcaaat gatccataga gggcattgga tagaagcttg gcgatggagc 6420
   gcatggtttg gttcttttcc ttgtccgcgc gctccttggc ggtgatgtta agctggacgt 6480
   actcgcgcgc cacacatttc cattcaggaa agatggttgt cagttcatcc ggaactattc 6540
   tgattcgcca tcccctattg tgcagggtta tcagatccac actggtggcc acctcgcctc 6600
   ggaggggctc attggtccag cagagtcgac ctccttttct tgaacagaaa gggggggagg 6660
   ggtctagcat gaactcatca ggggggtccg catctatggt aaatattccc ggtagcaaat 6720
   ctttgtcaaa atagctgatg gtggcgggat catccaaggt catctgccat tctcgaactg 6780
   ccagcgcgcg ctcatagggg ttaagagggg tgccccaggg catggggtgg gtgagcgcgg 6840
   aggcatacat gccacagata tcgtagacat agaggggctc ttcgaggatg ccgatgtaag 6900
   tgggataaca tcgcccccct ctgatgcttg ctcgcacata gtcatagagt tcatgtgagg 6960
   gggcaagaag acccgggccc agattggtgc ggttgggttt ttccgccctg taaacgatct 7020
   ggcgaaagat ggcatgggaa ttggaagaga tagtaggtct ctggaatatg ttaaaatggg 7080
   catgaggtaa gcctacagag tcccttatga agtgggcata tgactcttgc agcttggcta 7140
   ccagctcggc ggtgatgagt acatccaggg cacagtagtc gagagtttcc tggatgatgt 7200
   cataacgcgg ttggcttttc ttttcccaca gctcgcggtt gagaaggtat tcttcgtgat 7260
   ccttccagta ctcttcgagg ggaaacccgt ctttttctgc acggtaagag cccaacatgt 7320
   agaactgatt gactgccttg tagggacagc atcccttctc cactgggaga gagtatgctt 7380
   gggctgcatt gcgcagcgag gtatgagtga gggcaaaagt gtccctgacc atgactttga 7440
   ggaattgata cttgaagtcg atgtcatcac aggccccctg ttcccagagt tggaagtcca 7500
   cccgcttctt gtaggcgggg ttgggcaaag cgaaagtaac atcattgaag aggatcttgc 7560
   cggccctggg catgaaattt cgggtgattt tgaaaggctg aggaacctct gctcggttat 7620
   tgataacctg agcggccaag acgatctcat caaagccatt gatgttgtgc cccactatgt 7680
   acagttctaa gaatcgaggg gtgcccctga catgaggcag cttcttgagt tcttcaaaag 7740
   tgagatctgt agggtcagtg agagcatagt gttcgagggc ccattcgtgc acgtgagggt 7800
   tcgctttaag gaaggaggac cagaggtcca ctgccagtgc tgtttgtaac tggtcccggt 7860
   actgacgaaa atgctgtccg actgccatct tttctggggt gacgcaatag aaggtttggg 7920
   ggtcctgccg ccagcgatcc cacttgagtt ttatggcgag gtcataggcg atgttgacga 7980
   gccgctggtc tccagagagt ttcatgacca gcatgaaggg gattagctgc ttgccaaagg 8040
   accccatcca ggtgtaggtt tccacatcgt aggtgagaaa gagcctttct gtgcgaggat 8100
   gagagccaat cgggaagaac tggatctcct gccaccagtt ggaggaatgg ctgttgatgt 8160
   gatggaagta gaactccctg cgacgcgccg agcattcatg cttgtgcttg tacagacggc 8220
   cgcagtactc gcagcgattc acgggatgca ccttatgaat gagttgtacc tgacttcctt 8280
   tgacgagaaa tttcagtgga aaattgaggc ctggcgcttg tacctcgcgc tttactatgt 8340
   tgtctgcatc ggcatgacca tcttctgtct cgatggtggt catgctgacg agccctcgcg 8400
   ggaggcaagt ccagacctcg gcgcggcagg ggcggagctc gaggacgaga gcgcgcaggc 8460
   cggagctgtc cagggtcctg agacgctgcg gagtcaggtt agtaggcagt gtcaggagat 8520
   taacttgcat gatcttttgg agggcgtgag ggaggttcag atagtacttg atctcaacgg 8580
   gtccgttggt ggagatgtcg atggcttgca gggttccgtg tcccttgggc gctaccaccg 8640
   tgcccttgtt tttcattttg gacggcggtg gctctgttgc ttcttgcatg tttagaagcg 8700
   gtgtcgaggg cgcgcaccgg gcggcagggg cggctcggga cccggcggca tggctggcag 8760
   tggtacgtcg gcgccgcgcg cgggtaggtt ctggtactgc gccctgagaa gactcgcatg 8820
   cgcgacgacg cggcggttga catcctggat ctgacgcctc tgggtgaaag ctaccggccc 8880
   cgtgagcttg aacctgaaag agagttcaac agaatcaatc tcggtatcgt tgacggcggc 8940
   ttgcctaagg atttcttgca cgtcgccaga gttgtcctgg taggcgatct cggccatgaa 9000
   ctgctcgatc tcttcctctt gaagatctcc gcggcccgct ctctcgacgg tggccgcgag 9060
   gtcgttggag atgcgcccaa tgagttgaga gaatgcattc atgcccgcct cgttccagac 9120
   gcggctgtag accacagccc ccacgggatc tctcgcgcgc atgaccacct gggcgaggtt 9180
   gagctccacg tggcgggtga agaccgcata gttgcatagg cgctggaaaa ggtagttgag 9240
   tgtggtggcg atgtgctcgg tgacgaagaa atacatgatc catcgtctca gcggcatctc 9300
   gctgacatcg cccagcgctt ccaagcgctc catggcctcg tagaagtcca cggcaaagtt 9360
   aaaaaactgg gagttacgcg cggacacggt caactcctct tccagaagac ggataagttc 9420
   ggcgatggtg gtgcgcacct cgcgctcgaa agcccctggg atttcttcct caatctcttc 9480
   ttcttccact aacatctctt cctcttcagg tggggctgca ggaggagggg gaacgcggcg 9540
   acgccggcgg cgcacgggca gacggtcgat gaatctttca atgacctctc cgcggcggcg 9600
   gcgcatggtc tcggtgacgg cacgaccgtt ctccctgggt ctcagagtga agacgcctcc 9660
   gcgcatctcc ctgaagtggt gactgggagg ctctccgttg ggcagggaca ccgcgctgat 9720
   tatgcatttt atcaattgcc ccgtaggtac tccgcgcaag gacctgatcg tctcaagatc 9780
   cacgggatct gaaaaccttt cgacgaaagc gtctaaccag tcgcaatcgc aaggtaggct 9840
   gagcactgtt tcttgcgggc gggggcggct agacgctcgg tcggggttct ctctttcttc 9900
   tccttcctcc tcttgggagg gtgagacgat gctgctggtg atgaaattaa aataggcagt 9960
   tttgagacgg cggatggtgg cgaggagcac caggtctttg ggtccggctt gttggatacg 10020
   caggcgatga gccattcccc aagcattatc ctgacatctg gccagatctt tatagtagtc 10080
   ttgcatgagt cgttccacgg gcacttcttc ttcgcccgct ctgccatgca tgcgagtgat 10140
   cccgaacccg cgcatgggct ggacaagtgc caggtccgct acaacccttt cggcgaggat 10200
   ggcttgctgc acctgggtga gggtggcttg gaagtcgtca aagtccacga agcggtggta 10260
   ggccccggtg ttgattgtgt aggagcagtt ggccatgact gaccagttga ctgtctggtg 10320
   cccagggcgc acgagctcgg tgtacttgag gcgcgagtat gcgcgggtgt caaagatgta 10380
   atcgttgcag gtgcgcacca ggtactggta gccaatgaga aagtgtggcg gtggctggcg 10440
   gtacaggggc catcgctctg tagccggggc tccgggggcg aggtcttcca gcatgaggcg 10500
   gtggtagccg tagatgtacc tggacatcca ggtgataccg gaggcggtgg tggatgcacg 10560
   tgggaactcg cgcacgcggt tccagatgtt gcgcagcggc atgaagtagt tcatggtagg 10620
   cacggtctgg ccagtgaggc gcgcgcagtc attgacgctc tgtagacacg gagaaaacga 10680
   aagcgatgag cggctcgact ccgtggtctg ggggaacgtg aacgggttgg gtcgcggtgt 10740
   accccggttc gagtccaaag ctaagcgatc acgctcggat cggccggagc cgcggctaac 10800
   gtggtattgg ctatcccgtc tcgacccagc cgacgaatat ccagggtacg gagtagagtc 10860
   gtttttgctg ctttttcctg gacgtgtgcc attgccacgt caagctttag aacgctcagt 10920
   tctcgggccg tgagtggctc gcgcccgtag tctggagaat cagtcgccag ggttgcgttg 10980
   cggtatgccc cggttggagc ctaagcgcgg ctcgtatcgg ccggtttccg cgacaagcga 11040
   gggtttggca gccccgttat ttccaagacc ccgccagccg acttctccag tttacgggag 11100
   cgagcccttt tttttttttt tttgtttttg tcgcccagat gcatccagtg ctgcgacaga 11160
   tgcgccccca gcaacaggcc ccttctcagc aacagccaca aaaggctctt cttgctcctg 11220
   caactactgc agctgcagcc gtgagcggcg cgggacagcc cgcctatgat ctggacttgg 11280
   aagagggcga gggattggcg cgcctggggg ctccatcgcc cgagcggcac ccgcgggtgc 11340
   aactaaaaaa ggactctcgc gaggcgtacg tgccccagca gaacctgttc agggacagga 11400
   gcggcgagga gccagaggag atgcgagcat ctcgatttaa cgcgggtcgc gagctgcgcc 11460
   acggtctgga tcgaagacgg gtgctgcaag acgaggattt tgaggtcgat gaagtcacag 11520
   ggatcagccc agctagggca catgtggccg cggccaacct agtctcggcc tacgagcaga 11580
   ccgtgaagga ggagcgcaac ttccaaaaat cttttaacaa ccatgtgcgc accctgatcg 11640
   cccgcgagga agtgaccctg ggtctgatgc atctgtggga cctgatggag gctatcgccc 11700
   aaaaccccac tagcaaacca ctgacagctc agctgtttct ggtggttcaa catagcaggg 11760
   acaacgaggc attcagggag gcgttgttga acatcaccga gcctgatggg agatggctgt 11820
   atgatctgat caacatcctg caaagtatta tagtgcagga acgtagcctg ggtttggctg 11880
   agaaagtggc agctatcaac tactcggtct tgagcctggg caaatactac gctcgcaaga 11940
   tctacaagac cccctacgta cccatagaca aggaggtgaa gatagatggg ttttacatgc 12000
   gcatgactct gaaggtgctg actctgagcg acgatctggg ggtgtatcgc aatgacagga 12060
   tgcaccgcgc ggtgagcgcc agcaggaggc gcgagctgag cgacagagaa cttatgcaca 12120
   gcttgcaaag agctctaacg gggcccggga ctgatgggga gaactacttt gacatgggag 12180
   cggatttgca atggcaaccc agtcgcaggg ccatggaggc tgcagggtgt gagcttcctt 12240
   acatagaaga ggtggatgaa gtcgaggacg aggagggcga gtacttggaa gactgatggc 12300
   gcgacccgta tttttgctag atggaacagc agcaggcacc ggaccccgca atgcgggcgg 12360
   cgctgcagag ccagccgtcc ggcattaact cctcggacga ttggacccag gccatgcaac 12420
   gcataatggc gctgacgacc cgcaaccccg aagcctttag acagcaaccc caggccaacc 12480
   gcctttctgc catactggag gccgtagtgc cctcccgctc caaccccacc cacgagaagg 12540
   tcctggctat cgtgaacgcg ctggtggaga acaaggccat ccgtcccgat gaggccgggc 12600
   tggtatacaa tgctctcttg gagcgcgtgg cccgttacaa cagcagcaac gtgcaaacca 12660
   acctggaccg gatggtgacc gatgtgcgcg aggccgtgtc tcagcgcgag cgattccagc 12720
   gcgacgccaa cttggggtcg ttggtagcgc taaacgcttt cctcagcacc cagcccgcca 12780
   acgtgccccg tggtcagcaa gactatacaa actttttgag tgcattgaga ctcatggtag 12840
   ctgaggtgcc ccagagcgag gtgtaccagt ccgggccaga ttacttcttc cagaccagca 12900
   gacagggctt gcagacagtg aacctgactc aggctttcaa gaacctgaag ggtctgtggg 12960
   gagtgcacgc cccagtaggg gatcgcgcga ccgtgtctag cttgctgact cccaactccc 13020
   gcctgctgct gctgctggta tcccccttta ctgacagcgg tagcattgac cgcaactcgt 13080
   acttgggcta cctgcttaac ctgtatcgcg aggccatagg acagagccag gtggacgagc 13140
   agacctatca agaaatcacc caagtgagcc gcgccctggg tcaggaagac acgggcagtt 13200
   tggaagccac cctgaacttc ttgctaacca accggtcaca gaagatccct cctcagtatg 13260
   cgcttaccgc tgaggaggag cggatcctca gatacgtgca acagagcgtt ggactgttcc 13320
   tgatgcagga gggggcgaca cctaccgccg cgctggacat gacagctcga aacatggagc 13380
   ccagcatgta tgctagtaac aggcctttca ttaacaaact gctggactac ctgcacaggg 13440
   cggccgccat gaactctgat tatttcacca atgctatcct gaacccacac tggctgcccc 13500
   cacctggttt ctacactggc gagtacgaca tgcccgaccc caatgacggg ttcctgtggg 13560
   acgatgtgga cagcagcata ttttccccgc ctcccggtta tacagtttgg aagaaggaag 13620
   ggggcgatag aagacactct tccgtgtcgc tatccggaac ggctggtgct gccgcgaccg 13680
   tgcccgaagc tgcaagtcct ttccctagct tgcccttttc actaaacagc gttcgcagca 13740
   gtgaactggg gagaataacc cgcccgcgct tgatgggcga ggatgagtac ttgaatgact 13800
   ctttgctgag gccagagagg gaaaagaact tccccaacaa tggaatagag agtctggtgg 13860
   ataagatgag tagatggaag acctatgcgc aggatcacag agacgagccc aggatcttgg 13920
   gggctacaag cagaccgatc cgtagacgcc agcgccacga caggcagatg ggtcttgtgt 13980
   gggacgatga ggactctgcc gatgatagca gcgtgttgga cttgggtgga agaggagggg 14040
   gcaacccgtt cgctcatctg cgtcccagat tcgggcgcat gttgtaaaag tgaaagtaaa 14100
   ataaaaaggc aactcaccaa ggccatggcg accgagcgtg cgttcgttct tttttgttat 14160
   ctgtgtctag tacgatgagg agacgagccg tgctaggcgg agcggtggtg tatccggagg 14220
   gtcctcctcc ttcttacgag agcgtgatgc agcaacaggc ggcgatgata cagcccccac 14280
   tggaggctcc cttcgtaccc ccacggtacc tggcgcctac ggaagggaga aacagcattc 14340
   gttactcgga gctgtcgccc ctgtacgata ccaccaagtt gtatctggtg gacaacaagt 14400
   cggcggacat cgcctccctg aactatcaga acgaccacag caacttcctg accacggtgg 14460
   tgcagaacaa tgactttacc cccacggagg ctagcaccca gaccatcaac tttgacgagc 14520
   ggtcgcgatg gggcggtcag ctgaagacca tcatgcacac caacatgccc aacgtgaacg 14580
   agtacatgtt cagcaacaag ttcaaggcga gggtgatggt gtccagaaaa gctcctgaag 14640
   gtgttacagt aaatgacacc tatgatcata aagaggatat cttgaagtat gagtggtttg 14700
   agttcatttt accagaaggc aacttttcag ccaccatgac gatcgacctg atgaacaatg 14760
   ccatcattga caactacctg gaaattggca gacagaatgg agtgctggaa agtgacattg 14820
   gtgttaagtt tgacactaga aatttcaggc tcgggtggga ccccgaaact aagttgatta 14880
   tgccaggtgt ctacacttat gaggcattcc atcctgacat tgtattgctg cctggttgcg 14940
   gggtagactt tactgaaagc cgacttagca acttgcttgg catcaggaag agacatccat 15000
   tccaggaggg tttcaaaatc atgtatgaag atcttgaagg gggtaatatt cctgcccttt 15060
   tggatgtcac tgcctatgag gaaagcaaaa aggataccac tactgaaaca accacactgg 15120
   ctgttgcaga ggaaactagt gaagatgatg atataactag aggagatacc tatataactg 15180
   aaaaacaaaa acgtgaagct gcagctgctg aagttaaaaa agagttaaag atccaacctc 15240
   tagaaaaaga cagcaagagt agaagctaca atgtcttgga agacaaaatc aacacagcct 15300
   accgcagttg gtacctgtcc tacaattacg gtaaccctga gaaaggaata aggtcttgga 15360
   cactgctcac cacttcagat gtcacctgtg gggcagagca ggtctactgg tcgctccctg 15420
   acatgatgca agacccagtc accttccgct ccacaagaca agtcaacaac tacccagtgg 15480
   tgggtgcaga gcttatgccc gtcttctcaa agagtttcta caatgagcaa gccgtgtact 15540
   ctcagcagct ccgacaggcc acttcgctca cgcacgtctt caaccgcttc cctgagaacc 15600
   agatcctcat ccgcccgccg gcgcccacaa ttaccaccgt cagtgaaaac gttcctgctc 15660
   tcacagatca cgggaccctg ccgttacgca gcagtatccg gggagtccag cgcgtgaccg 15720
   ttactgacgc cagacgccgc acctgtccct acgtttacaa ggccctgggc atagtcgcgc 15780
   cgcgcgttct ttcaagccgc actttctaaa aaaaaaaaaa atgtccattc tcatctcgcc 15840
   cagtaataat accggttggg gactgtatgc gcccaccaag atgtatggag gcgcccgcaa 15900
   gcgctctacc cagcatcctg tgcgcgttcg cggtcatttc cgcgctccct ggggcgcact 15960
   caagggtcgt acccgcactc ggaccacggt cgatgatgtg atcgaccagg tggtcgccga 16020
   tgctcgtaat tatactccta ctgcgcctac atctactgtg gatgcagtta ttgacagtgt 16080
   ggtggcagac gcccgcgcct atgctcgccg gaagagccga aggaggcgca tcgccaggcg 16140
   ccacagggct actcccgcca tgcgagccgc aaaagctatt ctgcggaggg ccaaacgtgt 16200
   ggggcgaaga gccatgctta gagcggccag acgcgcggct tcaggtgcca gcagcggcag 16260
   gtcccgcagg cgcgcggcca cggcggcagc agcggccatt gccaacatgg cccaaccgcg 16320
   aagaggcaat gtgtactggg tgcgtgatgc cactaccggc cagcgcgtgc ccgtgcgcac 16380
   ccgcccccct cgcacttaga agatactgag cagtctccga tgttgtgtcc cagcggcaag 16440
   tatgtccaag cgcaaataca aggaagagat gctccaggtc atcgcgcctg aaatctacgg 16500
   tccgccggtg aaggatgaaa aaaagccccg caaaatcaag cgggtcaaaa ataacaaaaa 16560
   ggaagaagat gacgatgatg ggctggtgga gtttgtgcgc gagttcgccc caagacggcg 16620
   cgtgcagtgg cgcgggcgca aagtgcgtca agtgctcaga cccgggacca ctgtggtttt 16680
   tacacctggc gagcgttcca gcactacttt taaacggtcc tatgatgagg tgtacgggga 16740
   tgacgatatt cttgagcagg cggcagaccg ccttgacgag tttgcttatg gcaagcgcac 16800
   tagatccagt cccaaagagg aggctgtgtc cattcctttg gatcatggaa atcccacccc 16860
   cagcctcaaa ccagtcaccc tgcagcaagt gctgcccgtg cctgcgcgga gaggcgtaaa 16920
   gcgcgagggt gaggacctat atcccaccat gcagctaatg gtgcccaagc gccagaggct 16980
   agaagacgta ctggagaaaa tgaaagtgga tgccgatatc cagcctgagg tcaaagtaag 17040
   acctatcaag gaagtggcgc caggtttggg agtacaaacc ttcgacatca agattcccac 17100
   cgagtccatg gaagtgcaga ccgaacctgc aaaacccacc acctcaattg aggtgcaaac 17160
   ggaaccctgg atgcccgcgc ccgttgccgc ccccagcacc actcgaagat cacgacgaaa 17220
   gtacggccca gcaagtctgc taatgcccaa ctatgctctg cacccatcca tcattcccac 17280
   tccgggttac agaggcactc gctactatcg aaaccggagc aacacctctc gccgccgcaa 17340
   accacctgca agtcgcactc gccgtcgccg ccgccgcaac actgccagca aattgactcc 17400
   cgccgccctg gtgcggagag tgtaccgcga tggtcgcgct gaacctctga cgctgccgcg 17460
   cgcgcgctac catccaagca tcaccactta atgactgttg acgctgcctc cttgcagata 17520
   tggccctcac ttgccgcctt cgcgtcccca ttactggcta ccgaggaaga aactcgcgcc 17580
   gtagaaggat gttggggcga gggatgcgcc gccacagacg aaggcgcgct atcagcagac 17640
   gattaggggg tggctttttg ccagctctta tacccatcat cgccgcagcg atcggggcga 17700
   taccaggcat agcttcagtg gcggttcagg cctcgcagcg ccactaacat tggaaaaaac 17760
   ttataaataa aaaatagaat ggactctgac gctcctggtc ctgtgactat gtttttgtag 17820
   agatggaaga catcaatttt tcatccctgg ctccgcgaca cggcacgagg ccgtacatgg 17880
   gcacctggag cgacatcggc acgagccaac tgaacggggg cgccttcaat tggagcagta 17940
   tctggagcgg gcttaaaaat tttggctcga ccgtaaaaac ctatgggaac aaagcttgga 18000
   acagcagcac agggcaggct ctgagaaata agcttaagga acaaaacttc caacagaagg 18060
   tggtcgatgg gatcgcctct ggtattaacg gcgtagtgga tctggccaac caggctgtac 18120
   aaaaacagat aaacagccgc ctggacccgc cgcccgcaac ccctggtgaa atggaagtgg 18180
   aggaagaact tcctccgctg gaaaagcggg gcgacaagcg tccgcgaccc gagctagagc 18240
   agacgctggt gacgcgcgca gacgagcccc cttcatatga ggaggcagta aagctcggaa 18300
   tgcccactac caggcctgta gctcacatgg ctaccggggt gatgaaacct tctcagtcac 18360
   atcgacccgc caccttggac ttgcctcctc cccctgcttc tgcggcgcct gttcccaaac 18420
   ctgtcgctac cagaaagccc accgccgtac agcccgtcgc cgtagccaga ccgcgtcctg 18480
   ggggcacacc gcgcccgaaa gcaaactggc aaagtactct gaacagcatc gtgggtctgg 18540
   gcgtgcagag tgtaaagcgc cgtcgctgct attaattaaa tatggagtag cgcttaactt 18600
   gcttgtctgt gtgtatgtat catcaccacg ccgccgcagc agaggagaaa ggaagaggtc 18660
   gcgcgccgag gctgagttgc tttcaagatg gccaccccat cgatgatgcc ccaatgggca 18720
   tacatgcaca tcgccggaca ggatgcttcg gagtacctca gtccgggtct ggtgcagttc 18780
   gcccgtgcaa cagacaccta cttcagtatg gggaacaaat ttagaaaccc cacagtggcg 18840
   cccacccacg atgtgaccac cgaccgtagc cagcgcctga tgctgcgctt cgtgcccgtt 18900
   gaccgggaag acaataccta ctcttacaaa gttcgctaca cgctggctgt aggcgacaac 18960
   agagtgcttg acatggccag cacattcttt gacattcggg gggtgcttga tagaggtcct 19020
   agcttcaagc catattccgg cacagcttac aattcactcg ctcctaaggg cgcgcccaat 19080
   acatctcagt ggatagttac aacaaatggg gacaatgcag taactaccac cacaaacaca 19140
   tttggcattg cttccatgaa gggagacaat attactaaag aaggtttgca aattgggaaa 19200
   gacattacca ctactgaagg agaagaaaag cccatttatg ccgataaaac atatcagcca 19260
   gagcctcaag ttggagaaga atcatggact gatactgatg gaacaaatga aaagtttggt 19320
   ggaagagccc ttaaaccagc taccaacatg aagccatgct acgggtcttt tgcaagacct 19380
   acaaacataa aagggggcca agctaaaaac agaaaagtaa aaccaacaac cgaaggaggg 19440
   gttgaaactg aggaaccaga tattgatatg gaatttttcg atggtagaga tgctgttgca 19500
   ggagctttag cgcctgaaat tgtgctttat acggaaaatg taaatttgga aactccagac 19560
   agtcatgtgg tatataaacc agaaacgtct aataactctc atgcaaattt gggtcaacaa 19620
   gccatgccta acagacccaa ttacattggc ttcagggata acttcgtagg cctaatgtac 19680
   tacaacagta ctggaaatat gggagttttg gctggccaag catcacaact gaatgcagtg 19740
   gttgacttgc aggacagaaa tactgaactg tcatatcagc ttttgcttga ttctctggga 19800
   gacagaacca gatacttcag catgtggaat caggctgtgg acagttacga tcccgatgtt 19860
   cgcattattg aaaatcatgg catcgaggat gaactgccta attactgttt tcctctgaat 19920
   ggcataggac cagggcacac atatcaaggc attaaagtta aaaccgatga cactaatgga 19980
   tgggaaaaag atgctaatgt tgctccagct aatgaaataa ccataggcaa caacctggct 20040
   atggaaatta atatccaagc taacctttgg agaagttttc tgtactctaa tgtggctttg 20100
   taccttccag atgtttacaa gtacacgcca cctaacatta ctttgcccac taacaccaac 20160
   acctatgagt acatgaacgg gcgagtggta tccccatccc tggttgattc atacatcaac 20220
   attggcgcca ggtggtctct tgacccaatg gacaatgtga atccattcaa ccaccaccgc 20280
   aatgctggtc tgcgctacag gtccatgctt ctgggaaatg gtcgttatgt gcctttccac 20340
   atacaagtgc ctcagaaatt ctttgctgtc aagaacctac ttcttctacc tggctcctac 20400
   acctacgagt ggaacttccg aaaggatgtg aacatggtcc tgcaaagttc ccttggaaat 20460
   gacctcagaa cggatggtgc taccataagt ttcaccagca tcaatctcta tgccaccttc 20520
   ttccccatgg ctcacaacac agcttccacc cttgaagcca tgctgcgcaa cgataccaat 20580
   gatcagtcat ttaacgacta cctctctgca gctaacatgc tttaccccat tcctgccaat 20640
   gcaaccaaca ttccaatttc catcccatct cgcaactggg cagccttcag gggctggtcc 20700
   ttcaccagac tcaaaaccaa ggagactcca tctcttggat cagggttcga tccctacttc 20760
   gtatattctg gatctattcc ctacctggat ggcacctttt accttaacca cactttcaag 20820
   aaggtctcca tcatgtttga ctcctcagtc agctggcctg gcaatgacag gctgttgagt 20880
   ccaaatgagt ttgaaatcaa gcgcactgtg gacggggaag gatacaacgt ggcacaatgc 20940
   aacatgacca aagactggtt cctggttcag atgcttgcca attacaacat tggctaccag 21000
   ggcttttaca tccctgaggg atacaaggat cgcatgtact cctttttcag aaacttccag 21060
   cctatgagca ggcaggtggt tgatgaggtt aattacactg actacaaagc cgtcacctta 21120
   ccataccaac acaacaactc tggctttgta gggtaccttg cacctactat gagacaaggg 21180
   gaaccttacc cagccaatta tccatacccg ctcatcggaa ctactgccgt taagagtgtc 21240
   acccagaaaa agttcctgtg cgacaggacc atgtggcgca ttcccttctc cagcaacttc 21300
   atgtccatgg gggcccttac cgacctggga cagaacatgc tctatgccaa ctcagcccat 21360
   gcgctggaca tgacttttga ggtggatccc atggatgagc ccaccctgct ttatcttctt 21420
   ttcgaagtct tcgacgtggt cagagtgcac cagccacacc gcggcgtcat cgaggccgtc 21480
   tacctgcgca caccgttctc ggccggcaac gccaccacat aagaagcctc ttgcttcttg 21540
   caagcagcag ctgcagccat gacatgcggg tccggaaacg gctccagcga gcaagagctc 21600
   aaagccatcg tccgagacct gggctgcgga ccctatttcc tgggaacctt tgacaagcgt 21660
   ttcccggggt tcatggcccc cgacaagctc gcctgcgcca tagtcaacac tgccggacgc 21720
   gagacggggg gagagcactg gctggctttt ggttggaacc cgcgctacaa cacctgctac 21780
   ctttttgatc cttttgggtt ctcggatgag cggctcaaac agatttacca gtttgagtac 21840
   gaggggctcc tgcgtcgcag tgcccttgct accaaagacc gctgcatcac cctggagaag 21900
   tctacccaaa gcgtgcaggg tccgcgctca gccgcctgtg gacttttttg ctgtatgttc 21960
   cttcatgcct ttgtgcactg gcccgaccgc cccatggacg gaaaccccac catgaagttg 22020
   ctgactgggg tgtccaacag catgctccaa tcaccccaag tccagcccac cctgcgccgc 22080
   aaccaggagg tgctataccg cttcctaaac acccactcat cttactttcg ttctcaccgc 22140
   gcgcgcattg aaagggccac cgcgtttgac cgtatggata tgcaataagt catgtaaaac 22200
   cgtgttcaat aaacagcact ttatttttac atgcactgag gctctggttt tgctcatttg 22260
   tttcatcatt tactcagaag tcgaatgggt tctggcggga gtcagagtga cccgcgggca 22320
   gggatacgtt gcggaactgt aacctgttct gccacttgaa ctcggggatt accagcttgg 22380
   gaactggaat ctcgggaaag gtgtcttgcc acaactttct ggtcagttgc atagcgccaa 22440
   gcaggtcagg agcagagatc ttgaaatcac agttggggcc ggcattctgg acacgggagt 22500
   tgcgatacac tgggttgcaa cactggaaca ctatcaacgc tgggtgtctt acgcttgcca 22560
   acacggttgg gtcactgatg gtagtcacat ccaagtcttc agcattggcc atcccaaagg 22620
   gggtcatctt acatgtctgc ctgcccatca cgggagcgca gcctggcttg tggttgcaat 22680
   cacaatgaat ggggatcagc atcatcctgg cttggtcggg agttatccct gggtacacag 22740
   ccttcatgaa ggcttcgtac tgcttaaaag cttcctgggc cttacttccc tcggtgtaga 22800
   acatcccaca ggacttgctg gaaaattgat tagtagtaca gttggcatca ttcacacaac 22860
   agcgggcatc gttgttggcc aactgaacca catttctgcc ccagcggttt tgggtgatct 22920
   tggctctgtc tggattctcc ttcatagcgc gctgcccgtt ctcgctcgcc acatccatct 22980
   cgataatgtg gtccttctgg atcatgatag tgccatgcag gcatttcacc ttgccttcat 23040
   aatcggtgca tccatgagcc cacagagcgc acccggtgca ctcccaatta ttgtgggcga 23100
   tctcagaata ataatgtacc aatccctgca tgaatcttcc catcattgtt gtcaaggtct 23160
   tcatgctggt aaatgtcagc gggatgccac ggtgctcctc gttcacatac tggtggcaga 23220
   tacgcttgta ttgctcgtgc tgctctggca tcagcttgaa agaggttctc agatcattat 23280
   ccagcctgta cctttccatt agcacagcca tcacttccat gcccttctcc caggcagata 23340
   ccaggggcag actcaaagga ttcctaacag caataaaagt agctccttta gctatagggt 23400
   cattcttgtc gatcttctca acacttctct tgccatcctt ctcaatgatg cgcaccgggg 23460
   ggtagctgaa gcccacggcc accaactgag cctgttctct ttcttcttca ctatcctggc 23520
   tgatgtcttg cagagggaca tgcttggtct tcctgggctt cttcttggga gggatcgggg 23580
   gaggactgtt gctccgctcc ggagacaggg atgactgcga agtttcgctt accaatacca 23640
   cctggctctc ggtagaagaa ccggacccca cacgacggta ggtgttcctc ttcgggggca 23700
   gaggtggagg cgactgagat gggctgcggt ctggccttgg aggcggatgg ctggcagagc 23760
   tcattccgcg ttcgggggtg tgctcccggt ggcggtcgct tgactgattt cctccgcggc 23820
   tggccattgt gttctcctag gcagagaaac aacagacatg gaaactcagc catcactgcc 23880
   aacatcgctg caagcaccat cacacctcgc ccccagcagc gacgaggagg agagcttaac 23940
   caccccacca cccagtcccg ctaccaccac ctctaccctc gatgatgagg aggaggtcga 24000
   cgcagcccag gagatgcagg cgcaggataa tgtgaaaacg gaagagattg aggcagatgt 24060
   cgagcaggac ccgggctatg tgacgccggc ggagcaccag gaggagctga aacgctttct 24120
   agacagagag gatgacgacc gcccagagca tcaagcagat ggcgtttacc aggaggctgg 24180
   gatcagggat catgtcgccg actacctcac cggccttggt ggggaggacg tgctcctcaa 24240
   acatctagca aggcagtcga tcatagttaa agacgcattg ctcgatctca ctgaagtgcc 24300
   catcagtgtg gaagagctta gccgcgccta cgagctgaac ctcttttcgc ctcaggtacc 24360
   ccccaagcgg cagccaaacg gcacctgcga ggccaaccct cgactcaact tctatccagc 24420
   ttttactatc cccgaagtgt tggccaccta ccacatcttt ttcaagaacc aaaagattcc 24480
   agtctcctgc cgcgccaacc gcacccgcgc cgatgccctg ctcaacttgg gtccgggagc 24540
   tcgcttacct gatatagctt ccttggaaga ggttccaaag atctttgagg gtctgggaag 24600
   tgatgagaca cgggccgcaa atgctctgca acagggagag aatgacatgg atgaacacca 24660
   cagcgctctg gtggaactgg agggtgacaa tgcccggatt gcagtgctca agcgcagtat 24720
   cgtggtcacc cattttgcct accccgctgt taacctgccc cccaaagtta tgagcgctgt 24780
   catggaccat ctgctcatca aacgagcaag acctctttca gaaaaccaga acatgcagga 24840
   tccagacgcc tcggacgagg gcaagccggt agtcagtgac gagcagctat ctcgctggct 24900
   gggtaccaac tccccccgag atttggaaga gaggcgcaag cttatgatgg ctgtagtgct 24960
   agtaactgtg gagctggagt gtctgcgccg ctttttcacc gaccctgaga ccctgcgcaa 25020
   gctagaggag aacctgcact acacctttag acatggcttc gtgcggcagg catgcaagat 25080
   ctccaacgtg gagcttacca acctggtttc ttacatgggc attttgcatg agaaccgact 25140
   agggcagagc gtcctgcaca ccacccttaa aggggaggcc cgccgtgact acatccgaga 25200
   ctgtgtctac ctctacctct gccatacctg gcaaactggt atgggtgtgt ggcaacagtg 25260
   tttggaagag cagaacctaa aagagctgga caagctcttg cagagatccc tcaaagccct 25320
   gtggacaggt tttgatgagc gcaccgtcgc ctcggacctg gcagacatca tcttccccga 25380
   gcgtctcagg gttactctgc gaaacggcct gccagacttt atgagccaga gcatgcttaa 25440
   caactttcgc tctttcatcc tggaacgctc cggtatcctg cctgccacct gctgtgcgct 25500
   gccctccgac tttgtgcctc tcacctaccg cgagtgccca ccgccgctat ggagccactg 25560
   ctacctgttc cgcctggcca actacctctc ctaccactcg gatgttatag aggatgtgag 25620
   cggagacggc ctgctggaat gccactgccg ctgcaatctt tgcacacccc accgctccct 25680
   tgcctgcaac ccccagttgc tgagcgagac ccagattatc ggcaccttcg agctgcaggg 25740
   tcccagaagt aaaggcgagg ggtcttctcc ggggcagagt ttgaaactga caccggggct 25800
   gtggacctcc gcctacctgc gcaagtttca ccccgaggac taccatccct atgagatcag 25860
   gttctatgag gaccaatcac atcctcccaa agtcgagctc tcagcctgcg tcatcaccca 25920
   gggagcaatt ctggcccaat tgcaagccat ccaaaaatct cgccaagaat ttctgctaaa 25980
   aaagggaaac ggggtctacc ttgaccctca gaccggtgag gagctcaaca caaggttccc 26040
   ccaggatgtc ccatcgccga ggaagcaaga agttgaaggt gcagctgtcg cccccagagg 26100
   atatgaagga agactgggac agtcaggcag aggaggagat ggaagattgg gacagccagg 26160
   cagaggaggt ggacagcctg gaggaagaca gtttggagga ggaagacgag gaggcagagg 26220
   aggtggaaga agcaaccgcc gccaaacagt tgtcatcggc ggcggagaca agcaagtccc 26280
   cagacagcag cacggctacc atctccgctc cgggtcgggg ggcccagcgg cggcccaaca 26340
   gtagatggga cgagaccggg cgattcccaa acccgaccac cgcttccaag accggtaaga 26400
   aggagcgaca gggatacaag tcctggcgtg gacataaaaa cgctatcatc tcctgcttgc 26460
   atgaatgcgg gggcaacata tccttcaccc ggcgatacct gctcttccac cacggtgtaa 26520
   acttcccccg caatatcttg cattactacc gtcacctcca cagcccctac tgcagtcagc 26580
   aagtcccggc aaccccgaca gaaaaataca gcagcgacaa cggtgaccag aaaaccagca 26640
   gttagaaaat ccacaacaag tgcaccagga ggaggactga ggatcacagc gaacgagcca 26700
   gcgcagacca gagagctgag gaaccggatc tttccaaccc tctatgccat tttccagcag 26760
   agtcgggggc aagagcagga actgaaagta aaaaaccgat ctctgcgctc gctcaccaga 26820
   agttgtttgt atcacaagag cgaagaccaa cttcagcgca ctctcgagga cgccgaggct 26880
   ctcttcaaca agtactgcgc gctgactctt aaagagtagc ccttgcccgc gctcattttg 26940
   aaaacggcgg gaatcacgtc acccttggca cctgtccttt gcccttgtca tgagtaaaga 27000
   gattcccacg ccttacatgt ggagctatca gccccaaatg gggttggcag caggcgcttc 27060
   ccaggactac tccacccgca tgaattggct tagcgccggg ccctcaatga tatcacgggt 27120
   taatgatata cgagcttatc gaaaccagtt actcctagaa cagtcagctc tcaccaccac 27180
   accccgtcaa caccttaatc cccgaaattg gcccgccacc ctggtgtacc aggaaaatcc 27240
   cgctcccacc accgtactac ttcctcgaga cgcccaggcc gaagttcaga tgactaacgc 27300
   aggtgtacag ctggcgggcg gttccgccct atgtcgtcac cgacctcaac agagtataaa 27360
   acgcctggtg attagaggcc gaggtatcca gctcaacgac gagtcggtta gctcttcgct 27420
   tggtctgcga ccagacggag tcttccaaat cgccggctgt gggagatctt ccttcactcc 27480
   tcgtcaggct gtgctgactt tggagagttc gtcctcgcag ccccgctcgg gcggcattgg 27540
   aactctccag tttgtggagg agtttactcc ctctgtctac ttcaacccct tctccggctc 27600
   tcctggccag tacccggacg agttcatacc aaacttcgac gcaatcagcg agtcagtgga 27660
   tggctatgat tgatgtctaa tggtggtgcg gctgagctag ctcgactgcg acacctagac 27720
   cactgccgcc gctttcgctg cttcgcccgg gaactcaccg agttcatcta cttcgaactc 27780
   tccgaggagc accctcaggg tccggcccac ggagtgcgga ttaccatcga agggggaata 27840
   gactctcgcc tgcatcgcat cttctcccag cggcccgtgc taattgaacg cgaccaggga 27900
   aatacaacca tctccatcta ctgcatctgt aaccaccccg gattgcatga aagcctttgc 27960
   tgtcttgttt gtgctgagtt taataaaaac tgagttaaga ccctcctacg gactaccgct 28020
   tcttcaatca ggactttaca acaccaacca gatcttccag aagacccaga cccttcctcc 28080
   tttcatccag gactctaact ctaccttacc agcaccctcc actactaacc ttcccgaaac 28140
   aaacaagctt gcatctcatc tgcaacaccg cctttcacga agccttcttt ctgccaatac 28200
   taccactccc aaaaccggag gtgagctccg cggtcttcct actgacgacc cctgggtggt 28260
   agcgggtttt gtaacgttag gagtagttgc gggtgggctt gtgctgatcc tttgctacct 28320
   atacacacct tgctgtgcat atttagtcat attgtgctgt tggtttaaga aatgggggcc 28380
   atactagtcg tgcttgcttt actttcgctt ttgggtctgg gctctgctaa tctcaatcct 28440
   ctcgatcacg atccatgttt agacttcgac ccagaaaact gcacacttac ttttgcaccc 28500
   gacacaagcc gtctctgtgg agttcttatt aagtgcggat gggactgcag gtccgttgaa 28560
   attacacata ataacaaaac atggaacaat accttatcca ccacatggga gccaggagtt 28620
   ccccagtggt atactgtctc tgtccgaggt cctgacggtt ccatccgcat tagtaacaac 28680
   actttcattt tttctgaaat gtgcgatctg gccatgttca tgagcagaca gtatgaccta 28740
   tggcctccca gcaaagagaa cattgtggca ttttccattg cttattgctt ggtaacatgc 28800
   atcatcactg ctatcatttg tgtgtgcata cacttgctta tagttattcg ccctagacaa 28860
   agcaatgagg aaaaagagaa aatgccttaa cctttttcct catacctttt ctttacagca 28920
   tggcttctgt tacagctcta attattgcca gcattgtcac tgtcgctcac gggcaaacaa 28980
   ttgtccatat taccttagga cataatcaca ctcttgtagg gcccccaatt acttcagagg 29040
   ttatttggac caaacttgga agtgttgatt attttgatat aatttgcaac aaaactaaac 29100
   caatatttgt aatctgtaac agacaaaatc tcacgttaat taatgttagc aaaatttata 29160
   acggttacta ttatggttat gacagatcca gtagtcaata taaaaattac ttagttcgca 29220
   taactcagcc caaattaaca gtgccaacta tgacaataat taaaatggct aataaagcat 29280
   tagaaaattt tacatcacca acaacaccca atgaaaaaaa cattccaaat tcaatgattg 29340
   caattattgc ggcggtggca ttgggaatgg cactaataat aatatgcatg ctcctatatg 29400
   cttgttacta taaaaagttt caacataaac aggatccact actaaatttt aacatttaat 29460
   tttttataca gatgatttcc actacaattt ttatcattac tagccttgca gctgtaactt 29520
   atggccgttc acacctaact gtacctgttg gctcaacatg tacactacaa ggaccccaag 29580
   aaggctatgt cacttggtgg agaatatatg ataatggagg gttcgctaga ccatgtgatc 29640
   agcctggtac aaaattttca tgcaacggaa gagacttgac cattattaac ataacatcaa 29700
   atgagcaagg cttctattat ggaaccaact ataaaaatag tttagattac aacattattg 29760
   tagtgccagc caccacttct gctccccgca aatccacttt ctctagcagc agtgccaaag 29820
   caagcacaat tcctaaaaca gcttctgcta tgttaaagct tccaaaaatc gctttaagta 29880
   attccacagc cgctcccaat acaattccta aatcaacaat tggcatcatt actgccgtgg 29940
   tagtgggatt aatgattata tttttgtgta taatgtacta cgcctgctgc tatagaaaac 30000
   atgaacaaaa aggtgatgca ttactaaatt ttgatattta attttttata gaattatgat 30060
   attgtttcaa tcaaatacca ctacctccta tgcatacaca aacattcagc ctaaatacgc 30120
   tatgcaacta gaaatcacaa tactaattgt aattggaatt cttatactat ctgttattct 30180
   ttattttata ttctgccgtc aaatacccaa tgttcataga aattctaaaa gacgtcccat 30240
   ctattctcct atgattagtc gtccccatat ggctctgaat gaaatctaag atcttttttt 30300
   ttttctctta cagtatggtg aacatcaatc atgatcccta gaaatttctt cttcaccata 30360
   ctcatctgtg cttttaatgt ctgtgctact ttcacagcag tagccactgc aagcccagac 30420
   tgtataggac catttgcttc ctatgcactt tttgccttcg ttacttgcat ctgcgtgtgt 30480
   agcatagtct gcctggttat taattttttc caactggtag actggatctt tgtgcgaatt 30540
   gcctacctac gtcaccatcc cgaataccgc aatcaaaatg ttgcggcact tcttaggctt 30600
   atttaaaacc atgcaggcta tgctaccagt cattttaatt ttgctactac cctgcattcc 30660
   cctagcttcc accgccactc gcgctacacc tgaacaactt agaaaatgca aatttcaaca 30720
   accatggtca tttcttgatt gctaccatga aaaatctgat tttcccacat actggatagt 30780
   gattgttgga ataattaaca tactttcatg tacctttttc tcaatcacaa tataccccac 30840
   atttaatttt gggtggaatt ctcccaatgc actgggttac ccacaagaac cagatgaaca 30900
   tattccacta caacacatac aacaaccact agcactggta cagtatgaaa atgagccaca 30960
   accttcactg ccccctgcca ttagttactt caacctaacc ggcggagatg actgacccaa 31020
   tcgccacatc atccaccgct gccaaggagc tgctggacat ggacggacgt gcctcagaac 31080
   agcgactcat ccaactacgc attcgtcagc agcaggaacg agcagtaaaa gagctaaggg 31140
   atgccattgg gattcaccag tgcaaaaaag gcatattctg cttagtaaaa caatccaaaa 31200
   tctcctacga gatcaccgct actgaccatc gtctctcata cgagctcggt ccgcagcgac 31260
   aaaaattcac ctgcatggtg ggaatcaacc ccatagttat cacccagcag tctggagata 31320
   ctaagggttg tatccagtgt tcctgtgatt ccaccgagtg catctacaca ctgctgaaga 31380
   ccctctgcgg ccttcgagac ctcctaccca tgaactaatc attgccccta ccttacccaa 31440
   tcaaaatatt aataaagaca cttacttgaa atcagcaata cagtctttgt caaaactttc 31500
   taccagcagc acctcaccct cttcccaact ctggtactct aaacgtcgga gggtggcata 31560
   ctttctccac actttgaaag ggatgtcaaa ttttatttcc tcttctttgc ccacaatctt 31620
   catttcttta tccccagatg gccaagcgag ctcggctaag cacttccttc aacccggtgt 31680
   acccttatga agatgaaagc agctcacaac acccatttat aaatcctggt ttcatttccc 31740
   ctgacgggtt cacacaaagt ccaaacgggg ttttaagtct taaatgtgtt aatccactta 31800
   ccactgcaag cggctccctc caacttaaag tgggaagtgg tcttacagta gacactactg 31860
   atggatcctt agaagaaaac atcaaagtta acacccccct aacaaagtca aaccattcta 31920
   taaatttacc aataggaaac ggtttgcaaa tagaacaaaa caaactttgc agtaaactcg 31980
   gaaatggtct tacatttgac tcttccaatt ctattgcact gaaaaataac actttatgga 32040
   caggtccaaa accagaagcc aactgcataa ttgaatacgg gaaacaaaac ccagatagca 32100
   aactaacttt aatccttgta aaaaatggag gaattgttaa tggatatgta acgctaatgg 32160
   gagcctcaga ctacgttaac accttattta aaaacaaaaa tgtctccatt aatgtagaac 32220
   tatactttga tgccactggt catatattac cagactcatc ttctcttaaa acagatctag 32280
   aactaaaata caagcaaacc gctgacttta gtgcaagagg ttttatgcca agtactacag 32340
   cgtatccatt tgtccttcct aatgcgggaa cacataatga aaattatatt tttggtcaat 32400
   gctactacaa agcaagcgat ggtgcccttt ttccgttgga agttactgtt atgcttaata 32460
   aacgcctgcc agatagtcgc acatcctatg ttatgacttt tttatggtcc ttgaatgctg 32520
   gtctagctcc agaaactact caggcaaccc tcataacctc cccatttacc ttttcctata 32580
   ttagagaaga tgactgacaa caaaaataaa gttcaacatt ttttattgaa attcctttta 32640
   cagtattcga gtagttattt tgcctccccc ttcccattta acagaataca ccaatctctc 32700
   cccacgcaca gctttaaaca tttggatacc attagagata gacatagttt tagattccac 32760
   attccaaaca gtttcagagc gagccaatct ggggtcagta atacataaaa atgcatcggg 32820
   atagtctttt aaagcgcttt cacagtccaa ctgttgcgga tgcgactccg gagtctgaat 32880
   cacggtcatc tggaagaaga acgatgggaa tcataatccg aaaacggaat cgggcgattg 32940
   tgtctcatca aacccacaag caaccgctgt ctgcgtcgct ccgtgcgact gctgtttatg 33000
   ggatcggggt ccgcagtgtc ctgaagcatg attttaatag cccttaacat taactttctg 33060
   gtgcgatgcg cgcagcaacg cattctgatt tcacttagat tactacagta ggtacagcac 33120
   attatcacaa tattgtttaa taaaccataa ttaaaagcgc tccagccaaa actcatatct 33180
   gatataatcg cccctgcatg accatcatac caaagtttaa tataaattaa atgtcgttcc 33240
   ctcaaaaaca cactacccac atacatgatc tcttttggca tgtgcatatt aacaatttgt 33300
   ctgtaccatg gacaacgttg gttaatcatg caacccaata taaccttccg gaaccacact 33360
   gccaacaccg ctcccccagc catgcattga agtgaaccct gctgattaca atgacaatga 33420
   agaacccaat tctctcgacc atgaatcact tgagactgaa aaatatctat agtagcacaa 33480
   caaagacata aatgcatgca tcttctcata atttttaact cctctggatt taaaaacata 33540
   tcccaaggaa tgggaaactc ttgcagaaca gtaaagctgg cagaacaagg aagaccacga 33600
   acacaactta cactatgcat agtcatagta tcacaatctg gcaacagcgg gtggtcttca 33660
   gtcatagaag ctcgggtttc attttcctca catcgtggta attgggctct ggtgtaaggg 33720
   tgatgtctgg cgcatgatgt ggagcgtgcg cgcaaccttg tcataatgga gttgcttcct 33780
   gacattctcg tattttgtat agcaaaacgc tgccctggca caacacactc ttcttcgtct 33840
   tctatcctgc cgcttagtgt gttccgtctg ataattcaag tacagccaca ctcttaagtt 33900
   ggtcaaaaga atgctggctt cagttgtaat caaaactcca tcatatttaa ttgttctaag 33960
   gaaatcatcc acggtagcat atgcaaatcc caaccaagca atgcaactgg attgcgtttc 34020
   aagcagcaga ggagagggaa gagacggaag aatcatgtta atttttattc caaacgatct 34080
   cgcagtactt caaattgtag atcgcgcaga tggcatctat cgcccccact gtgttggtga 34140
   aaaagcacag ctaaatcaaa agaaatgcga ttttcaaggt gctcaacggt ggcttccaac 34200
   aaagcctcca cgcgcacatc caaaaacaaa agaataccaa aagaaggagc attttctaac 34260
   tcctcaaaca tcatattaca ttcctgcacc attcccagat aattttcagc tttccagcct 34320
   tgaattattc gtgtcagttc ttgtggtaaa tccaaaccac acattacaaa caggtcccgg 34380
   agggcgccct ccaccaccat tcttaaacac accctcataa tgacaaaata tcttgctcct 34440
   gtgtcacctg tagcaaatta agaatggcat catcaattga catgcccttg gctctaagtt 34500
   cttctctaag ttctagttgt agatactctc tcatattatc accaaactgc ttagccagaa 34560
   gccccccggg aacaatagca ggggacgcta cagtgcagta caagcgcaga cctccccaat 34620
   tggctccagc aaaaacaaga ttagaataag catactggga accaccagta atatcatcaa 34680
   agttgctgga aatataatca ggcagagttt cttgtaaaaa ttgaataaaa gaaaaatttt 34740
   ccaaagaaac attcaaaacc gttgggatgc aaatacaata ggttaccgcg ctgcgctcca 34800
   acattgttag ttttgaatta gtctgcaaaa taaaagaaac aagcgtcata tcatagtagc 34860
   ctgtcgaaca ggtggaaaaa tcagtctttc catcacaaga caagccacag ggtctccagc 34920
   tcgaccctcg taaaacctgt cattgtgatt aaacaacagc accgaaagtt cctcgcggtg 34980
   gccagcatga ataattcttg atgaagcata caatccagac atgttagcat cagttaaaga 35040
   gaaaaaacag ccaacatagc ctctgggtat aattatgctt aattttaagt atagcaaagc 35100
   cacccctcgc ggatacaaag taaaaggcac aggagaataa aaaatataat tatttctctg 35160
   ctgctgttca ggcaacgttg ctcccggtcc ctctaaatag acatacaaag cctcatcagc 35220
   catggcttac caggcaaagt acagcgggcg cacaaagcac aagctctaaa gaagctctaa 35280
   aaacactctc caacctctcc acaatatata cacaagccct aaactgacgt aatgggagta 35340
   aagtgaaaaa aaaataccgc caagcccaac acacaccccg aaactgcgtc agcaggaaaa 35400
   agtacagttt cacttccgca ttcccaacaa gcgtaacttc ctctttctca tggtacgtca 35460
   catccgatta acttgcaacg tcattttccc acggtcgcgc cgcccctttt agccgttaac 35520
   cccgcagcca atcaccacac agcgcgcact tttttaaatt acctcattta catgttggca 35580
   ccattccatc tataaggtat attatataga tag 35613
<210> 19
   <211> 254
   <212> DNA
   <213> Artificial Sequence
<220>
   <223> hTERT
<400> 19
   ctgcgctgtc ggggccaggc cgggctccca gtggattcgc gggcacagac gcccaggacc 60
   gcgcttccca cgtggcggag ggactgggga cccgggcacc cgtcctgccc cttcaccttc 120
   cagctccgcc tcctccgcgc ggaccccgcc ccgtcccgac ccctcccggg tccccggccc 180
   agccccctcc gggccctccc agcccctccc cttcctttcc gcggccccgc cctctcctcg 240
   cggcgcgagt ttca 254
<210> 20
   <211> 36923
   <212> DNA
   <213> Artificial Sequence
<220>
   <223> Ad3-hTERT-CD40L-E1A
<400> 20
   aaatctatct atataatata ccttatagat ggaatggtgc caacatgtaa atgaggtaat      60
   ttaaaaaagt gcgcgctgtg tggtgattgg ctgcggggtt aacggctaaa aggggcggcg     120
   cgaccgtggg aaaatgacgt gacttatgtg ggaggagtta tgttgcaagt tattacggta     180
   aatgtgacgt aaaacgaggt gtggtttgaa cacggaagta gacagttttc ccacgcttac     240
   tgacaggata tgaggtagtt ttgggcggat gcaagtgaaa attctccatt ttcgcgcgaa     300
   aactaaatga ggaagtgaat ttctgagtca tttcgcggtt atgccagggt ggagtatttg     360
   ccgagggccg agtagacttt gaccgtttac gtggaggttt cgattaccgt gtttttcacc     420
   taaatttccg cgtacggtgt caaagttctg tgtttttacg taggtgtcag ctgatcgctg     480
   atcggacgtc ctgcgctgtc ggggccaggc cgggctccca gtggattcgc gggcacagac     540
   gcccaggacc gcgcttccca cgtggcggag ggactgggga cccgggcacc cgtcctgccc     600
   cttcaccttc cagctccgcc tcctccgcgc ggaccccgcc ccgtcccgac ccctcccggg     660
   tccccggccc agccccctcc gggccctccc agcccctccc cttcctttcc gcggccccgc     720
   cctctcctcg cggcgcgagt ttcaggcagc gctgcgtcct gctgcgcacg tgggaagccc     780
   tggcccaatt gatgatcgaa acatacaacc aaacttctcc ccgatctgcg gccactggac     840
   tgcccatcag catgaaaatt tttatgtatt tacttactgt ttttcttatc acccagatga     900
   ttgggtcagc actttttgct gtgtatcttc atagaaggct ggacaagata gaagatgaaa     960
   ggaatcttca tgaagatttt gtattcatga aaacgataca gagatgcaac acaggagaaa    1020
   gatccttatc cttactgaac tgtgaggaga ttaaaagcca gtttgaaggc tttgtgaagg    1080
   atataatgtt aaacaaagag gagacgaaga aagaaaacag ctttgaaatg caaaaaggtg    1140
   atcagaatcc tcaaattgcg gcacatgtca taagtgaggc cagcagtaaa acaacatctg    1200
   tgttacagtg ggctgaaaaa ggatactaca ccatgagcaa caacttggta accctggaaa    1260
   atgggaaaca gctgaccgtt aaaagacaag gactctatta tatctatgcc caagtcacct    1320
   tctgttccaa tcgggaagct tcgagtcaag ctccatttat agccagcctc tgcctaaagt    1380
   cccccggtag attcgagaga atcttactca gagctgcaaa tacccacagt tccgccaaac    1440
   cttgcgggca acaatccatt cacttgggag gagtatttga attgcaacca ggtgcttcgg    1500
   tgtttgtcaa tgtgactgat ccaagccaag tgagccatgg cactggcttc acgtcctttg    1560
   gaattcgccc ctctccctcc ccccccccta acgttactgg ccgaagccgc ttggaataag 1620
   gccggtgtgc gtttgtctat atgttatttt ccaccatatt gccgtctttt ggcaatgtga 1680
   gggcccggaa acctggccct gtcttcttga cgagcattcc taggggtctt tcccctctcg 1740
   ccaaaggaat gcaaggtctg ttgaatgtcg tgaaggaagc agttcctctg gaagcttctt 1800
   gaagacaaac aacgtctgta gcgacccttt gcaggcagcg gaacccccca cctggcgaca 1860
   ggtgcctctg cggccaaaag ccacgtgtat aagatacacc tgcaaaggcg gcacaacccc 1920
   agtgccacgt tgtgagttgg atagttgtgg aaagagtcaa atggctctcc tcaagcgtat 1980
   tcaacaaggg gctgaaggat gcccagaagg taccccattg tatgggatct gatctggggc 2040
   ctcggtgcac atgctttaca tgtgtttagt cgaggttaaa aaaacgtcta ggccccccga 2100
   accacgggga cgtggttttc ctttgaaaaa cacgatgata atatggccac aattaatgat 2160
   gggctcgaat cgtgccagcg agaagagttt tctcctccgc gccgcaagtc agttctgcgc 2220
   tttgaaaatg agacacctgc gcttcctgcc acaggaggtt atctccagtg agaccgggat 2280
   cgaaatactg gagtttgtgg taaataccct aatgggagac gacccggaac cgccagtgca 2340
   gcctttcgat ccacctacgc tgcacgatct gtatgattta gagatagacg ggccggagga 2400
   tcccaatgag gaagctgtga atgggttttt tactgattct atgctgctag ctgctgatga 2460
   aggattggac ataaaccctc ctcctgagac acttgttacc ccaggggtgg ttgtggaaag 2520
   cggcataggt gggaaaaaat tgcctgatct gggagcagct gaaatggact tgcgttgtta 2580
   tgaagagggt tttcctccca gtgatgatga agatggggaa actgagcagt ccatccatac 2640
   cgcagtaaat gagggagtaa aagctgccag cgatgttttt aagttggact gtccggagct 2700
   gcctggacat ggctgtaagt cttgtgaatt tcacaggaat aacactggaa tgaaagaact 2760
   attgtgctcg ctttgctata tgagaatgca ctgccacttt atttacagta agtgtattta 2820
   agtgaaattt aaaggaatag tgtagctatt taataactgt tgaatggtag atttatgttt 2880
   ttttcttgcg attttttgta ggtcctgtgt ctgatgatga gtcaccttct cctgattcaa 2940
   ctacctcacc tcctgaaatt caggcgcccg cacctgcaaa cgtatgcaag cccattcctg 3000
   tgaagcctaa gcctgggaaa cgccctgctg tggataagct tgaggacttg ttggagggtg 3060
   gggatggacc tttggacctt agtacccgga aactgccaag gcaatgagtg ccctgcagct 3120
   gtgtttattt aatgtgacgt catgtaataa aattatgtca gctgctgagt gttttattac 3180
   ttcttgggtg gggacttgga tatataagta ggagcagatc tgtgtggtta gctcacagca 3240
   acctgctgcc atccatggag gtttgggcta tcttggaaga cctcagacag actaagctac 3300
   tgctagaaaa cgcctcggac ggagtctctg gcctttggag attctggttc ggtggtgatc 3360
   tagctaggct agtgtttagg ataaaacagg actacaggga agaatttgaa aagttattgg 3420
   acgatagtcc gggacttttt gaagctctta acttgggtca tcaggctcat tttaaggaga 3480
   aggttttatc agttttagat ttttctactc ctggtagaac tgctgctgct gtagcttttc 3540
   ttacttttat attggataaa tggatccgcc aaactcactt cagcaaggga tacgttttgg 3600
   atttcatagc agcagctttg tggagaacat ggaaggctcg caggatgagg acaatcttag 3660
   attactggcc agtgcagcct ctgggagtag cagggatact gagacaccca ccgaccatgc 3720
   cagcggttct gcaggaggag cagcaggagg acaatccgag agccggcctg gaccctccgg 3780
   tggaggagta gctgacctgt ttcctgaact gcgacgggtg cttactaggt ctacgaccag 3840
   tggacagaac aggggaatta agagggagag gaatcctagt gggaataatt caagaaccga 3900
   gttggcttta agtttaatga gccgcaggcg tcctgaaact gtttggtggc atgaggttca 3960
   gagcgaaggc agggatgaag tttcaatatt gcaggagaaa tattcactag aacaacttaa 4020
   gacctgttgg ttggaacctg aggatgattg ggaggtggcc attaggaatt atgctaagat 4080
   atctctgagg cctgataaac aatatagaat tactaagaag attaatatta gaaatgcatg 4140
   ctacatatca gggaatgggg cagaggttat aatagataca caagataaag cagtttttag 4200
   atgttgtatg atgggtatgt ggccaggggt tgtcggcatg gaagcagtaa cacttatgaa 4260
   tattaggttt aaaggggatg ggtataatgg cattgtattt atggctaaca ctaagctgat 4320
   tctacatggt tgtagctttt ttgggtttaa taatacgtgt gtagaagctt gggggcaagt 4380
   tagtgtgagg ggttgtagtt tttatgcatg ctggattgca acatcaggta gggtcaagag 4440
   tcagttgtct gtgaagaaat gcatgtttga gagatgtaat cttggcatac tgaatgaagg 4500
   tgaagcaagg gtccgccact gcgcagctac agaaactggc tgcttcattc taataaaggg 4560
   aaatgccagt gtgaagcata atatgatctg tggacattcg gatgagaggc cttatcagat 4620
   gctgacctgc gctggtggac attgcaatat tcttgctacc gtgcatatcg tttcacatgc 4680
   acgcaaaaaa tggcctgtat ttgaacataa tgtgattacc aagtgcacca tgcacatagg 4740
   tggtcgcagg ggaatgttta tgccttacca gtgtaacatg aatcatgtga aggtaatgtt 4800
   ggaaccagat gccttttcca gagtgagctt aacaggaatc tttgatatga atattcaact 4860
   atggaagatc ctgagatatg atgacactaa accaagggtg cgcgcatgcg aatgcggagg 4920
   caagcatgct agattccagc cggtgtgcgt ggatgtgact gaagacttga gacccgatca 4980
   tttggtgctt gcctgcactg gagcggagtt cggttctagt ggtgaagaaa ctgactaaag 5040
   tgagtagtgg ggcaagatgt ggatggggac tttcaggttg gtaaggtggg cagattgggt 5100
   aaattttgtt aatttctgtc ttgcagctgc catgagtgga agcgcttctt ttgagggggg 5160
   agtatttagc ccttatctga cgggcaggct cccaccatgg gcaggagttc gtcagaatgt 5220
   catgggatcc actgtggatg ggagacccgt ccagcccgcc aattcctcaa cgctgaccta 5280
   tgccactttg agttcgtcac cattggatgc agctgcagcc gccgccgcta ctgctgccgc 5340
   caacaccatc cttggaatgg gctattatgg aagcatcgtt gccaattcca gttcctctaa 5400
   taacccttca accctggctg aggacaagct acttgttctc ttggcgcagc tcgaggcctt 5460
   aacccaacgc ttaggcgaac tgtctaagca ggtggcccag ttgcgtgagc aaactgagtc 5520
   tgctgttgcc acagcaaagt ctaaataaag atctcaaatc aataaataaa gaaatacttg 5580
   atataaaaca aatgaatgtt tatttgattt ttcgcgcgcg gtatgccctg gaccatcggt 5640
   ctcgatcatt gagaacgcgg tggatctttt ccagtaccct gtaaaggtgg gattgaatgt 5700
   ttagatacat gggcattagt ccgtctcggg ggtggagata gctccattga agagcctctt 5760
   gctccggggt agtgttataa atcacccagt catagcaagg tcggagtgca tggtgttgca 5820
   caatatcttt taggagcaga ctaattgcaa cggggaggcc cttagtgtag gtgtttacaa 5880
   atctgttgag ctgggacggg tgcatccggg gtgaaattat atgcattttg gactggatct 5940
   tgaggttggc aatgttgccg cctagatccc gtctcgggtt catattgtgc aggaccacca 6000
   agacagtgta tccggtgcac ttgggaaatt tatcatgcag cttagaggga aaagcatgaa 6060
   aaaatttgga gacgcctttg tgacccccca gattctccat gcactcatcc ataatgatag 6120
   cgatggggcc gtgggcagcg gcacgggcga acacgttccg ggggtctgaa acatcatagt 6180
   tatgctcctg agtcaggtca tcataagcca ttttaataaa ctttgggcgg agggtgccag 6240
   attgggggat gaaagttccc tctggcccgg gagcatagtt tccctcacat atttgcattt 6300
   cccaggcttt cagttcagag ggggggatca tgtccacctg cggggctata aaaaataccg 6360
   tttctggagc cggggtgatt aactgggacg agagcaaatt cctaagcagc tgagacttgc 6420
   cgcacccagt gggaccgtaa atgaccccaa ttacgggttg cagatggtag tttagggaac 6480
   gacagctgcc gtcctcccgg agcagggggg ccacttcgtt catcatttcc cttacatgga 6540
   tattttcccg caccaagtcc gttaggaggc gctctccccc aagggataga agctcctgga 6600
   gcgaggagaa gtttttcagc ggcttcagcc cgtcagccat gggcattttg gaaagagtct 6660
   gttgcaagag ctcgagccgg tcccagagct cggtgatgtg ctctatggca tctcgatcca 6720
   gcagacctcc tcgtttcgcg ggttgggacg gctcctggag tagggaatca gacgatgggc 6780
   gtccagcgct gccagggtcc gatccttcca tggtcgcagc gtccgagtca gggttgtttc 6840
   cgtcacggtg aaggggtgcg cgcctggttg ggcgcttgcg agggtgcgct tcagactcat 6900
   cctgctggtc gagaaccgct gccgattggc gccctgcatg tcggccaggt agcagtttac 6960
   catgagttcg tagttgagcg cctcggccgc gtggcctttg gcacggagct tacctttgga 7020
   agttttatgg caggcggggc agtagataca tttgagggca tacaacttgg gcgcgaggaa 7080
   aatggattcg ggggagtatg catccgcacc gcaggaggcg cagacggttt cgcactccac 7140
   gagccaggtc agatccggct catcggggtc aaaaacaagt tttccgccat gttttttgat 7200
   gcgtttctta cctttggttt ccatgagttc gtgtccccgc tgggtgacaa agaggctgtc 7260
   cgtgtccccg tagaccgact ttatgggcct gtcctcgagc ggagtgcctc ggtcctcttc 7320
   gtagaggaac ccagcccact ctgatacaaa agcgcgtgtc caggccagca caaaggaggc 7380
   cacgtgggag gggtagcggt cgttgtcaac cagggggtcc accttctcta cggtatgtaa 7440
   acacatgtcc ccctcctcca catccaagaa tgtgattggc ttgtaagtgt aggccacgtg 7500
   accaggggtc cccgccgggg gggtataaaa gggggcgggc ctctgttcgt cctcactgtc 7560
   ttccggatcg ctgtccagga gcgccagctg ttggggtagg tattccctct cgaaggcggg 7620
   catgacctct gcactcaggt tgtcagtttc taggaacgag gaggatttga tattgacagt 7680
   accagcagag atgcctttca taagactctc gtccatctgg tcagaaaaca caatcttctt 7740
   gttgtccagc ttggtggcaa atgatccata gagggcattg gatagaagct tggcgatgga 7800
   gcgcatggtt tggttctttt ccttgtccgc gcgctccttg gcggtgatgt taagctggac 7860
   gtactcgcgc gccacacatt tccattcagg aaagatggtt gtcagttcat ccggaactat 7920
   tctgattcgc catcccctat tgtgcagggt tatcagatcc acactggtgg ccacctcgcc 7980
   tcggaggggc tcattggtcc agcagagtcg acctcctttt cttgaacaga aaggggggag 8040
   ggggtctagc atgaactcat caggggggtc cgcatctatg gtaaatattc ccggtagcaa 8100
   atctttgtca aaatagctga tggtggcggg atcatccaag gtcatctgcc attctcgaac 8160
   tgccagcgcg cgctcatagg ggttaagagg ggtgccccag ggcatggggt gggtgagcgc 8220
   ggaggcatac atgccacaga tatcgtagac atagaggggc tcttcgagga tgccgatgta 8280
   agtgggataa catcgccccc ctctgatgct tgctcgcaca tagtcataga gttcatgtga 8340
   gggggcaaga agacccgggc ccagattggt gcggttgggt ttttccgccc tgtaaacgat 8400
   ctggcgaaag atggcatggg aattggaaga gatagtaggt ctctggaata tgttaaaatg 8460
   ggcatgaggt aagcctacag agtcccttat gaagtgggca tatgactctt gcagcttggc 8520
   taccagctcg gcggtgatga gtacatccag ggcacagtag tcgagagttt cctggatgat 8580
   gtcataacgc ggttggcttt tcttttccca cagctcgcgg ttgagaaggt attcttcgtg 8640
   atccttccag tactcttcga ggggaaaccc gtctttttct gcacggtaag agcccaacat 8700
   gtagaactga ttgactgcct tgtagggaca gcatcccttc tccactggga gagagtatgc 8760
   ttgggctgca ttgcgcagcg aggtatgagt gagggcaaaa gtgtccctga ccatgacttt 8820
   gaggaattga tacttgaagt cgatgtcatc acaggccccc tgttcccaga gttggaagtc 8880
   cacccgcttc ttgtaggcgg ggttgggcaa agcgaaagta acatcattga agaggatctt 8940
   gccggccctg ggcatgaaat ttcgggtgat tttgaaaggc tgaggaacct ctgctcggtt 9000
   attgataacc tgagcggcca agacgatctc atcaaagcca ttgatgttgt gccccactat 9060
   gtacagttct aagaatcgag gggtgcccct gacatgaggc agcttcttga gttcttcaaa 9120
   agtgagatct gtagggtcag tgagagcata gtgttcgagg gcccattcgt gcacgtgagg 9180
   gttcgcttta aggaaggagg accagaggtc cactgccagt gctgtttgta actggtcccg 9240
   gtactgacga aaatgctgtc cgactgccat cttttctggg gtgacgcaat agaaggtttg 9300
   ggggtcctgc cgccagcgat cccacttgag ttttatggcg aggtcatagg cgatgttgac 9360
   gagccgctgg tctccagaga gtttcatgac cagcatgaag gggattagct gcttgccaaa 9420
   ggaccccatc caggtgtagg tttccacatc gtaggtgaga aagagccttt ctgtgcgagg 9480
   atgagagcca atcgggaaga actggatctc ctgccaccag ttggaggaat ggctgttgat 9540
   gtgatggaag tagaactccc tgcgacgcgc cgagcattca tgcttgtgct tgtacagacg 9600
   gccgcagtac tcgcagcgat tcacgggatg caccttatga atgagttgta cctgacttcc 9660
   tttgacgaga aatttcagtg gaaaattgag gcctggcgct tgtacctcgc gctttactat 9720
   gttgtctgca tcggcatgac catcttctgt ctcgatggtg gtcatgctga cgagccctcg 9780
   cgggaggcaa gtccagacct cggcgcggca ggggcggagc tcgaggacga gagcgcgcag 9840
   gccggagctg tccagggtcc tgagacgctg cggagtcagg ttagtaggca gtgtcaggag 9900
   attaacttgc atgatctttt ggagggcgtg agggaggttc agatagtact tgatctcaac 9960
   gggtccgttg gtggagatgt cgatggcttg cagggttccg tgtcccttgg gcgctaccac 10020
   cgtgcccttg tttttcattt tggacggcgg tggctctgtt gcttcttgca tgtttagaag 10080
   cggtgtcgag ggcgcgcacc gggcggcagg ggcggctcgg gacccggcgg catggctggc 10140
   agtggtacgt cggcgccgcg cgcgggtagg ttctggtact gcgccctgag aagactcgca 10200
   tgcgcgacga cgcggcggtt gacatcctgg atctgacgcc tctgggtgaa agctaccggc 10260
   cccgtgagct tgaacctgaa agagagttca acagaatcaa tctcggtatc gttgacggcg 10320
   gcttgcctaa ggatttcttg cacgtcgcca gagttgtcct ggtaggcgat ctcggccatg 10380
   aactgctcga tctcttcctc ttgaagatct ccgcggcccg ctctctcgac ggtggccgcg 10440
   aggtcgttgg agatgcgccc aatgagttga gagaatgcat tcatgcccgc ctcgttccag 10500
   acgcggctgt agaccacagc ccccacggga tctctcgcgc gcatgaccac ctgggcgagg 10560
   ttgagctcca cgtggcgggt gaagaccgca tagttgcata ggcgctggaa aaggtagttg 10620
   agtgtggtgg cgatgtgctc ggtgacgaag aaatacatga tccatcgtct cagcggcatc 10680
   tcgctgacat cgcccagcgc ttccaagcgc tccatggcct cgtagaagtc cacggcaaag 10740
   ttaaaaaact gggagttacg cgcggacacg gtcaactcct cttccagaag acggataagt 10800
   tcggcgatgg tggtgcgcac ctcgcgctcg aaagcccctg ggatttcttc ctcaatctct 10860
   tcttcttcca ctaacatctc ttcctcttca ggtggggctg caggaggagg gggaacgcgg 10920
   cgacgccggc ggcgcacggg cagacggtcg atgaatcttt caatgacctc tccgcggcgg 10980
   cggcgcatgg tctcggtgac ggcacgaccg ttctccctgg gtctcagagt gaagacgcct 11040
   ccgcgcatct ccctgaagtg gtgactggga ggctctccgt tgggcaggga caccgcgctg 11100
   attatgcatt ttatcaattg ccccgtaggt actccgcgca aggacctgat cgtctcaaga 11160
   tccacgggat ctgaaaacct ttcgacgaaa gcgtctaacc agtcgcaatc gcaaggtagg 11220
   ctgagcactg tttcttgcgg gcgggggcgg ctagacgctc ggtcggggtt ctctctttct 11280
   tctccttcct cctcttggga gggtgagacg atgctgctgg tgatgaaatt aaaataggca 11340
   gttttgagac ggcggatggt ggcgaggagc accaggtctt tgggtccggc ttgttggata 11400
   cgcaggcgat gagccattcc ccaagcatta tcctgacatc tggccagatc tttatagtag 11460
   tcttgcatga gtcgttccac gggcacttct tcttcgcccg ctctgccatg catgcgagtg 11520
   atcccgaacc cgcgcatggg ctggacaagt gccaggtccg ctacaaccct ttcggcgagg 11580
   atggcttgct gcacctgggt gagggtggct tggaagtcgt caaagtccac gaagcggtgg 11640
   taggccccgg tgttgattgt gtaggagcag ttggccatga ctgaccagtt gactgtctgg 11700
   tgcccagggc gcacgagctc ggtgtacttg aggcgcgagt atgcgcgggt gtcaaagatg 11760
   taatcgttgc aggtgcgcac caggtactgg tagccaatga gaaagtgtgg cggtggctgg 11820
   cggtacaggg gccatcgctc tgtagccggg gctccggggg cgaggtcttc cagcatgagg 11880
   cggtggtagc cgtagatgta cctggacatc caggtgatac cggaggcggt ggtggatgca 11940
   cgtgggaact cgcgcacgcg gttccagatg ttgcgcagcg gcatgaagta gttcatggta 12000
   ggcacggtct ggccagtgag gcgcgcgcag tcattgacgc tctgtagaca cggagaaaac 12060
   gaaagcgatg agcggctcga ctccgtggtc tgggggaacg tgaacgggtt gggtcgcggt 12120
   gtaccccggt tcgagtccaa agctaagcga tcacgctcgg atcggccgga gccgcggcta 12180
   acgtggtatt ggctatcccg tctcgaccca gccgacgaat atccagggta cggagtagag 12240
   tcgtttttgc tgctttttcc tggacgtgtg ccattgccac gtcaagcttt agaacgctca 12300
   gttctcgggc cgtgagtggc tcgcgcccgt agtctggaga atcagtcgcc agggttgcgt 12360
   tgcggtatgc cccggttgga gcctaagcgc ggctcgtatc ggccggtttc cgcgacaagc 12420
   gagggtttgg cagccccgtt atttccaaga ccccgccagc cgacttctcc agtttacggg 12480
   agcgagccct tttttttttt tttttgtttt tgtcgcccag atgcatccag tgctgcgaca 12540
   gatgcgcccc cagcaacagg ccccttctca gcaacagcca caaaaggctc ttcttgctcc 12600
   tgcaactact gcagctgcag ccgtgagcgg cgcgggacag cccgcctatg atctggactt 12660
   ggaagagggc gagggattgg cgcgcctggg ggctccatcg cccgagcggc acccgcgggt 12720
   gcaactaaaa aaggactctc gcgaggcgta cgtgccccag cagaacctgt tcagggacag 12780
   gagcggcgag gagccagagg agatgcgagc atctcgattt aacgcgggtc gcgagctgcg 12840
   ccacggtctg gatcgaagac gggtgctgca agacgaggat tttgaggtcg atgaagtcac 12900
   agggatcagc ccagctaggg cacatgtggc cgcggccaac ctagtctcgg cctacgagca 12960
   gaccgtgaag gaggagcgca acttccaaaa atcttttaac aaccatgtgc gcaccctgat 13020
   cgcccgcgag gaagtgaccc tgggtctgat gcatctgtgg gacctgatgg aggctatcgc 13080
   ccaaaacccc actagcaaac cactgacagc tcagctgttt ctggtggttc aacatagcag 13140
   ggacaacgag gcattcaggg aggcgttgtt gaacatcacc gagcctgatg ggagatggct 13200
   gtatgatctg atcaacatcc tgcaaagtat tatagtgcag gaacgtagcc tgggtttggc 13260
   tgagaaagtg gcagctatca actactcggt cttgagcctg ggcaaatact acgctcgcaa 13320
   gatctacaag accccctacg tacccataga caaggaggtg aagatagatg ggttttacat 13380
   gcgcatgact ctgaaggtgc tgactctgag cgacgatctg ggggtgtatc gcaatgacag 13440
   gatgcaccgc gcggtgagcg ccagcaggag gcgcgagctg agcgacagag aacttatgca 13500
   cagcttgcaa agagctctaa cgggggccgg gactgatggg gagaactact ttgacatggg 13560
   agcggatttg caatggcaac ccagtcgcag ggccatggag gctgcagggt gtgagcttcc 13620
   ttacatagaa gaggtggatg aagtcgagga cgaggagggc gagtacttgg aagactgatg 13680
   gcgcgacccg tatttttgct agatggaaca gcagcaggca ccggaccccg caatgcgggc 13740
   ggcgctgcag agccagccgt ccggcattaa ctcctcggac gattggaccc aggccatgca 13800
   acgcataatg gcgctgacga cccgcaaccc cgaagccttt agacagcaac cccaggccaa 13860
   ccgcctttct gccatactgg aggccgtagt gccctcccgc tccaacccca cccacgagaa 13920
   ggtcctggct atcgtgaacg cgctggtgga gaacaaggcc atccgtcccg atgaggccgg 13980
   gctggtatac aatgctctct tggagcgcgt ggcccgttac aacagcagca acgtgcaaac 14040
   caacctggac cggatggtga ccgatgtgcg cgaggccgtg tctcagcgcg agcgattcca 14100
   gcgcgacgcc aacttggggt cgttggtagc gctaaacgct ttcctcagca cccagcccgc 14160
   caacgtgccc cgtggtcagc aagactatac aaactttttg agtgcattga gactcatggt 14220
   agctgaggtg ccccagagcg aggtgtacca gtccgggcca gattacttct tccagaccag 14280
   cagacagggc ttgcagacag tgaacctgac tcaggctttc aagaacctga agggtctgtg 14340
   gggagtgcac gccccagtag gggatcgcgc gaccgtgtct agcttgctga ctcccaactc 14400
   ccgcctgctg ctgctgctgg tatccccctt tactgacagc ggtagcattg accgcaactc 14460
   gtacttgggc tacctgctta acctgtatcg cgaggccata ggacagagcc aggtggacga 14520
   gcagacctat caagaaatca cccaagtgag ccgcgccctg ggtcaggaag acacgggcag 14580
   tttggaagcc accctgaact tcttgctaac caaccggtca cagaagatcc ctcctcagta 14640
   tgcgcttacc gctgaggagg agcggatcct cagatacgtg caacagagcg ttggactgtt 14700
   cctgatgcag gagggggcga cacctaccgc cgcgctggac atgacagctc gaaacatgga 14760
   gcccagcatg tatgctagta acaggccttt cattaacaaa ctgctggact acctgcacag 14820
   ggcggccgcc atgaactctg attatttcac caatgctatc ctgaacccac actggctgcc 14880
   cccacctggt ttctacactg gcgagtacga catgcccgac cccaatgacg ggttcctgtg 14940
   ggacgatgtg gacagcagca tattttcccc gcctcccggt tatacagttt ggaagaagga 15000
   agggggcgat agaagacact cttccgtgtc gctatccgga acggctggtg ctgccgcgac 15060
   cgtgcccgaa gctgcaagtc ctttccctag cttgcccttt tcactaaaca gcgttcgcag 15120
   cagtgaactg gggagaataa cccgcccgcg cttgatgggc gaggatgagt acttgaatga 15180
   ctctttgctg aggccagaga gggaaaagaa cttccccaac aatggaatag agagtctggt 15240
   ggataagatg agtagatgga agacctatgc gcaggatcac agagacgagc ccaggatctt 15300
   gggggctaca agcagaccga tccgtagacg ccagcgccac gacaggcaga tgggtcttgt 15360
   gtgggacgat gaggactctg ccgatgatag cagcgtgttg gacttgggtg gaagaggagg 15420
   gggcaacccg ttcgctcatc tgcgtcccag attcgggcgc atgttgtaaa agtgaaagta 15480
   aaataaaaag gcaactcacc aaggccatgg cgaccgagcg tgcgttcgtt cttttttgtt 15540
   atctgtgtct agtacgatga ggagacgagc cgtgctaggc ggagcggtgg tgtatccgga 15600
   gggtcctcct ccttcttacg agagcgtgat gcagcaacag gcggcgatga tacagccccc 15660
   actggaggct cccttcgtac ccccacggta cctggcgcct acggaaggga gaaacagcat 15720
   tcgttactcg gagctgtcgc ccctgtacga taccaccaag ttgtatctgg tggacaacaa 15780
   gtcggcggac atcgcctccc tgaactatca gaacgaccac agcaacttcc tgaccacggt 15840
   ggtgcagaac aatgacttta cccccacgga ggctagcacc cagaccatca actttgacga 15900
   gcggtcgcga tggggcggtc agctgaagac catcatgcac accaacatgc ccaacgtgaa 15960
   cgagtacatg ttcagcaaca agttcaaggc gagggtgatg gtgtccagaa aagctcctga 16020
   aggtgttaca gtaaatgaca cctatgatca taaagaggat atcttgaagt atgagtggtt 16080
   tgagttcatt ttaccagaag gcaacttttc agccaccatg acgatcgacc tgatgaacaa 16140
   tgccatcatt gacaactacc tggaaattgg cagacagaat ggagtgctgg aaagtgacat    16200
   tggtgttaag tttgacacta gaaatttcag gctcgggtgg gaccccgaaa ctaagttgat    16260
   tatgccaggt gtctacactt atgaggcatt ccatcctgac attgtattgc tgcctggttg 16320
   cggggtagac tttactgaaa gccgacttag caacttgctt ggcatcagga agagacatcc 16380
   attccaggag ggtttcaaaa tcatgtatga agatcttgaa gggggtaata ttcctgccct 16440
   tttggatgtc actgcctatg aggaaagcaa aaaggatacc actactgaaa caaccacact 16500
   ggctgttgca gaggaaacta gtgaagatga tgatataact agaggagata cctatataac 16560
   tgaaaaacaa aaacgtgaag ctgcagctgc tgaagttaaa aaagagttaa agatccaacc 16620
   tctagaaaaa gacagcaaga gtagaagcta caatgtcttg gaagacaaaa tcaacacagc 16680
   ctaccgcagt tggtacctgt cctacaatta cggtaaccct gagaaaggaa taaggtcttg 16740
   gacactgctc accacttcag atgtcacctg tggggcagag caggtctact ggtcgctccc 16800
   tgacatgatg caagacccag tcaccttccg ctccacaaga caagtcaaca actacccagt 16860
   ggtgggtgca gagcttatgc ccgtcttctc aaagagtttc tacaatgagc aagccgtgta 16920
   ctctcagcag ctccgacagg ccacttcgct cacgcacgtc ttcaaccgct tccctgagaa 16980
   ccagatcctc atccgcccgc cggcgcccac aattaccacc gtcagtgaaa acgttcctgc 17040
   tctcacagat cacgggaccc tgccgttacg cagcagtatc cggggagtcc agcgcgtgac 17100
   cgttactgac gccagacgcc gcacctgtcc ctacgtttac aaggccctgg gcatagtcgc 17160
   gccgcgcgtt ctttcaagcc gcactttcta aaaaaaaaaa aaatgtccat tctcatctcg 17220
   cccagtaata ataccggttg gggactgtat gcgcccacca agatgtatgg aggcgcccgc 17280
   aagcgctcta cccagcatcc tgtgcgcgtt cgcggtcatt tccgcgctcc ctggggcgca 17340
   ctcaagggtc gtacccgcac tcggaccacg gtcgatgatg tgatcgacca ggtggtcgcc 17400
   gatgctcgta attatactcc tactgcgcct acatctactg tggatgcagt tattgacagt 17460
   gtggtggcag acgcccgcgc ctatgctcgc cggaagagcc gaaggaggcg catcgccagg 17520
   cgccacaggg ctactcccgc catgcgagcc gcaaaagcta ttctgcggag ggccaaacgt 17580
   gtggggcgaa gagccatgct tagagcggcc agacgcgcgg cttcaggtgc cagcagcggc 17640
   aggtcccgca ggcgcgcggc cacggcggca gcagcggcca ttgccaacat ggcccaaccg 17700
   cgaagaggca atgtgtactg ggtgcgtgat gccactaccg gccagcgcgt gcccgtgcgc 17760
   acccgccccc ctcgcactta gaagatactg agcagtctcc gatgttgtgt cccagcggca 17820
   agtatgtcca agcgcaaata caaggaagag atgctccagg tcatcgcgcc tgaaatctac 17880
   ggtccgccgg tgaaggatga aaaaaagccc cgcaaaatca agcgggtcaa aaataacaaa 17940
   aaggaagaag atgacgatga tgggctggtg gagtttgtgc gcgagttcgc cccaagacgg 18000
   cgcgtgcagt ggcgcgggcg caaagtgcgt caagtgctca gacccgggac cactgtggtt 18060
   tttacacctg gcgagcgttc cagcactact tttaaacggt cctatgatga ggtgtacggg 18120
   gatgacgata ttcttgagca ggcggcagac cgccttgacg agtttgctta tggcaagcgc 18180
   actagatcca gtcccaaaga ggaggctgtg tccattcctt tggatcatgg aaatcccacc 18240
   cccagcctca aaccagtcac cctgcagcaa gtgctgcccg tgcctgcgcg gagaggcgta 18300
   aagcgcgagg gtgaggacct atatcccacc atgcagctaa tggtgcccaa gcgccagagg 18360
   ctagaagacg tactggagaa aatgaaagtg gatgccgata tccagcctga ggtcaaagta 18420
   agacctatca aggaagtggc gccaggtttg ggagtacaaa ccttcgacat caagattccc 18480
   accgagtcca tggaagtgca gaccgaacct gcaaaaccca ccacctcaat tgaggtgcaa 18540
   acggaaccct ggatgcccgc gcccgttgcc gcccccagca ccactcgaag atcacgacga 18600
   aagtacggcc cagcaagtct gctaatgccc aactatgctc tgcacccatc catcattccc 18660
   actccgggtt acagaggcac tcgctactat cgaaaccgga gcaacacctc tcgccgccgc 18720
   aaaccacctg caagtcgcac tcgccgtcgc cgccgccgca acactgccag caaattgact 18780
   cccgccgccc tggtgcggag agtgtaccgc gatggtcgcg ctgaacctct gacgctgccg 18840
   cgcgcgcgct accatccaag catcaccact taatgactgt tgacgctgcc tccttgcaga 18900
   tatggccctc acttgccgcc ttcgcgtccc cattactggc taccgaggaa gaaactcgcg 18960
   ccgtagaagg atgttggggc gagggatgcg ccgccacaga cgaaggcgcg ctatcagcag 19020
   acgattaggg ggtggctttt tgccagctct tatacccatc atcgccgcag cgatcggggc 19080
   gataccaggc atagcttcag tggcggttca ggcctcgcag cgccactaac attggaaaaa 19140
   acttataaat aaaaaataga atggactctg acgctcctgg tcctgtgact atgtttttgt 19200
   agagatggaa gacatcaatt tttcatccct ggctccgcga cacggcacga ggccgtacat 19260
   gggcacctgg agcgacatcg gcacgagcca actgaacggg ggcgccttca attggagcag 19320
   tatctggagc gggcttaaaa attttggctc gaccgtaaaa acctatggga acaaagcttg 19380
   gaacagcagc acagggcagg ctctgagaaa taagcttaag gaacaaaact tccaacagaa 19440
   ggtggtcgat gggatcgcct ctggtattaa cggcgtagtg gatctggcca accaggctgt 19500
   acaaaaacag ataaacagcc gcctggaccc gccgcccgca acccctggtg aaatggaagt 19560
   ggaggaagaa cttcctccgc tggaaaagcg gggcgacaag cgtccgcgac ccgagctaga 19620
   gcagacgctg gtgacgcgcg cagacgagcc cccttcatat gaggaggcag taaagctcgg 19680
   aatgcccact accaggcctg tagctcacat ggctaccggg gtgatgaaac cttctcagtc 19740
   acatcgaccc gccaccttgg acttgcctcc tccccctgct tctgcggcgc ctgttcccaa 19800
   acctgtcgct accagaaagc ccaccgccgt acagcccgtc gccgtagcca gaccgcgtcc 19860
   tgggggcaca ccgcgcccga aagcaaactg gcaaagtact ctgaacagca tcgtgggtct 19920
   gggcgtgcag agtgtaaagc gccgtcgctg ctattaatta aatatggagt agcgcttaac 19980
   ttgcttgtct gtgtgtatgt atcatcacca cgccgccgca gcagaggaga aaggaagagg 20040
   tcgcgcgccg aggctgagtt gctttcaaga tggccacccc atcgatgatg ccccaatggg 20100
   catacatgca catcgccgga caggatgctt cggagtacct cagtccgggt ctggtgcagt 20160
   tcgcccgtgc aacagacacc tacttcagta tggggaacaa atttagaaac cccacagtgg 20220
   cgcccaccca cgatgtgacc accgaccgta gccagcgcct gatgctgcgc ttcgtgcccg 20280
   ttgaccggga agacaatacc tactcttaca aagttcgcta cacgctggct gtaggcgaca 20340
   acagagtgct tgacatggcc agcacattct ttgacattcg gggggtgctt gatagaggtc 20400
   ctagcttcaa gccatattcc ggcacagctt acaattcact cgctcctaag ggcgcgccca 20460
   atacatctca gtggatagtt acaacaaatg gggacaatgc agtaactacc accacaaaca 20520
   catttggcat tgcttccatg aagggagaca atattactaa agaaggtttg caaattggga 20580
   aagacattac cactactgaa ggagaagaaa agcccattta tgccgataaa acatatcagc 20640
   cagagcctca agttggagaa gaatcatgga ctgatactga tggaacaaat gaaaagtttg 20700
   gtggaagagc ccttaaacca gctaccaaca tgaagccatg ctacgggtct tttgcaagac 20760
   ctacaaacat aaaagggggc caagctaaaa acagaaaagt aaaaccaaca accgaaggag 20820
   gggttgaaac tgaggaacca gatattgata tggaattttt cgatggtaga gatgctgttg 20880
   caggagcttt agcgcctgaa attgtgcttt atacggaaaa tgtaaatttg gaaactccag 20940
   acagtcatgt ggtatataaa ccagaaacgt ctaataactc tcatgcaaat ttgggtcaac 21000
   aagccatgcc taacagaccc aattacattg gcttcaggga taacttcgta ggcctaatgt 21060
   actacaacag tactggaaat atgggagttt tggctggcca agcatcacaa ctgaatgcag 21120
   tggttgactt gcaggacaga aatactgaac tgtcatatca gcttttgctt gattctctgg 21180
   gagacagaac cagatacttc agcatgtgga atcaggctgt ggacagttac gatcccgatg 21240
   ttcgcattat tgaaaatcat ggcatcgagg atgaactgcc taattactgt tttcctctga 21300
   atggcatagg accagggcac acatatcaag gcattaaagt taaaaccgat gacactaatg 21360
   gatgggaaaa agatgctaat gttgctccag ctaatgaaat aaccataggc aacaacctgg 21420
   ctatggaaat taatatccaa gctaaccttt ggagaagttt tctgtactct aatgtggctt 21480
   tgtaccttcc agatgtttac aagtacacgc cacctaacat tactttgccc actaacacca 21540
   acacctatga gtacatgaac gggcgagtgg tatccccatc cctggttgat tcatacatca 21600
   acattggcgc caggtggtct cttgacccaa tggacaatgt gaatccattc aaccaccacc 21660
   gcaatgctgg tctgcgctac aggtccatgc ttctgggaaa tggtcgttat gtgcctttcc 21720
   acatacaagt gcctcagaaa ttctttgctg tcaagaacct acttcttcta cctggctcct 21780
   acacctacga gtggaacttc cgaaaggatg tgaacatggt cctgcaaagt tcccttggaa 21840
   atgacctcag aacggatggt gctaccataa gtttcaccag catcaatctc tatgccacct 21900
   tcttccccat ggctcacaac acagcttcca cccttgaagc catgctgcgc aacgatacca 21960
   atgatcagtc atttaacgac tacctctctg cagctaacat gctttacccc attcctgcca 22020
   atgcaaccaa cattccaatt tccatcccat ctcgcaactg ggcagccttc aggggctggt 22080
   ccttcaccag actcaaaacc aaggagactc catctcttgg atcagggttc gatccctact 22140
   tcgtatattc tggatctatt ccctacctgg atggcacctt ttaccttaac cacactttca 22200
   agaaggtctc catcatgttt gactcctcag tcagctggcc tggcaatgac aggctgttga 22260
   gtccaaatga gtttgaaatc aagcgcactg tggacgggga aggatacaac gtggcacaat 22320
   gcaacatgac caaagactgg ttcctggttc agatgcttgc caattacaac attggctacc 22380
   agggctttta catccctgag ggatacaagg atcgcatgta ctcctttttc agaaacttcc 22440
   agcctatgag caggcaggtg gttgatgagg ttaattacac tgactacaaa gccgtcacct 22500
   taccatacca acacaacaac tctggctttg tagggtacct tgcacctact atgagacaag 22560
   gggaacctta cccagccaat tatccatacc cgctcatcgg aactactgcc gttaagagtg 22620
   tcacccagaa aaagttcctg tgcgacagga ccatgtggcg cattcccttc tccagcaact 22680
   tcatgtccat gggggccctt accgacctgg gacagaacat gctctatgcc aactcagccc 22740
   atgcgctgga catgactttt gaggtggatc ccatggatga gcccaccctg ctttatcttc 22800
   ttttcgaagt cttcgacgtg gtcagagtgc accagccaca ccgcggcgtc atcgaggccg 22860
   tctacctgcg cacaccgttc tcggccggca acgccaccac ataagaagcc tcttgcttct 22920
   tgcaagcagc agctgcagcc atgacatgcg ggtccggaaa cggctccagc gagcaagagc 22980
   tcaaagccat cgtccgagac ctgggctgcg gaccctattt cctgggaacc tttgacaagc 23040
   gtttcccggg gttcatggcc cccgacaagc tcgcctgcgc catagtcaac actgccggac 23100
   gcgagacggg gggagagcac tggctggctt ttggttggaa cccgcgctac aacacctgct 23160
   acctttttga tccttttggg ttctcggatg agcggctcaa acagatttac cagtttgagt 23220
   acgaggggct cctgcgtcgc agtgcccttg ctaccaaaga ccgctgcatc accctggaga 23280
   agtctaccca aagcgtgcag ggtccgcgct cagccgcctg tggacttttt tgctgtatgt 23340
   tccttcatgc ctttgtgcac tggcccgacc gccccatgga cggaaacccc accatgaagt 23400
   tgctgactgg ggtgtccaac agcatgctcc aatcacccca agtccagccc accctgcgcc 23460
   gcaaccagga ggtgctatac cgcttcctaa acacccactc atcttacttt cgttctcacc 23520
   gcgcgcgcat tgaaagggcc accgcgtttg accgtatgga tatgcaataa gtcatgtaaa 23580
   accgtgttca ataaacagca ctttattttt acatgcactg aggctctggt tttgctcatt 23640
   tgtttcatca tttactcaga agtcgaatgg gttctggcgg gagtcagagt gacccgcggg 23700
   cagggatacg ttgcggaact gtaacctgtt ctgccacttg aactcgggga ttaccagctt 23760
   gggaactgga atctcgggaa aggtgtcttg ccacaacttt ctggtcagtt gcatagcgcc 23820
   aagcaggtca ggagcagaga tcttgaaatc acagttgggg ccggcattct ggacacggga 23880
   gttgcgatac actgggttgc aacactggaa cactatcaac gctgggtgtc ttacgcttgc 23940
   caacacggtt gggtcactga tggtagtcac atccaagtct tcagcattgg ccatcccaaa 24000
   gggggtcatc ttacatgtct gcctgcccat cacgggagcg cagcctggct tgtggttgca 24060
   atcacaatga atggggatca gcatcatcct ggcttggtcg ggagttatcc ctgggtacac 24120
   agccttcatg aaggcttcgt actgcttaaa agcttcctgg gccttacttc cctcggtgta 24180
   gaacatccca caggacttgc tggaaaattg attagtagta cagttggcat cattcacaca 24240
   acagcgggca tcgttgttgg ccaactgaac cacatttctg ccccagcggt tttgggtgat 24300
   cttggctctg tctggattct ccttcatagc gcgctgcccg ttctcgctcg ccacatccat 24360
   ctcgataatg tggtccttct ggatcatgat agtgccatgc aggcatttca ccttgccttc 24420
   ataatcggtg catccatgag cccacagagc gcacccggtg cactcccaat tattgtgggc 24480
   gatctcagaa taataatgta ccaatccctg catgaatctt cccatcattg ttgtcaaggt 24540
   cttcatgctg gtaaatgtca gcgggatgcc acggtgctcc tcgttcacat actggtggca 24600
   gatacgcttg tattgctcgt gctgctctgg catcagcttg aaagaggttc tcagatcatt 24660
   atccagcctg tacctttcca ttagcacagc catcacttcc atgcccttct cccaggcaga 24720
   taccaggggc agactcaaag gattcctaac agcaataaaa gtagctcctt tagctatagg 24780
   gtcattcttg tcgatcttct caacacttct cttgccatcc ttctcaatga tgcgcaccgg 24840
   ggggtagctg aagcccacgg ccaccaactg agcctgttct ctttcttctt cactatcctg 24900
   gctgatgtct tgcagaggga catgcttggt cttcctgggc ttcttcttgg gagggatcgg 24960
   gggaggactg ttgctccgct ccggagacag ggatgactgc gaagtttcgc ttaccaatac 25020
   cacctggctc tcggtagaag aaccggaccc cacacgacgg taggtgttcc tcttcggggg 25080
   cagaggtgga ggcgactgag atgggctgcg gtctggcctt ggaggcggat ggctggcaga 25140
   gctcattccg cgttcggggg tgtgctcccg gtggcggtcg cttgactgat ttcctccgcg 25200
   gctggccatt gtgttctcct aggcagagaa acaacagaca tggaaactca gccatcactg 25260
   ccaacatcgc tgcaagcacc atcacacctc gcccccagca gcgacgagga ggagagctta 25320
   accaccccac cacccagtcc cgctaccacc acctctaccc tcgatgatga ggaggaggtc 25380
   gacgcagccc aggagatgca ggcgcaggat aatgtgaaaa cggaagagat tgaggcagat 25440
   gtcgagcagg acccgggcta tgtgacgccg gcggagcacc aggaggagct gaaacgcttt 25500
   ctagacagag aggatgacga ccgcccagag catcaagcag atggcgttta ccaggaggct 25560
   gggatcaggg atcatgtcgc cgactacctc accggccttg gtggggagga cgtgctcctc 25620
   aaacatctag caaggcagtc gatcatagtt aaagacgcat tgctcgatct cactgaagtg 25680
   cccatcagtg tggaagagct tagccgcgcc tacgagctga acctcttttc gcctcaggta 25740
   ccccccaagc ggcagccaaa cggcacctgc gaggccaacc ctcgactcaa cttctatcca 25800
   gcttttacta tccccgaagt gttggccacc taccacatct ttttcaagaa ccaaaagatt 25860
   ccagtctcct gccgcgccaa ccgcacccgc gccgatgccc tgctcaactt gggtccggga 25920
   gctcgcttac ctgatatagc ttccttggaa gaggttccaa agatctttga gggtctggga 25980
   agtgatgaga cacgggccgc aaatgctctg caacagggag agaatgacat ggatgaacac 26040
   cacagcgctc tggtggaact ggagggtgac aatgcccgga ttgcagtgct caagcgcagt 26100
   atcgtggtca cccattttgc ctaccccgct gttaacctgc cccccaaagt tatgagcgct 26160
   gtcatggacc atctgctcat caaacgagca agacctcttt cagaaaacca gaacatgcag 26220
   gatccagacg cctcggacga gggcaagccg gtagtcagtg acgagcagct atctcgctgg 26280
   ctgggtacca actccccccg agatttggaa gagaggcgca agcttatgat ggctgtagtg 26340
   ctagtaactg tggagctgga gtgtctgcgc cgctttttca ccgaccctga gaccctgcgc 26400
   aagctagagg agaacctgca ctacaccttt agacatggct tcgtgcggca ggcatgcaag 26460
   atctccaacg tggagcttac caacctggtt tcttacatgg gcattttgca tgagaaccga 26520
   ctagggcaga gcgtcctgca caccaccctt aaaggggagg cccgccgtga ctacatccga 26580
   gactgtgtct acctctacct ctgccatacc tggcaaactg gtatgggtgt gtggcaacag 26640
   tgtttggaag agcagaacct aaaagagctg gacaagctct tgcagagatc cctcaaagcc 26700
   ctgtggacag gttttgatga gcgcaccgtc gcctcggacc tggcagacat catcttcccc 26760
   gagcgtctca gggttactct gcgaaacggc ctgccagact ttatgagcca gagcatgctt 26820
   aacaactttc gctctttcat cctggaacgc tccggtatcc tgcctgccac ctgctgtgcg 26880
   ctgccctccg actttgtgcc tctcacctac cgcgagtgcc caccgccgct atggagccac 26940
   tgctacctgt tccgcctggc caactacctc tcctaccact cggatgttat agaggatgtg 27000
   agcggagacg gcctgctgga atgccactgc cgctgcaatc tttgcacacc ccaccgctcc 27060
   cttgcctgca acccccagtt gctgagcgag acccagatta tcggcacctt cgagctgcag 27120
   ggtcccagaa gtaaaggcga ggggtcttct ccggggcaga gtttgaaact gacaccgggg 27180
   ctgtggacct ccgcctacct gcgcaagttt caccccgagg actaccatcc ctatgagatc 27240
   aggttctatg aggaccaatc acatcctccc aaagtcgagc tctcagcctg cgtcatcacc 27300
   cagggagcaa ttctggccca attgcaagcc atccaaaaat ctcgccaaga atttctgcta 27360
   aaaaagggaa acggggtcta ccttgaccct cagaccggtg aggagctcaa cacaaggttc 27420
   ccccaggatg tcccatcgcc gaggaagcaa gaagttgaag gtgcagctgt cgcccccaga 27480
   ggatatgaag gaagactggg acagtcaggc agaggaggag atggaagatt gggacagcca 27540
   ggcagaggag gtggacagcc tggaggaaga cagtttggag gaggaagacg aggaggcaga 27600
   ggaggtggaa gaagcaaccg ccgccaaaca gttgtcatcg gcggcggaga caagcaagtc 27660
   cccagacagc agcacggcta ccatctccgc tccgggtcgg ggggcccagc ggcggcccaa 27720
   cagtagatgg gacgagaccg ggcgattccc aaacccgacc accgcttcca agaccggtaa 27780
   gaaggagcga cagggataca agtcctggcg tggacataaa aacgctatca tctcctgctt 27840
   gcatgaatgc gggggcaaca tatccttcac ccggcgatac ctgctcttcc accacggtgt 27900
   aaacttcccc cgcaatatct tgcattacta ccgtcacctc cacagcccct actgcagtca 27960
   gcaagtcccg gcaaccccga cagaaaaata cagcagcgac aacggtgacc agaaaaccag 28020
   cagttagaaa atccacaaca agtgcaccag gaggaggact gaggatcaca gcgaacgagc 28080
   cagcgcagac cagagagctg aggaaccgga tctttccaac cctctatgcc attttccagc 28140
   agagtcgggg gcaagagcag gaactgaaag taaaaaaccg atctctgcgc tcgctcacca 28200
   gaagttgttt gtatcacaag agcgaagacc aacttcagcg cactctcgag gacgccgagg 28260
   ctctcttcaa caagtactgc gcgctgactc ttaaagagta gcccttgccc gcgctcattt 28320
   tgaaaacggc gggaatcacg tcacccttgg cacctgtcct ttgcccttgt catgagtaaa 28380
   gagattccca cgccttacat gtggagctat cagccccaaa tggggttggc agcaggcgct 28440
   tcccaggact actccacccg catgaattgg cttagcgccg ggccctcaat gatatcacgg 28500
   gttaatgata tacgagctta tcgaaaccag ttactcctag aacagtcagc tctcaccacc 28560
   acaccccgtc aacaccttaa tccccgaaat tggcccgcca ccctggtgta ccaggaaaat 28620
   cccgctccca ccaccgtact acttcctcga gacgcccagg ccgaagttca gatgactaac 28680
   gcaggtgtac agctggcggg cggttccgcc ctatgtcgtc accgacctca acagagtata 28740
   aaacgcctgg tgattagagg ccgaggtatc cagctcaacg acgagtcggt tagctcttcg 28800
   cttggtctgc gaccagacgg agtcttccaa atcgccggct gtgggagatc ttccttcact 28860
   cctcgtcagg ctgtgctgac tttggagagt tcgtcctcgc agccccgctc gggcggcatt 28920
   ggaactctcc agtttgtgga ggagtttact ccctctgtct acttcaaccc cttctccggc 28980
   tctcctggcc agtacccgga cgagttcata ccaaacttcg acgcaatcag cgagtcagtg 29040
   gatggctatg attgatgtct aatggtggtg cggctgagct agctcgactg cgacacctag 29100
   accactgccg ccgctttcgc tgcttcgccc gggaactcac cgagttcatc tacttcgaac 29160
   tctccgagga gcaccctcag ggtccggccc acggagtgcg gattaccatc gaagggggaa 29220
   tagactctcg cctgcatcgc atcttctccc agcggcccgt gctaattgaa cgcgaccagg 29280
   gaaatacaac catctccatc tactgcatct gtaaccaccc cggattgcat gaaagccttt 29340
   gctgtcttgt ttgtgctgag tttaataaaa actgagttaa gaccctccta cggactaccg 29400
   cttcttcaat caggacttta caacaccaac cagatcttcc agaagaccca gacccttcct 29460
   cctttcatcc aggactctaa ctctacctta ccagcaccct ccactactaa ccttcccgaa 29520
   acaaacaagc ttgcatctca tctgcaacac cgcctttcac gaagccttct ttctgccaat 29580
   actaccactc ccaaaaccgg aggtgagctc cgcggtcttc ctactgacga cccctgggtg 29640
   gtagcgggtt ttgtaacgtt aggagtagtt gcgggtgggc ttgtgctgat cctttgctac 29700
   ctatacacac cttgctgtgc atatttagtc atattgtgct gttggtttaa gaaatggggg 29760
   ccatactagt cgtgcttgct ttactttcgc ttttgggtct gggctctgct aatctcaatc 29820
   ctctcgatca cgatccatgt ttagacttcg acccagaaaa ctgcacactt acttttgcac 29880
   ccgacacaag ccgtctctgt ggagttctta ttaagtgcgg atgggactgc aggtccgttg 29940
   aaattacaca taataacaaa acatggaaca ataccttatc caccacatgg gagccaggag 30000
   ttccccagtg gtatactgtc tctgtccgag gtcctgacgg ttccatccgc attagtaaca 30060
   acactttcat tttttctgaa atgtgcgatc tggccatgtt catgagcaga cagtatgacc 30120
   tatggcctcc cagcaaagag aacattgtgg cattttccat tgcttattgc ttggtaacat 30180
   gcatcatcac tgctatcatt tgtgtgtgca tacacttgct tatagttatt cgccctagac 30240
   aaagcaatga ggaaaaagag aaaatgcctt aacctttttc ctcatacctt ttctttacag 30300
   catggcttct gttacagctc taattattgc cagcattgtc actgtcgctc acgggcaaac 30360
   aattgtccat attaccttag gacataatca cactcttgta gggcccccaa ttacttcaga 30420
   ggttatttgg accaaacttg gaagtgttga ttattttgat ataatttgca acaaaactaa 30480
   accaatattt gtaatctgta acagacaaaa tctcacgtta attaatgtta gcaaaattta 30540
   taacggttac tattatggtt atgacagatc cagtagtcaa tataaaaatt acttagttcg 30600
   cataactcag cccaaattaa cagtgccaac tatgacaata attaaaatgg ctaataaagc 30660
   attagaaaat tttacatcac caacaacacc caatgaaaaa aacattccaa attcaatgat 30720
   tgcaattatt gcggcggtgg cattgggaat ggcactaata ataatatgca tgctcctata 30780
   tgcttgttac tataaaaagt ttcaacataa acaggatcca ctactaaatt ttaacattta 30840
   attttttata cagatgattt ccactacaat ttttatcatt actagccttg cagctgtaac 30900
   ttatggccgt tcacacctaa ctgtacctgt tggctcaaca tgtacactac aaggacccca 30960
   agaaggctat gtcacttggt ggagaatata tgataatgga gggttcgcta gaccatgtga 31020
   tcagcctggt acaaaatttt catgcaacgg aagagacttg accattatta acataacatc 31080
   aaatgagcaa ggcttctatt atggaaccaa ctataaaaat agtttagatt acaacattat 31140
   tgtagtgcca gccaccactt ctgctccccg caaatccact ttctctagca gcagtgccaa 31200
   agcaagcaca attcctaaaa cagcttctgc tatgttaaag cttccaaaaa tcgctttaag 31260
   taattccaca gccgctccca atacaattcc taaatcaaca attggcatca ttactgccgt 31320
   ggtagtggga ttaatgatta tatttttgtg tataatgtac tacgcctgct gctatagaaa 31380
   acatgaacaa aaaggtgatg cattactaaa ttttgatatt taatttttta tagaattatg 31440
   atattgtttc aatcaaatac cactacctcc tatgcataca caaacattca gcctaaatac 31500
   gctatgcaac tagaaatcac aatactaatt gtaattggaa ttcttatact atctgttatt 31560
   ctttatttta tattctgccg tcaaataccc aatgttcata gaaattctaa aagacgtccc 31620
   atctattctc ctatgattag tcgtccccat atggctctga atgaaatcta agatcttttt 31680
   ttttttctct tacagtatgg tgaacatcaa tcatgatccc tagaaatttc ttcttcacca 31740
   tactcatctg tgcttttaat gtctgtgcta ctttcacagc agtagccact gcaagcccag 31800
   actgtatagg accatttgct tcctatgcac tttttgcctt cgttacttgc atctgcgtgt 31860
   gtagcatagt ctgcctggtt attaattttt tccaactggt agactggatc tttgtgcgaa 31920
   ttgcctacct acgtcaccat cccgaatacc gcaatcaaaa tgttgcggca cttcttaggc 31980
   ttatttaaaa ccatgcaggc tatgctacca gtcattttaa ttttgctact accctgcatt 32040
   cccctagctt ccaccgccac tcgcgctaca cctgaacaac ttagaaaatg caaatttcaa 32100
   caaccatggt catttcttga ttgctaccat gaaaaatctg attttcccac atactggata 32160
   gtgattgttg gaataattaa catactttca tgtacctttt tctcaatcac aatatacccc 32220
   acatttaatt ttgggtggaa ttctcccaat gcactgggtt acccacaaga accagatgaa 32280
   catattccac tacaacacat acaacaacca ctagcactgg tacagtatga aaatgagcca 32340
   caaccttcac tgccccctgc cattagttac ttcaacctaa ccggcggaga tgactgaccc 32400
   aatcgccaca tcatccaccg ctgccaagga gctgctggac atggacggac gtgcctcaga 32460
   acagcgactc atccaactac gcattcgtca gcagcaggaa cgagcagtaa aagagctaag 32520
   ggatgccatt gggattcacc agtgcaaaaa aggcatattc tgcttagtaa aacaatccaa 32580
   aatctcctac gagatcaccg ctactgacca tcgtctctca tacgagctcg gtccgcagcg 32640
   acaaaaattc acctgcatgg tgggaatcaa ccccatagtt atcacccagc agtctggaga 32700
   tactaagggt tgtatccagt gttcctgtga ttccaccgag tgcatctaca cactgctgaa 32760
   gaccctctgc ggccttcgag acctcctacc catgaactaa tcattgcccc taccttaccc 32820
   aatcaaaata ttaataaaga cacttacttg aaatcagcaa tacagtcttt gtcaaaactt 32880
   tctaccagca gcacctcacc ctcttcccaa ctctggtact ctaaacgtcg gagggtggca 32940
   tactttctcc acactttgaa agggatgtca aattttattt cctcttcttt gcccacaatc 33000
   ttcatttctt tatccccaga tggccaagcg agctcggcta agcacttcct tcaacccggt 33060
   gtacccttat gaagatgaaa gcagctcaca acacccattt ataaatcctg gtttcatttc 33120
   ccctgacggg ttcacacaaa gtccaaacgg ggttttaagt cttaaatgtg ttaatccact 33180
   taccactgca agcggctccc tccaacttaa agtgggaagt ggtcttacag tagacactac 33240
   tgatggatcc ttagaagaaa acatcaaagt taacaccccc ctaacaaagt caaaccattc 33300
   tataaattta ccaataggaa acggtttgca aatagaacaa aacaaacttt gcagtaaact 33360
   cggaaatggt cttacatttg actcttccaa ttctattgca ctgaaaaata acactttatg 33420
   gacaggtcca aaaccagaag ccaactgcat aattgaatac gggaaacaaa acccagatag 33480
   caaactaact ttaatccttg taaaaaatgg aggaattgtt aatggatatg taacgctaat 33540
   gggagcctca gactacgtta acaccttatt taaaaacaaa aatgtctcca ttaatgtaga 33600
   actatacttt gatgccactg gtcatatatt accagactca tcttctctta aaacagatct 33660
   agaactaaaa tacaagcaaa ccgctgactt tagtgcaaga ggttttatgc caagtactac 33720
   agcgtatcca tttgtccttc ctaatgcggg aacacataat gaaaattata tttttggtca 33780
   atgctactac aaagcaagcg atggtgccct ttttccgttg gaagttactg ttatgcttaa 33840
   taaacgcctg ccagatagtc gcacatccta tgttatgact tttttatggt ccttgaatgc 33900
   tggtctagct ccagaaacta ctcaggcaac cctcataacc tccccattta ccttttccta 33960
   tattagagaa gatgactgac aacaaaaata aagttcaaca ttttttattg aaattccttt 34020
   tacagtattc gagtagttat tttgcctccc ccttcccatt taacagaata caccaatctc 34080
   tccccacgca cagctttaaa catttggata ccattagaga tagacatagt tttagattcc 34140
   acattccaaa cagtttcaga gcgagccaat ctggggtcag taatacataa aaatgcatcg 34200
   ggatagtctt ttaaagcgct ttcacagtcc aactgttgcg gatgcgactc cggagtctga 34260
   atcacggtca tctggaagaa gaacgatggg aatcataatc cgaaaacgga atcgggcgat 34320
   tgtgtctcat caaacccaca agcaaccgct gtctgcgtcg ctccgtgcga ctgctgttta 34380
   tgggatcggg gtccgcagtg tcctgaagca tgattttaat agcccttaac attaactttc 34440
   tggtgcgatg cgcgcagcaa cgcattctga tttcacttag attactacag taggtacagc 34500
   acattatcac aatattgttt aataaaccat aattaaaagc gctccagcca aaactcatat 34560
   ctgatataat cgcccctgca tgaccatcat accaaagttt aatataaatt aaatgtcgtt 34620
   ccctcaaaaa cacactaccc acatacatga tctcttttgg catgtgcata ttaacaattt 34680
   gtctgtacca tggacaacgt tggttaatca tgcaacccaa tataaccttc cggaaccaca 34740
   ctgccaacac cgctccccca gccatgcatt gaagtgaacc ctgctgatta caatgacaat 34800
   gaagaaccca attctctcga ccatgaatca cttgagactg aaaaatatct atagtagcac 34860
   aacaaagaca taaatgcatg catcttctca taatttttaa ctcctctgga tttaaaaaca 34920
   tatcccaagg aatgggaaac tcttgcagaa cagtaaagct ggcagaacaa ggaagaccac 34980
   gaacacaact tacactatgc atagtcatag tatcacaatc tggcaacagc gggtggtctt 35040
   cagtcataga agctcgggtt tcattttcct cacatcgtgg taattgggct ctggtgtaag 35100
   ggtgatgtct ggcgcatgat gtggagcgtg cgcgcaacct tgtcataatg gagttgcttc 35160
   ctgacattct cgtattttgt atagcaaaac gctgccctgg cacaacacac tcttcttcgt 35220
   cttctatcct gccgcttagt gtgttccgtc tgataattca agtacagcca cactcttaag 35280
   ttggtcaaaa gaatgctggc ttcagttgta atcaaaactc catcatattt aattgttcta 35340
   aggaaatcat ccacggtagc atatgcaaat cccaaccaag caatgcaact ggattgcgtt 35400
   tcaagcagca gaggagaggg aagagacgga agaatcatgt taatttttat tccaaacgat 35460
   ctcgcagtac ttcaaattgt agatcgcgca gatggcatct atcgccccca ctgtgttggt 35520
   gaaaaagcac agctaaatca aaagaaatgc gattttcaag gtgctcaacg gtggcttcca 35580
   acaaagcctc cacgcgcaca tccaaaaaca aaagaatacc aaaagaagga gcattttcta 35640
   actcctcaaa catcatatta cattcctgca ccattcccag ataattttca gctttccagc 35700
   cttgaattat tcgtgtcagt tcttgtggta aatccaaacc acacattaca aacaggtccc 35760
   ggagggcgcc ctccaccacc attcttaaac acaccctcat aatgacaaaa tatcttgctc 35820
   ctgtgtcacc tgtagcaaat taagaatggc atcatcaatt gacatgccct tggctctaag 35880
   ttcttctcta agttctagtt gtagatactc tctcatatta tcaccaaact gcttagccag 35940
   aagccccccg ggaacaatag caggggacgc tacagtgcag tacaagcgca gacctcccca 36000
   attggctcca gcaaaaacaa gattagaata agcatactgg gaaccaccag taatatcatc 36060
   aaagttgctg gaaatataat caggcagagt ttcttgtaaa aattgaataa aagaaaaatt 36120
   ttccaaagaa acattcaaaa ccgttgggat gcaaatacaa taggttaccg cgctgcgctc 36180
   caacattgtt agttttgaat tagtctgcaa aataaaagaa acaagcgtca tatcatagta 36240
   gcctgtcgaa caggtggaaa aatcagtctt tccatcacaa gacaagccac agggtctcca 36300
   gctcgaccct cgtaaaacct gtcattgtga ttaaacaaca gcaccgaaag ttcctcgcgg 36360
   tggccagcat gaataattct tgatgaagca tacaatccag acatgttagc atcagttaaa 36420
   gagaaaaaac agccaacata gcctctgggt ataattatgc ttaattttaa gtatagcaaa 36480
   gccacccctc gcggatacaa agtaaaaggc acaggagaat aaaaaatata attatttctc 36540
   tgctgctgtt caggcaacgt tgctcccggt ccctctaaat agacatacaa agcctcatca 36600
   gccatggctt accaggcaaa gtacagcggg cgcacaaagc acaagctcta aagaagctct 36660
   aaaaacactc tccaacctct ccacaatata tacacaagcc ctaaactgac gtaatgggag 36720
   taaagtgaaa aaaaaatacc gccaagccca acacacaccc cgaaactgcg tcagcaggaa 36780
   aaagtacagt ttcacttccg cattcccaac aagcgtaact tcctctttct catggtacgt 36840
   cacatccgat taacttgcaa cgtcattttc ccacggtcgc gccgcccctt ttagccgtta 36900
   accccgcagc caatcaccaa ttt 36923
<210> 21
   <211> 770
   <212> DNA
   <213> Artificial Sequence
<220>
   <223> CD40L
<400> 21
   atgatcgaaa catacaacca aacttctccc cgatctgcgg ccactggact gcccatcagc 60
   atgaaaattt ttatgtattt acttactgtt tttcttatca cccagatgat tgggtcagca 120
   ctttttgctg tgtatcttca tagaaggctg gacaagatag aagatgaaag gaatcttcat 180
   gaagattttg tattcatgaa aacgatacag agatgcaaca caggagaaag atccttatcc 240
   ttactgaact gtgaggagat taaaagccag tttgaaggct ttgtgaagga tataatgtta 300
   aacaaagagg agacgaagaa agaaaacagc tttgaaatgc aaaaaggtga tcagaatcct      360
   caaattgcgg cacatgtcat aagtgaggcc agcagtaaaa caacatctgt gttacagtgg      420
   gctgaaaaag gatactacac catgagcaac aacttggtaa ccctggaaaa tgggaaacag      480
   ctgaccgtta aaagacaagg actctattat atctatgccc aagtcacctt ctgttccaat      540
   cgggaagctt cgagtcaagc tccatttata gccagcctct gcctaaagtc ccccggtaga      600
   ttcgagagaa tcttactcag agctgcaaat acccacagtt ccgccaaacc ttgcgggcaa      660
   caatccattc acttgggagg agtatttgaa ttgcaaccag gtgcttcggt gtttgtcaat      720
   gtgactgatc caagccaagt gagccatggc actggcttca cgtcctttgg                 770

## Description

### Field of the invention

The present invention relates to the field of medicine. Specifically, the invention relates to cancer therapies. More specifically, the present invention relates to oncolytic human adenoviral vectors and cells and pharmaceutical compositions comprising said vectors. The present invention also relates to a use of said vectors in the manufacture of a medicament for treating cancer in a subject. Furthermore, the present invention relates to a method of producing an adenoviral vector.

### Background of the invention

Cancer can be treated with surgery, hormonal therapies, chemotherapies and/or radiotherapies but in many cases, cancers, which are often characterized by an advanced stage, cannot be cured with present therapeutics. Therefore, novel cancer cell targeted approaches such as gene therapies are needed.

During the last twenty years gene transfer technology has been under intensive examination. The aim of cancer gene therapies is to introduce a therapeutic gene into a tumor cell. These therapeutic genes introduced to a target cell may for example correct mutated genes, suppress active oncogenes or generate additional properties to the cell. Suitable exogenous therapeutic genes include but are not limited to immunotherapeutic, anti-angiogenic, chemoprotective and "suicide" genes, and they can be introduced to a cell by utilizing modified virus vectors or non-viral methods including electroporation, gene gun and lipid or polymer coatings.

Requirements of optimal viral vectors include an efficient capability to find specific target cells and express the viral genome in the target cells. Furthermore, optimal vectors have to stay active in the target tissues or cells. All these properties of viral vectors have been developed during the last decades and for example retroviral, adenoviral and adeno-associated viral vectors have been widely studied in biomedicine.

To further improve tumor penetration and local amplification of the anti-tumor effect, selectively oncolytic agents, e.g. conditionally replicating adenoviruses, have been constructed. Oncolytic adenoviruses are a promising tool for treatment of cancers. Tumor cells are killed by oncolytic adenoviruses due to a replication of the virus in a tumor cell, the last phase of the replication resulting in a release of thousands of virions into the surrounding tumor tissues for effective tumor penetration and vascular re-infection. Treatment approaches take advantage of molecular differences between the normal and tumor cells. Tumor cells allow replication of the virus while normal cells are spared due to engineered changes in the virus genome, which prevent replication in non-tumor cells. An optimal oncolytic adenovirus would infect and replicate only in cancer cells and would be potent enough to kill all cancer cells before the immunological response neutralizes the virus.

Making certain deletions or adding tissue specific promoters can make the virus more selective while adding transgenes can make the approach more potent. Indeed, in addition to replication mediated cell killing, oncolytic adenoviruses can also be armed with different therapeutic transgenes. This approach combines the advantages of conventional gene delivery with the potency of replication competent agents. One goal of arming viruses is induction of an immune reaction towards the cells that allow virus replication. Virus replication alone, although immunogenic, is normally not enough to induce effective anti-tumor immunity. To strengthen induction of therapeutic immunity, viruses can be armed with stimulatory proteins such as cytokines and by facilitating the introduction of tumor antigens to dendritic cells. Introduction of immunotherapeutic genes into tumor cells and furthermore, translation of the proteins, leads to activation of the immune response and efficient destruction of tumor cells.

Adenoviruses are medium-sized (90-100nm), nonenveloped icosahedral viruses, which have double stranded linear DNA of about 36 kilo base pairs in a protein capsid. The viral capsid has fiber structures, which participate in attachment of the virus to the target cell. First, the knob domain of the fiber protein binds to the receptor of the target cell (e.g. CD46 or coxsackievirus adenovirus receptor (CAR)), secondly, the virus interacts with an integrin molecule and thirdly, the virus is endocytosed into the target cell. Next, the viral genome is transported from endosomes into the nucleus and the replication machinery of the target cell is utilized also for viral purposes (Russell, W.C. 2000, J General Virol 81, 2573-2604).

The adenoviral genome has early (E1-E4), intermediate (IX and IVa2) and late genes (L1-L5), which are transcribed in sequential order. Early gene products affect defense mechanisms, cell cycle and cellular metabolism of the host cell. Intermediate and late genes encode structural viral proteins for production of new virions (Wu and Nemerow, 2004, Trends Microbiol 12: 162-168; Russell, W.C. 2000, J General Virol 81, 2573-2604; Volpers, C. and Kochanek, S. 2004, J Gene Med 6 suppl 1, S164-71; Kootstra, N.A. and Verma, I.M. 2003, Annu Rev Pharmacol Toxicol 43, 413-439).

More than 50 different serotypes of adenoviruses have been found in humans. Serotypes are classified into six subgroups A-F and different serotypes are known to be associated with different conditions i.e. respiratory diseases, conjunctivitis and gastroenteritis. Nevertheless, all cancer trials utilizing recombinant adenoviruses have featured subgroup C viruses, which in most cases has been Ad5. Advantages of serotype chimeric capsids in the context of an otherwise serotype 5 virus have also been recognised. Even though serotype chimerism allows partial escape from neutralizing antibodies (Nab) existing against Ad5, the fact that most of the virus capsid is still from Ad5 renders the escape incomplete (Särkioja, M. et al. 2008, Gene Ther 15: 921-929).

Thus, there is a need for a fully non-Ad5 oncolytic virus, which is able to escape from Nab existing against Ad5. These viruses are useful especially in the context of pre-existing NAb against Ad5. This situation might arise as a result of natural infection or subsequent to treatment with an Ad5 based oncolytic virus.

Currently, little has been published on serotype 3 adenoviruses in the context of gene therapy. Wild type Ad3 is known to cause mainly respiratory infections and conjuctivitis in humans. The complete DNA sequence was reported in 2005 and it has only 62.75% identity with serotype 5. The genomic organisation is similar to other human adenoviruses having early and delayed early transcription units, including late and major late units (Sirena D et al. 2005, Virology 343: 283-298). A few years ago a wild type Ad3 virus was studied under normoxic and hypoxic conditions (Shen, B.H. et al. 2006, Gene Ther 13: 986-990). Recently, the same group published ColoAd1, a complex Ad3/Ad11p chimeric virus (Kuhn I et al. 2008, PLoS ONE 3: e2409). ColoAd1 was made by pooling an array of serotypes, then passaging the pools under conditions that invite recombination between serotypes. The authors call this method "Directed Evolution". These highly diverse viral pools were then placed under stringent directed selection to generate and identify potent agents. In their experiments they found out that the Ad3/Ad11p chimeric virus was 2-3 orders of magnitude more potent and selective than the parent serotypes or the most clinically advanced oncolytic Ad, ONYX-015, *in vitro.* These results were further supported by *in vivo* and *ex vivo* studies. ColoAd1, however, given the approach utilized to develop it, lacks a rational basis for tumor selectivity. Also, its activity and safety *in vivo* is poorly understood as it has only been studied in one animal model, featuring colon cancer xenografts in mice.

The document WO2005010149 describes a.o. human type 3 adenovirus and uses thereof, e.g. for ablating neoplastic cells in a cell population.

The document US2005214923 describes oncolytic human adenoviral vectors comprising the E1A region under an hTERT promoter (see e.g. Example 4: OV1025).

The document WO2005030261 describes oncolytic human adenoviral vectors e.g. wherein expression of the E1A region is controlled by a promoter from the human telomerase (hTERT) gene.

Therefore, more efficient and accurate gene transfer based on non-Ad5 vectors as well as increased specificity and sufficient tumor killing ability of non-Ad5 gene therapies are warranted. Safety records of therapeutic vectors must also be excellent. The present invention provides a cancer therapeutic tool with the aforementioned properties by utilizing oncolytic human non-Ad5 viruses.

### Brief description of the invention

The invention in its broadest sense is as characterized in the independent claims.

The object of the disclosure is to provide novel methods and means for achieving the above-mentioned properties of adenoviruses and thus, solving the problems of current cancer therapies. More specifically, the invention provides novel methods and means for gene therapy as characterized in the claims.

The present application describes construction of oncolytic human Ad3 vectors and their use in tumor cells lines and animal models, and it provides an alternative for oncolytic serotype 5 adenoviral vectors in virotherapies. The vector of the invention is the first selectively oncolytic human adenovirus based only on serotype 3, as characterized in the claims. The vector is also the first non-Ad5 based oncolytic adenovirus controlled by a tumor specific promoter, as characterized in the claims.

The present invention relates to a fully serotype 3 oncolytic human adenoviral vector, as characterized in the claims.

The present invention further relates to a cell comprising the adenoviral vector of the invention, as characterized in the claims.

The present invention also relates to a pharmaceutical composition comprising the adenoviral vector of the invention, as characterized in the claims.

Furthermore, the present invention relates to a use of the adenoviral vector of the invention in the manufacture of a medicament for treating cancer in a subject, as characterized in the claims.

Also, the present invention relates to the vector or pharmaceutical composition of the invention as an in situ cancer vaccine. Also, the present invention relates to a vector of the invention for use in the treatment of cancer in a subject.

Still, the present invention relates to a method as recited in the claims of producing an adenoviral vector of the invention comprising:
i) providing a DNA vector comprising at least partial Ad3 DNA and optionally one or several promoters and/or optionally one or several transgenes, and
ii) inserting the remainder of the Ad3 genome and optionally one or several promoters and/or optionally one or several transgenes to the vector.

The present invention provides a tool for treatment of cancers, which are refractory to current approaches. Also, restrictions regarding tumor types suitable for treatment remain few in the present invention compared to many other treatments. In fact, all solid tumors may be treated with the proposed invention. The treatment can be given intratumorally, intracavitary, intra-arterially, intravenously and in a combination of these.

Ad3 based viruses are able to enter cancer stem cell type cells better than Ad5 viruses. Efficacy of the virus of the present invention was shown in tumor cell lines and the virus was at least as potent as Ad5 or Ad5/3 based controls in several murine models of human cancer.

Besides enabling the transport of the vector to the site of interest the vector of the invention may also assure the expression and persistence of a transgene(s). Induction of an immune reaction towards cells that allow replication of unarmed viruses is normally not strong enough to lead to development of therapeutic tumor immunity. In order to overcome this weakness, the present invention provides armed viruses with a potent inducer of anti-tumor immunity (e.g. GM-CSF), as recited in the claims.

The present invention also relates to human sodium iodide symporter (hNIS) transgene as recited in the claims. Said in hNIS may target radioiodide to the target cell. This approach allows tumor cells to be killed due to the oncolytic effect of the virus and due to radiation induced cell death. The approach also takes advantage of synergy between radiation and oncolytic adenovirus replication. By introducing hNIS as a transgene, radioiodide therapy can be used to treat cancers of non-thyroid origin. An additional useful aspect of hNIS is non-invasive imaging of transgene expression, which allows monitoring viral spread and persistence.

The present invention also solves a problem related to therapeutic resistance of conventional treatments as well as other adenoviral therapies. The use as claimed or the vector for the use as claimed may be for administration to the highly potential subject group of patients with high anti-Ad5 Nab titers or previously treated with Ad5 agents.

Furthermore, the vectors and uses as characterized in the claims provide for selective treatments, without toxicity or damages in healthy tissues. Also, advantages of the present invention may include different and reduced side effects in comparison to other therapeutics. Importantly, the approach is synergistic with many other forms of therapy including chemotherapy and radiation therapy, and can therefore be used in combination regimens.

Compared to adenoviral tools of the prior art, the present invention provides a more simple, more effective, inexpensive, non-toxic and/or safer tool for cancer therapy. Furthermore, helper viruses are not needed.

The novel products of the invention enable further improvements in cancer therapy.

### Brief description of the figures

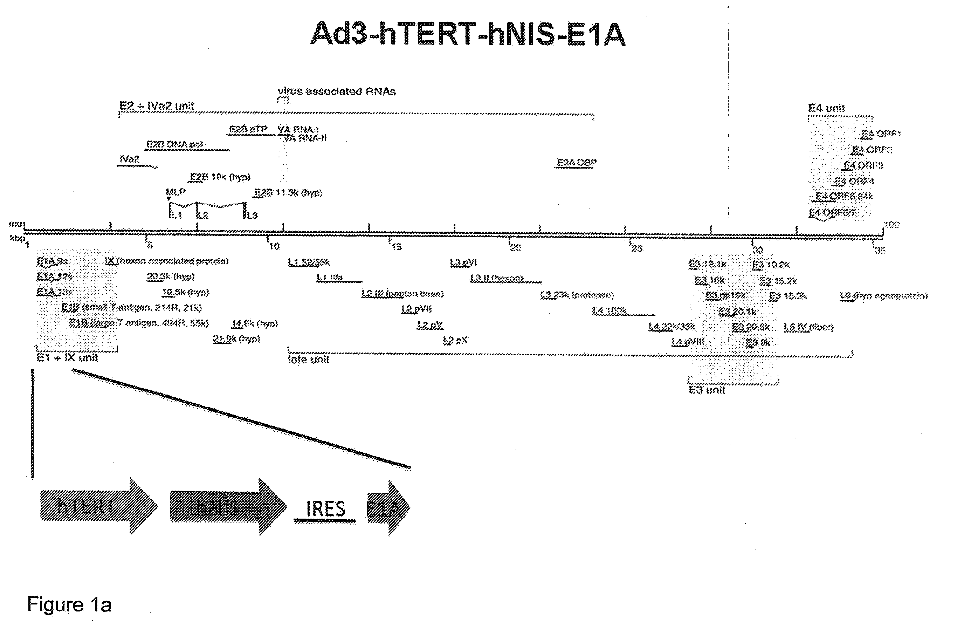

Figures 1a-e show schemas of Ad3-hTERT-hNIS-E1A (SEQ ID NO. 14); hNIS (SEQ ID NO. 15), Ad3-hTERT-GMCSF-E1A (SEQ ID NO. 16); GM-CSF (SEQ ID NO. 17), Ad3-hTERT-E1A-E3-hNIS, Ad3-hTERT-E1A-E3-GMCSF, and Ad3-hTERT-CD40L-E1A (SEQ ID NO. 20) partial; CD40L (SEQ ID NO 21).

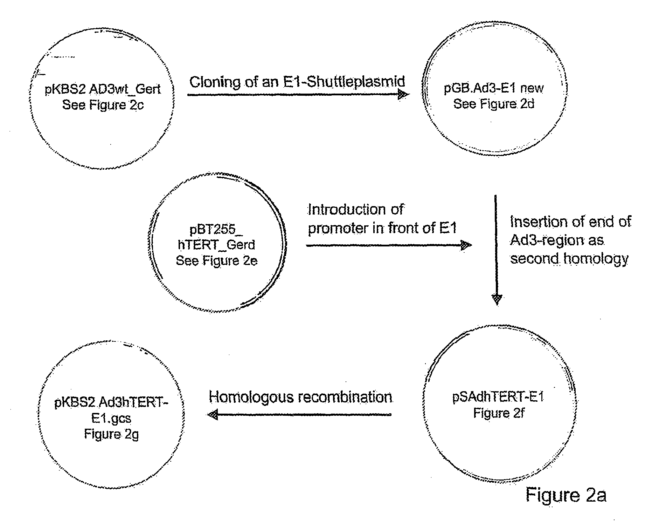

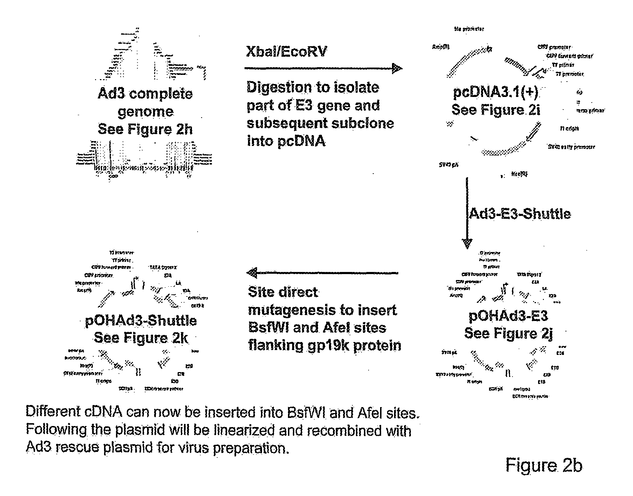

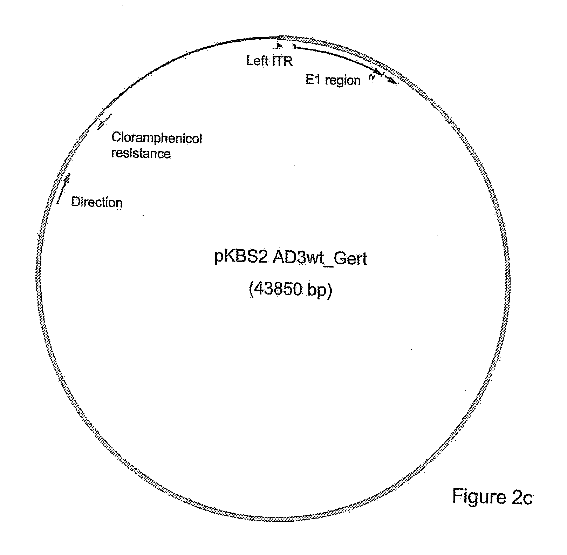

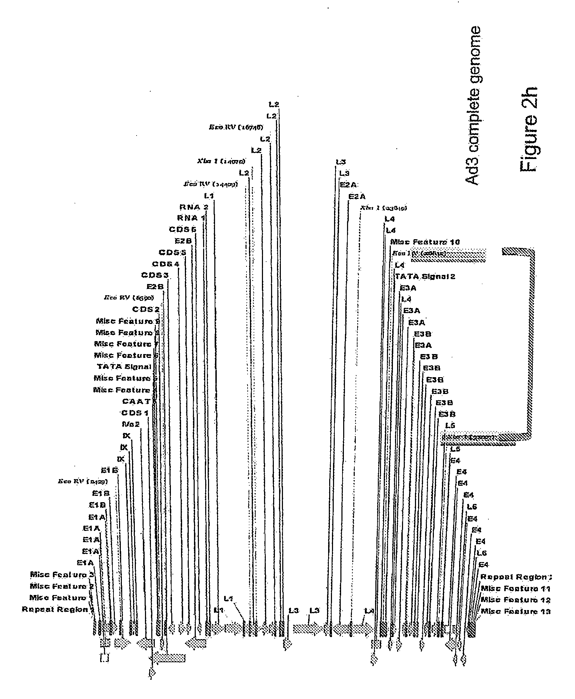

Figures 2a-k show the cloning of viruses described in Figures 1a-e. The cloning of viruses with the transgenes in E1A is demonstrated in Figures 2a and 2c-g, the cloning of viruses with transgenes in the deleted Ad3 E3gp19k locus is demonstrated in Figures 2b and 2h-k.

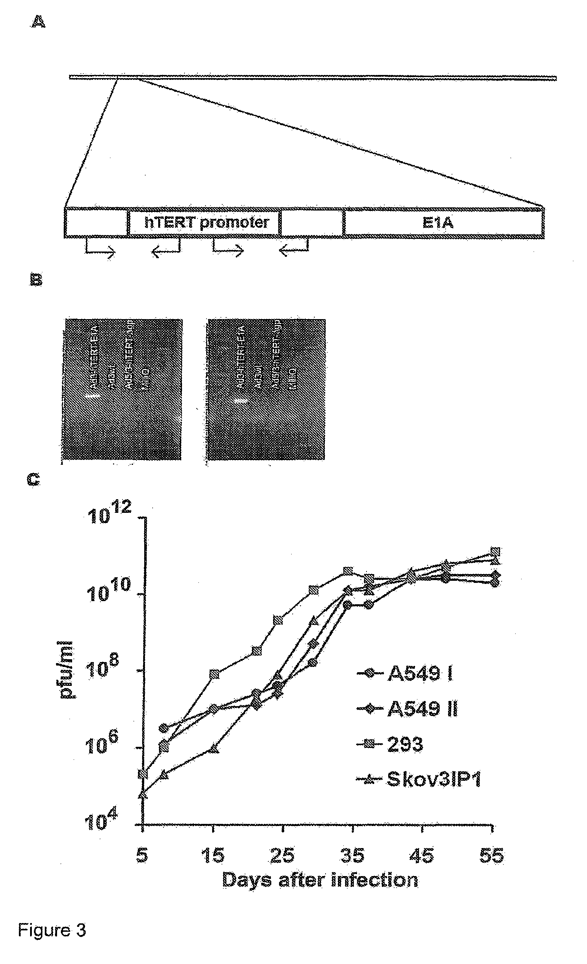

Figure 3 shows a) the structure of Ad3-hTERT-E1A (SEQ ID NO. 18); hTERT (SEQ ID NO. 19). The hTERT promoter (295bp) was inserted to replace the TATA-box in front of the E1A region of the adenovirus 3. Arrows indicate the places of PCR primers used in Figure 3b. b) PCR of Ad3-hTERT-E1A. The PCR confirms that the hTERT promoter is on the right place and that the backbone is serotype 3. On the left we can see a band of 270bps and on the right 210bps from both sides of the hTERT promoter insertion area. These fragments have been sequenced and they gave the expected sequence. As negative controls Ad3wt, Ad5/3-hTERT-Δgp and MilliQ water were used. The presence of wild type adenovirus serotype 3 and 5, and Ad5/3 capsid modified viruses was excluded in other PCR experiments. c) Progressive infectivity assay with Ad3-hTERT-E1A. The cells were plated on 96-well plates and the rows infected using 10-fold dilutions from 10⁻⁵ to 10⁻¹² of Ad3-hTERT-E1A, each dilution in ten duplicates. Plates were observed by microscope and virus wells were compared to the mock wells. From the observations pfu/ml was calculated in the similar way as in TCID₅₀ (Adeasy manual, Agilent Technologies, Inc. 2008). The media (DMEM, 5% FBS) were changed every 4 to 7 days. The pfu/ml titer plateaus after 30 days, suggesting slow *in vitro* replication kinetics for Ad3-hTERT-E1A.

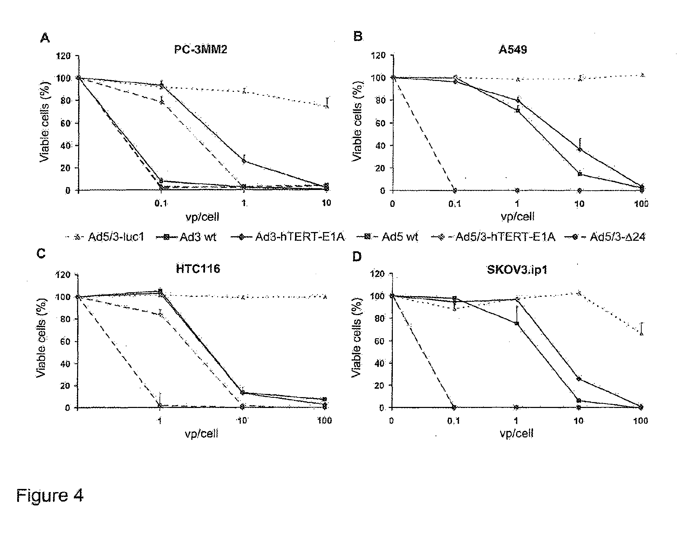

Figures 4A-D show cell killing assays with cancer cell lines. PC3-MM2 (prostate cancer) (A), A549 (lung cancer) (B), HTC116 (colon cancer) (C) and SKOV3.ip1 (ovarian cancer) (D) cells were used. Ad3-hTERT-E1A showed cell killing in all cell lines (P<0.05 versus Ad5/3luc1, the replication deficient control, at 10 VP/cell). Ad300wt, Ad5/3-hTERT-Agp and Ad5/3-Δ24 killed cells faster than Ad3-based viruses. Bars indicate SE.

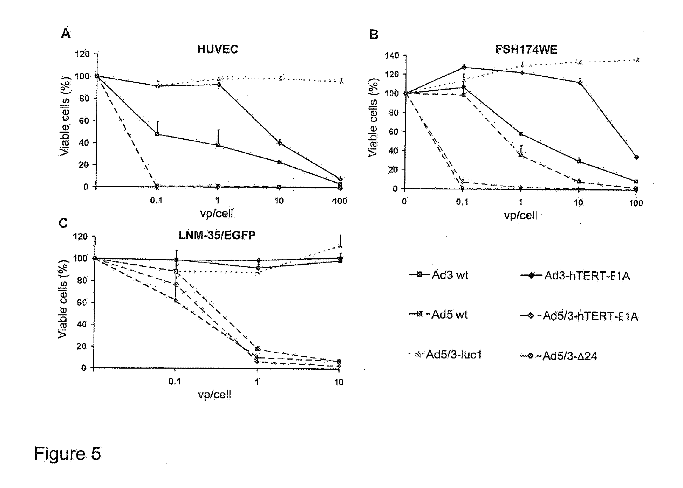

Figures 5A-C show cell killing assays with non-malignant and Ad3 receptor deficient cells. HUVEC (human umbilical vein endothelial cells) (A) and FSH173WE (fibroblasts) (B) were used to represent non-tumor cells. Ad3-hTERT-E1A did not differ from replication deficient Ad5/3-luc1 at low doses and killed significantly less cells than positive controls Ad3wt, Ad300wt, Ad5/3-Δ24 at 0.1, 1 and 10 VP/cell (P<0.05). It was known that LNM-35 (lung cancer) (C) cells lack the Ad3 receptor (Särkioja, M. et al. 2006, Cancer 107: 1578-1588). Ad3wt and Ad3-hTERT-E1A did not show any cytotoxicity on these cells. Bars indicate SE.

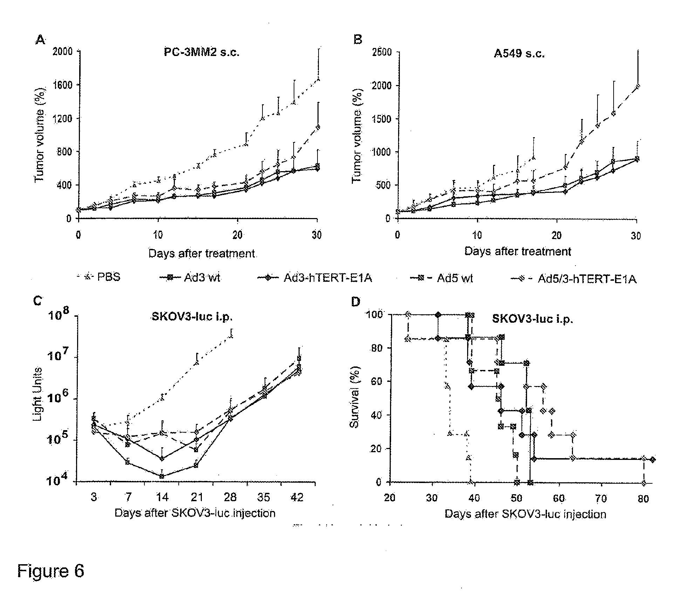

Figure 6 shows the *in vivo* efficacy of Ad3-hTERT-E1A in (A) PC3-MM2 (highly metastatic hormone refractory prostate carcinoma) tumors in nude mice. 10⁹ VP was injected into each tumor weekly for a total of three injections and tumors were measured every 2-3 days. Tumors treated with Ad3-hTERT-E1A grew more slowly than PBS treated tumors (P=0.0035). (B) Similar data were seen in mice with A549 (lung cancer) tumors, although the PBS group had to be terminated early due to large tumor size. On day 17 a difference bordering on significance (P=0.051) was seen between PBS and Ad3-hTERT-E1A. Also, on day 30 Ad3-hTERT-E1A was found significantly (P=0.01) better than Ad5/3-hTERT-E1A suggesting the utility of Ad3-hTERT-E1A in this model. (C) Luciferase expressing SKOV3-luc ovarian cancer cells were grown intraperitoneally in SCID mice. A single intraperitoneal 10⁹ VP virus injection was performed and the number of tumor cells as a function of time was estimated with repeated luciferase imaging of live animals. A significant difference in the luciferase signal was seen between all virus treated groups and the PBS group (P<0.0001), and there were no differences between virus groups. Please note that the Ad300wt virus (wild type Ad5) could only be included in C-D. Bars indicate SE. (D) In survival analysis, all virus treated groups survived longer than the PBS group (P≤0.01). The only long term survivor from the experiment was one out of 7 mice from the Ad3-hTERT-E1A group. It was healthy and tumor free in imaging and autopsy at 100 days, when the experiment was ended.

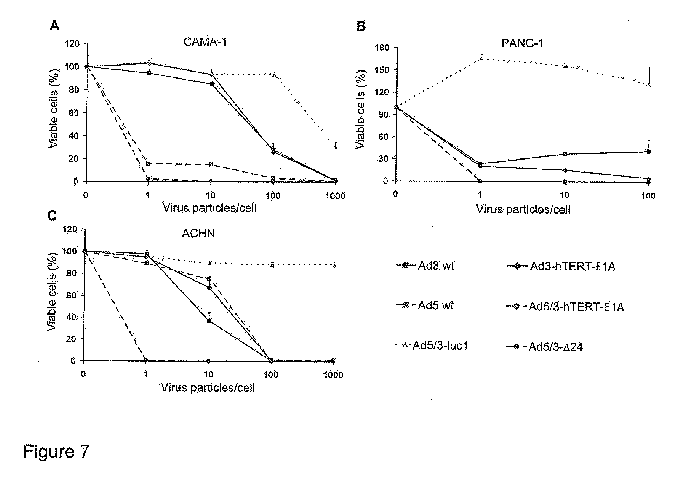

Figures 7A-C show cell killing assays with CAMA-1 (breast cancer) (A), PANC-1 (pancreas cancer) (B) and ACHN (renal cancer) (C) cells. Ad3-hTERT-E1A showed cell killing in all of these cancer types. In all experiments Ad3-hTERT-E1A was significally more efficient than the replication deficient control Ad5/3-luc1 in 100VP/cell (P<0.05).

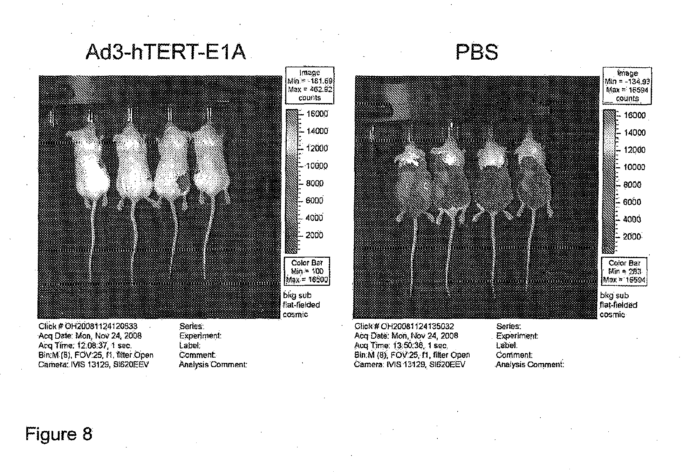

Figure 8 shows in vivo antitumor efficacy of Ad3-hTERT. A bioluminescence picture was taken on day 28.

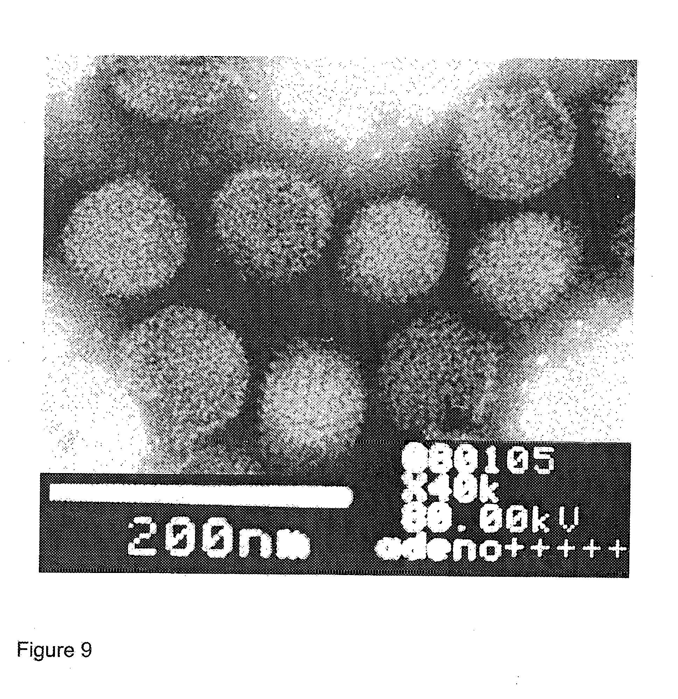

Figure 9 shows an electron microscope picture of the Ad3-hTERT-E1A.

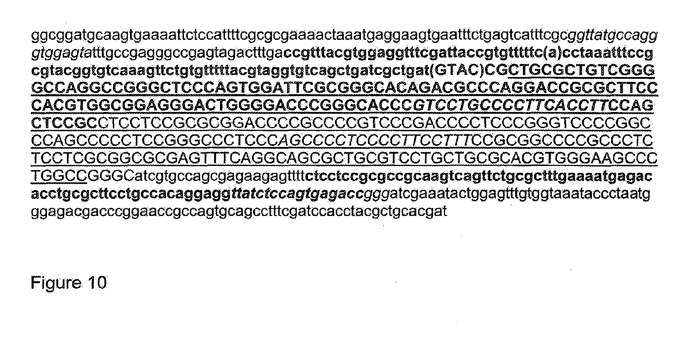

Figure 10 shows partial sequence of Ad3-hTERT-E1. The **sequenced DNA is marked in bold.** Underlined is the sequence of hTERT from pubmed. (Bases missing from the sequenced Ad3-hTERT-E1 compared to Ad3 backbone from pubmed are marked in brackets). BIG LETTERS IS THE hTERT INSERTION. *Primers used are marked in cursive.*

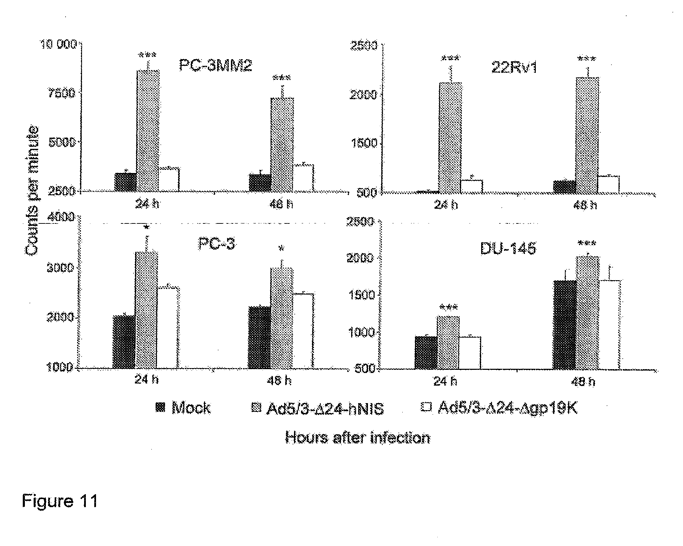

Figure 11 shows Ad5/3-Δ24-hNIS mediated iodide uptake in prostate cancer cells. Cells were infected with 10 VP/cell of Ad5/3-Δ24-hNIS or Ad5/3-Δ24-Δgp19K and exposed to ¹²⁵I 24 and 48 h post infection. The cells were analysed with gamma spectrometer to determine the radioactive iodide content of the cells. Non-infected cells were used as a negative control. Error bars present SEM. * p<0.05; ** p<0.01 and *** p<0.001.

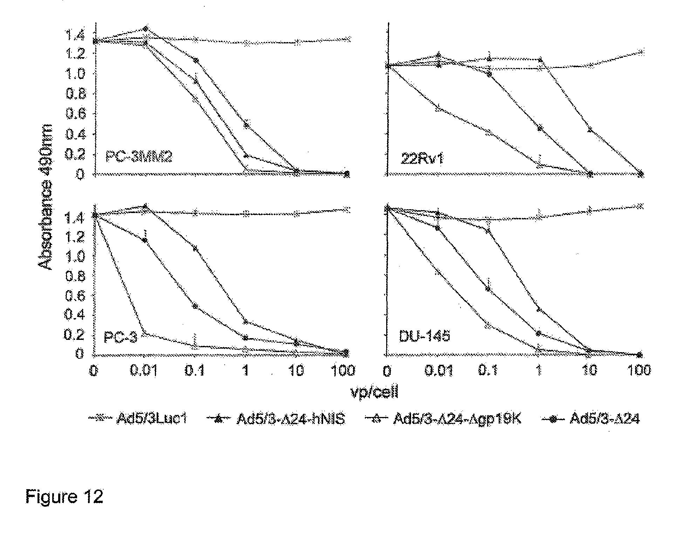

Figure 12 shows Ad5/3-Δ24-hNIS replication and oncolysis in prostate cancer cells without radioiodide. The cells were infected with various viral doses (0-100 VP/cell) and the cell viability was determined 6-9 days later by MTS assay. A replication deficient Ad5/31uc1 and oncolytic Ad5/3-Δ24 were used as controls. Error bars present SEM.

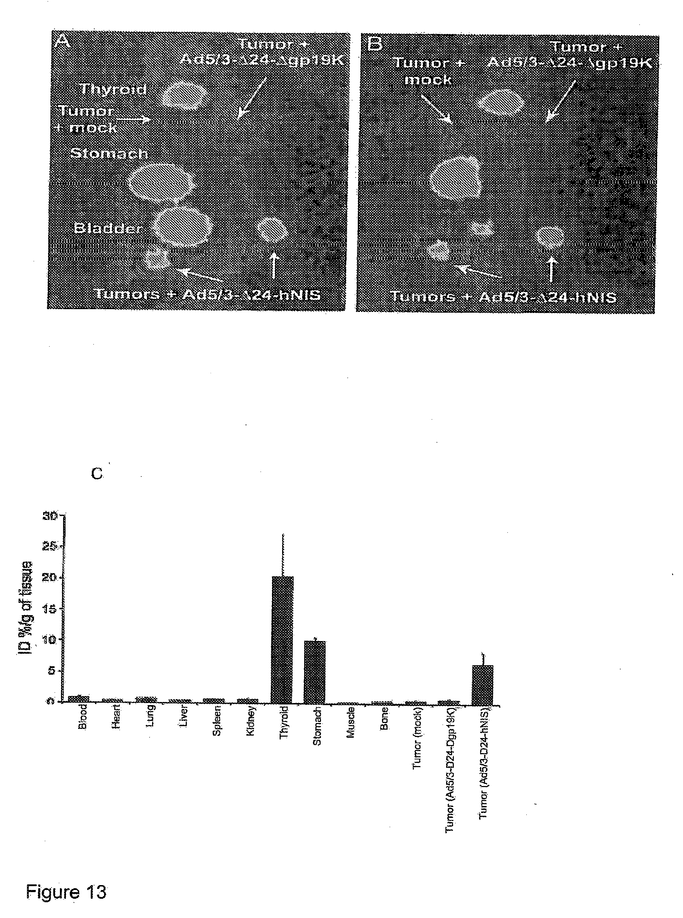

Figure 13 shows Ad5/3-Δ24-hNIS mediated iodide uptake *in vivo.* Mice bearing subcutaneous prostate tumors received Ad5/3-Δ24-hNIS intratumorally (lower flank tumors), Ad5/3-Δ24-Δgp19K (upper right tumor) or saline (upper left tumor) followed by an intravenous ¹²³I injection (1.85 MBq/mouse). The iodide uptake was imaged A) 2 h and B) 13 h after the intravenous ¹²³I-injection with gamma camera. The pixel size was 1.08 mm x 1.08 mm. C) Biodistribution of ¹²³I 13 h after the ¹²³I injection. Bars present SEM, n=3, except n=6 in hNIS tumors.

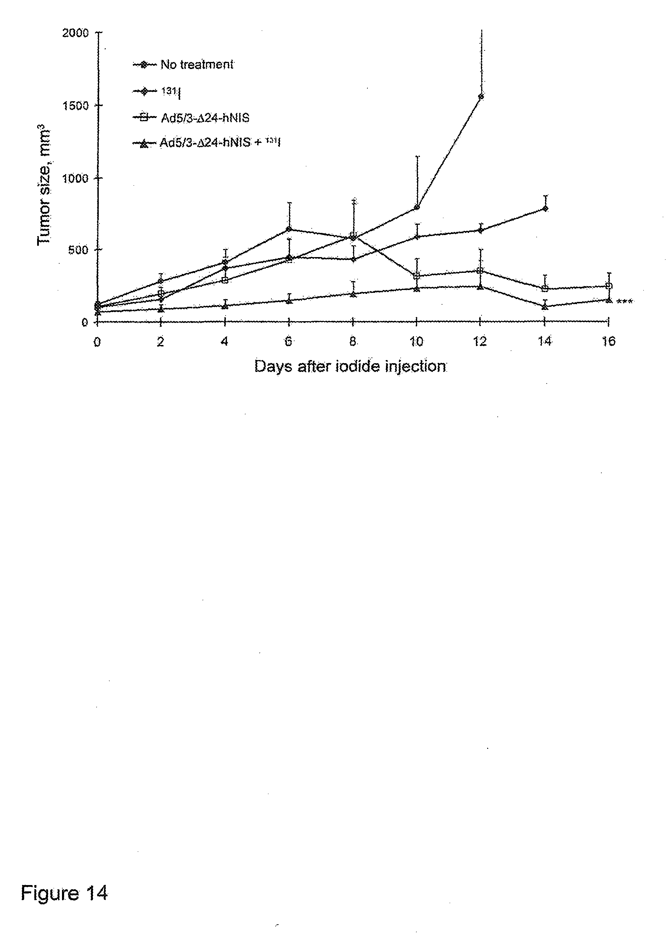

Figure 14 shows Ad5/3-Δ24-hNIS anti-tumor efficacy *in vivo.* Subcutaneous prostate tumor bearing mice (6 mice/group, 12 tumors/group) received Ad5/3-Δ24-hNIS or growth medium intratumorally on two consecutive days followed by a 50 MBq intraperitoneal injection of ¹³¹I or saline. The tumor size was measured every other day. The tumor growth rate was significantly slower in the combination treated group (Ad5/3-Δ24-hNIS + ¹³¹I) when compared to all the other groups (*** p < 0.001). The experiment had to be ended at a predetermined time point of 17 days after the iodide injection due to animal husbandry constraints.

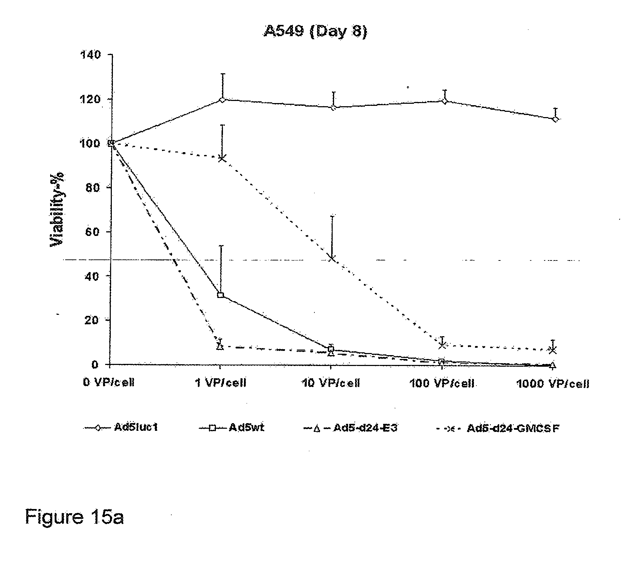

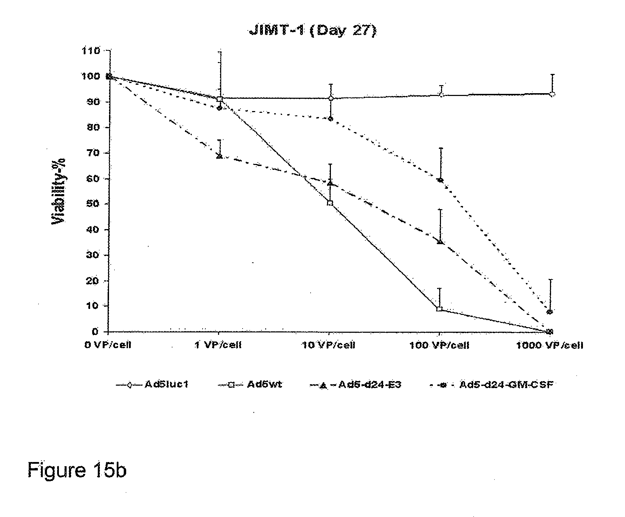

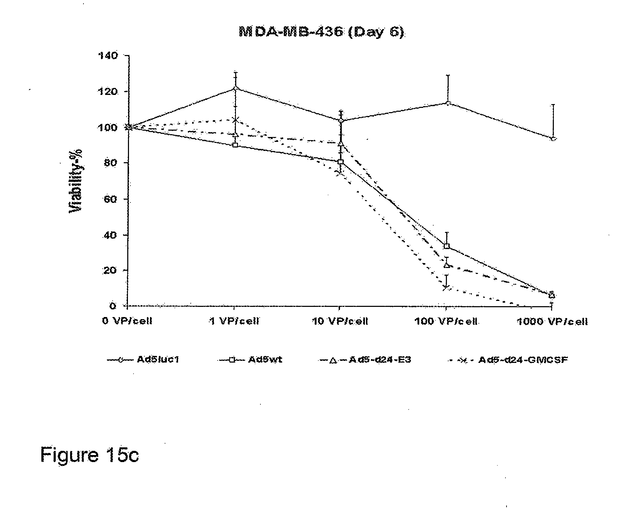

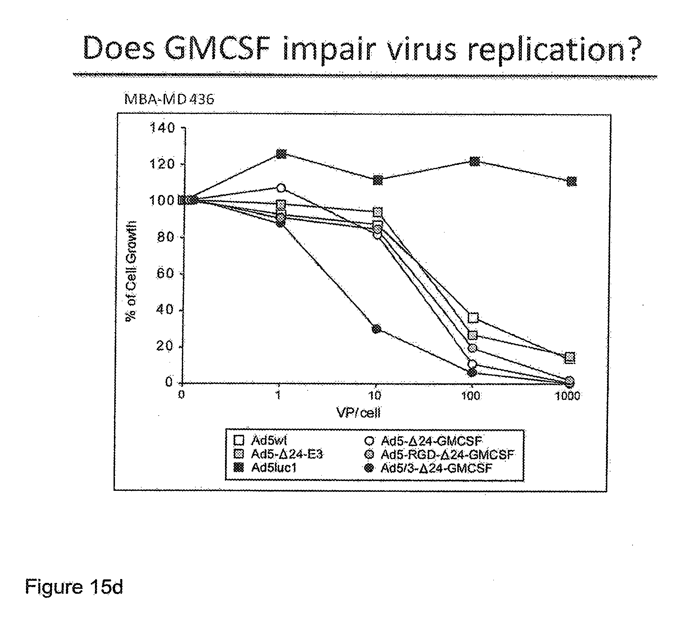

Figures 15a-d show that the GMCSF expression does not impair the virus replication and cell killing effect. Figure 15a represents results of a MTS assay showing the lung cancer derived (A549) cell killing efficiency of the new generated virus Ad5-D24-GMCSF. Figure 15b represents results of a MTS assay showing killing of JIMT-1 cancer initiating cells ("cancer stem cells") with Ad5-D24-GMCSF. Figure 15c represents results of a MTS assay showing the breast cancer cells (MDA-MB-436) killing efficiency with the new generated virus Ad5-D24-GMCSF. Figure 15d represents the results of a MTS assay showing the MDA-MB-436 killing efficiency with the new generated viruses Ad5-D24-GMCSF, Ad5-RGD-D24-GMCSF and Ad5/3-D24-GMCSF.

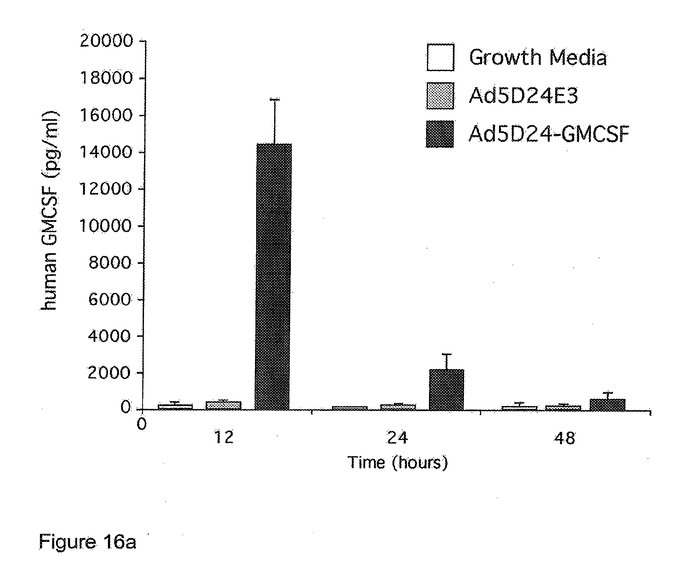

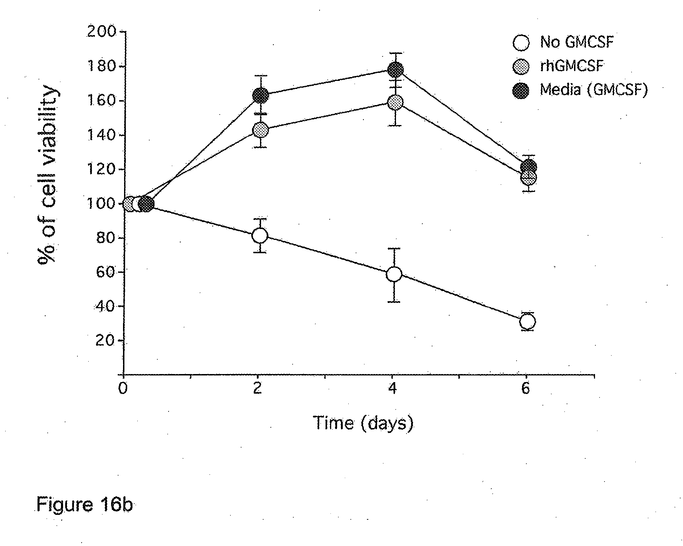

Figure 16a shows the adenovirus-coupled expression of human GMCSF. The A549 cell line was infected with Ad5D24 or Ad5D24-GMCSF, media were collected over time and analyzed for the expression of GMCSF by FACSARRAY. Figure 16b shows that the adenovirus-expressed GMCSF retains its biological activity in human lymphocytes. TF1 cells, which require human GMCSF for staying alive, were cultured in the presence of human recombinant GMCSF (E. coli-produced, purchased from Sigma) or supernatant from Ad5-D24-GMCSF infected cells.

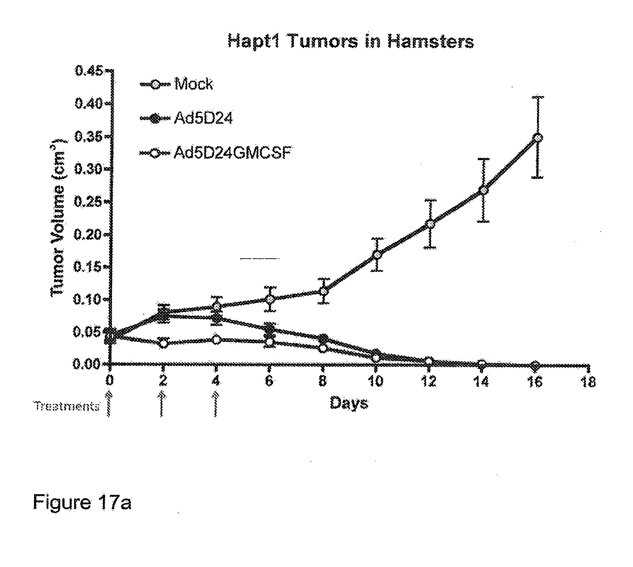

Figure 17a shows the *in vivo* efficiency of Ad5-D24-GMCSF in Syrian Hamsters (permissive for human adenovirus replication) bearing pancreatic cancer tumors. Both Ad5D24 and Ad5-D24-GMCSF eradicate the tumors within 16 days following the treatments. 1 x 10⁹ VP of virus were administered on days 0, 2 and 4.

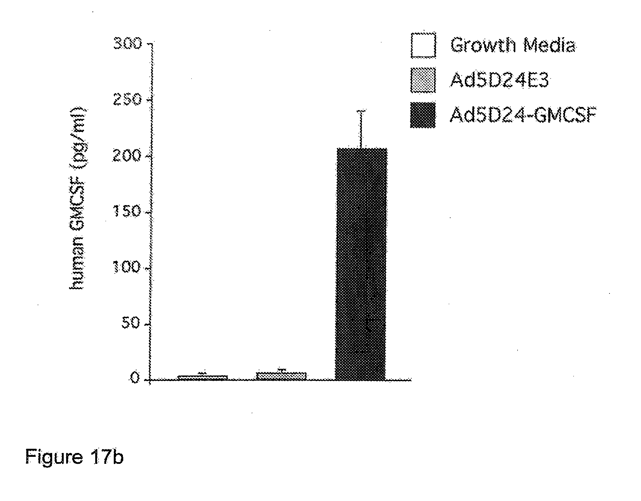

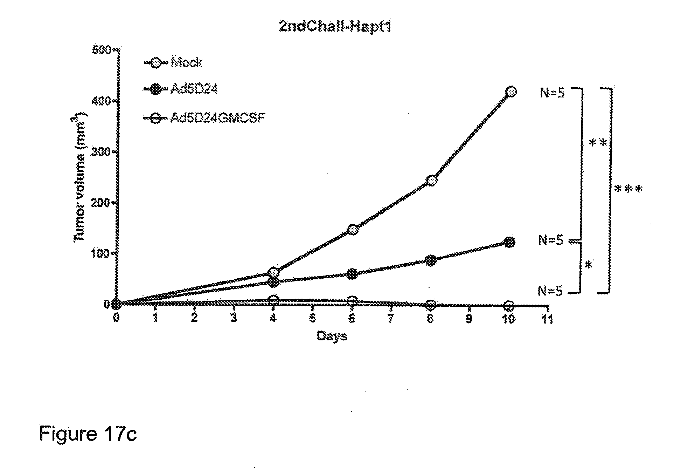

Figure 17b shows that an intratumoral injection of Ad5-D24-GMCSF resulted in high levels of hGMCSF in the serum of Syrian Hamsters. The animals treated with Ad5D24E3 or Ad5-D24-GMCSF were sampled on day 4 and the concentration of human GMCSF in the serum was assessed by FACSARRAY. Figure 17c shows that curing of HapT1 tumors with Ad5-D24-GMCSF (but not Ad5D24) protected Syrian hamsters from a subsequent rechallenge with HapT1. This demonstrates that Ad5-D24-GMCSF can induce a tumor specific immune response. The animals previously treated with Ad5D24 or Ad5D24-GMCSF (Figure 17a) were re-challenged with the same tumor and the tumor growth was measured over time.

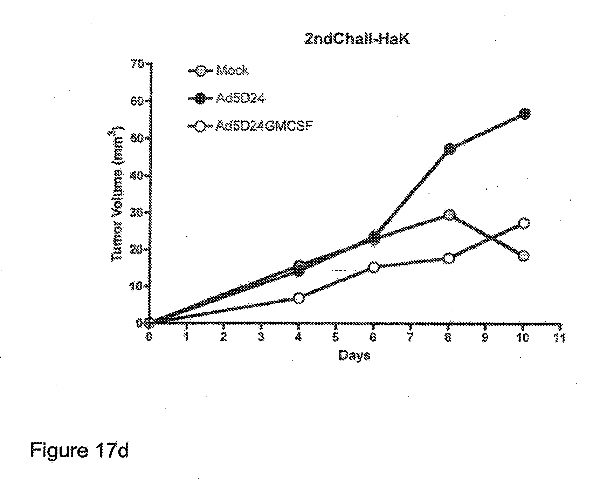

Figure 17d shows induction of a tumor specific immune response by Ad5-D24-GMCSF; curation of HapT1 tumors with Ad5-D24-GMCSF did not protect Syrian hamsters from HaK tumors. The animals with HapT1 tumors previously treated with Ad5D24, or Ad5D24-GMCSF (Figure 17a) were re-challenged with a different tumor and the tumor growth was measured over time.

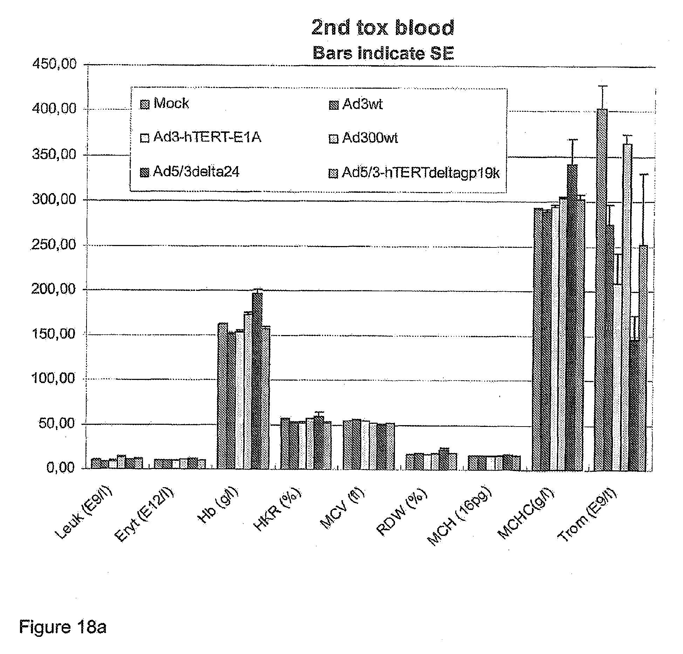

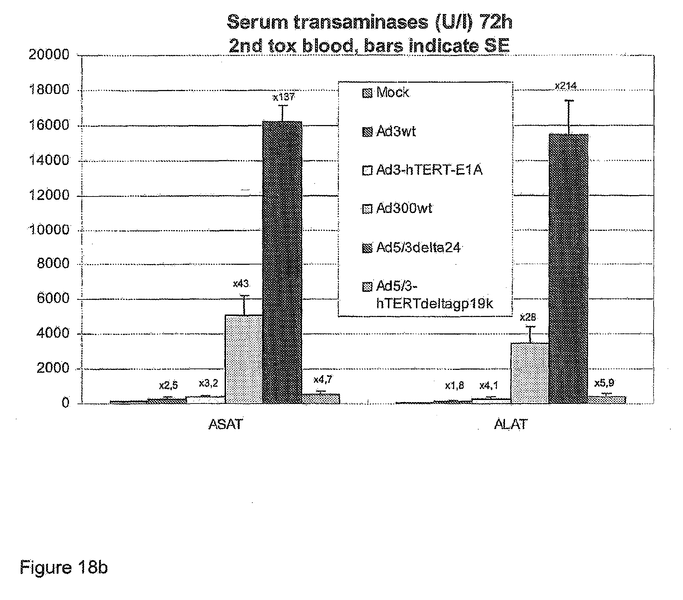

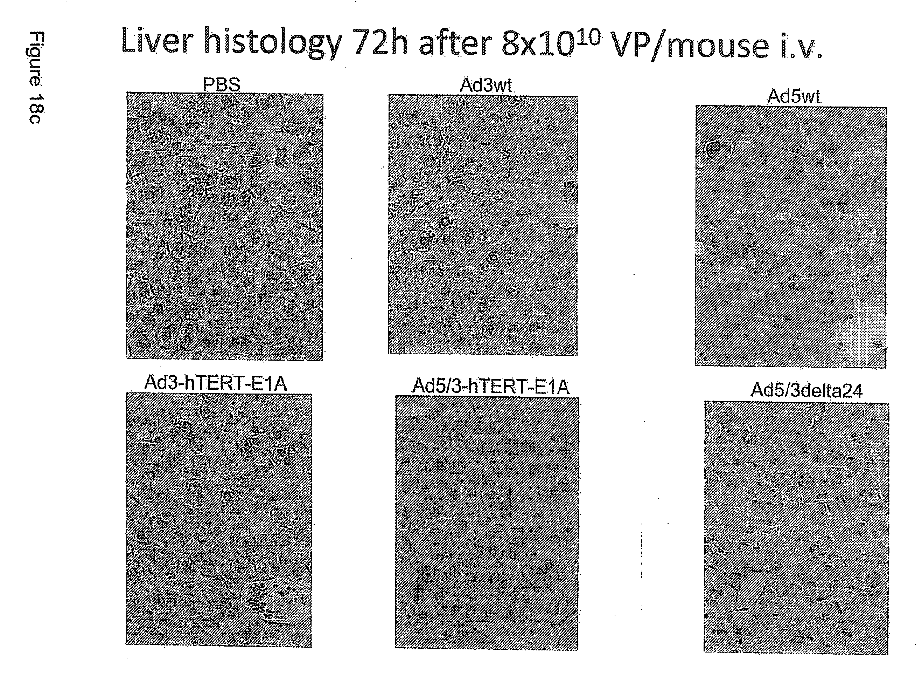

Figures 18a-c show toxicology in mice. In toxicity studies, Ad3-hTERT-E1A was found less toxic than the Ad5 and Ad5/3 control viruses in an immune competent murine model. The histology of all major organs and basic blood values were analyzed. 72h after 8x10¹⁰ VP given intravenously, significant differences were seen in liver histology and in liver enzymes. With serotype 3 only minor liver inflammation close to nonportal veins was seen in some mice. At the same time point and dose serotype 5 and 5/3 groups displayed features of acute liver toxicity and elevated liver enzymes. Other organs and blood values showed no signs of toxicity. Graph of blood samples and liver histology pictures are attached (Fig 18c). Here is the full histopathological report: PBS: 5/5 Normal. No mitoses. No inflammation. Ad3wt: Mostly normal. Mitoses in 5/5 samples. Minor inflammation near (nonportal)veins in the parenchyme 5/5. Ad3-hTERT-E1A: Mostly normal. No mitoses. Borderline inflammation near (nonportal) veins in the parenchyme were seen in 3/5 samples. Ad5wt: 5/5: Many apoptotic hepatocytes throughout the parenchyme, endothelitis and damage in central veins (at the portal area same can not be seen), no pericentral hepatocyte destruction, steatosis, nekrosis, no lymfocytes in the portal area, acute liver damage, damage througout the parenchyme. Ad5/3-hTERT-deltagp19k: 5/5: Almost normal, some point necrosis (not as much as in Ad5wt), some apoptotic cells, mitosis of hepatocytes, (lobular) inflammation in the parenchyme, inflammation (as with Ad3) around the veins. Ad5/3-Δ24: 5/5: Acute fulminant liver failure, 50% of tissue in necrosis, damage stronger near the portal area where all cells are dead, possible fatty generation, some mitoses.

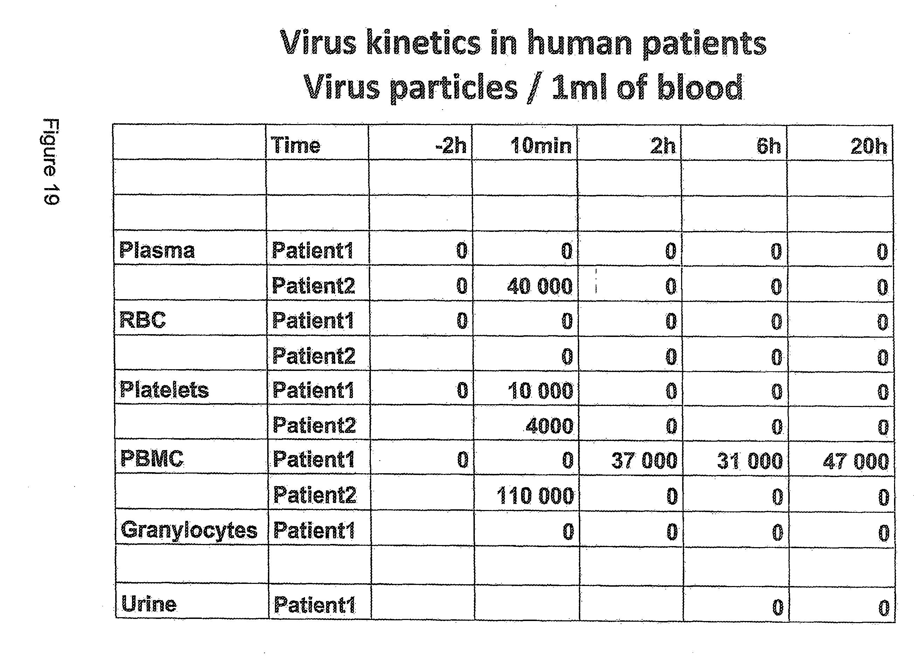

Figure 19 shows virus kinetics in 2 human patients with advanced solid tumors, refractory to standard therapies, which were treated with Ad3-hTERT-E1A. The blood cells were separated using Percoll gradients and the DNA was extracted. The samples were analyzed by qPCR. With patient 1 the virus seems to be cleared from the platelets and peripheral blood mononuclear cells (PBMC) in an hour. With patient 2 much of the virus seems to go to the PBMCs and plasma before rapid clearance from blood. With both patients no virus was detected from the red blood cells (RBC). Results suggest that treatment of cancer patients with Ad3-hTERT-E1A might be safe. Patient 1 gave two virus free urine samples suggesting that the virus is not secreted to the urine.

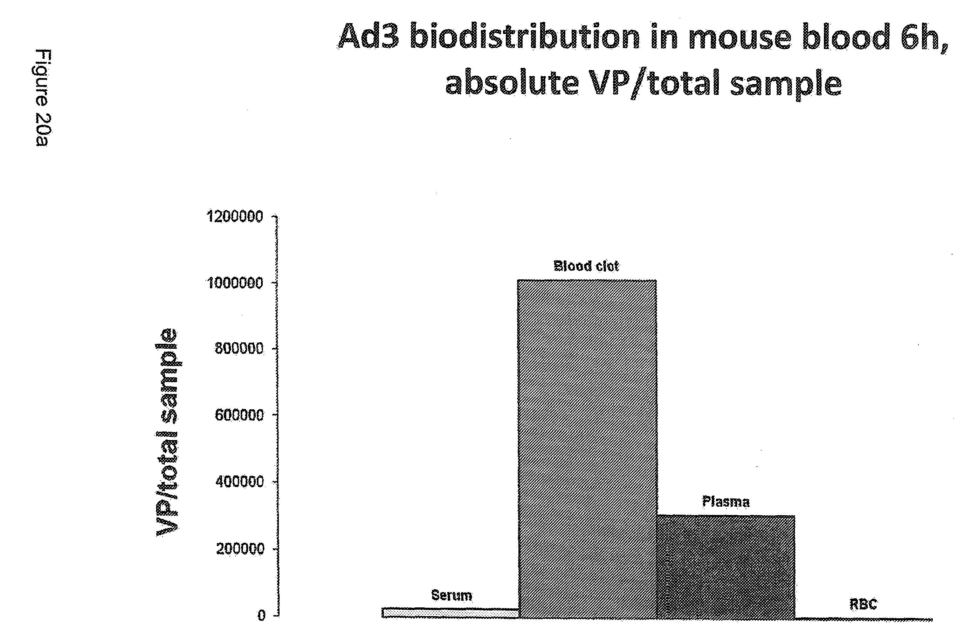

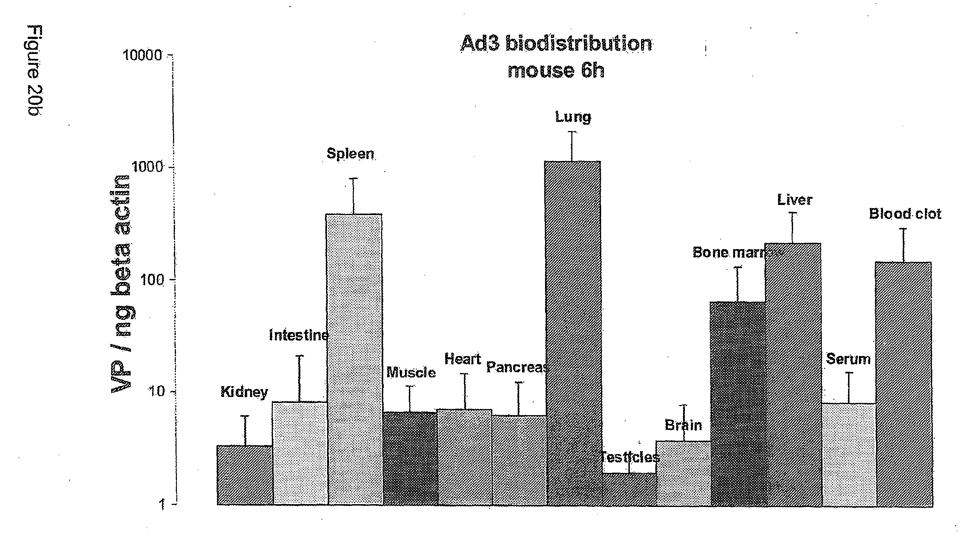

Figures 20a-b show biodistribution in mice. Murine biodistribution at 6h after 5x10¹⁰ VP intravenously suggested that much of the serotype 3 virus stays still in the blood and blood rich organs such as spleen, lung and the liver. All major organs were analyzed by qPCR (Figure 20b). Entry to other organs was found attenuated. In this assay blood clots β-actin was used for the serum values. Due to the lack of β-actin in blood compartments, blood values are best compared with absolute values as done in Fig 20a. The results suggest that Ad3 virus does not associate with RBCs or serum but is abundantly present in blood clots and plasma. Thus WBCs and platelets are possible Ad3 virus carriers in mice.

### Detailed description of the invention

The invention in its broadest sense is as characterized in the independent claims.

### Adenoviral vector

In the present invention, it is shown that an oncolytic human adenovirus vector based on serotype 3 and as recited in the claims can be constructed and it allows tumor cell killing.

In Ad3, as well as in other adenoviruses, an icosahedral capsid consists of at least three major proteins: hexon, penton base, and fiber, along with minor proteins such as VI, VIII, IX, IIIa, and IVa2 (Russell W.C. 2000, J General Virol 81, 2573-2604; Sirena D et al. 2005, Virology 343: 283-298).

Sirena et al. (2005, Virology 343: 283-298) have reported the complete nucleotide sequence of a wild type Ad3 (GeneBank accession no. DQ086466). The Ad3 genome contains early (E1-4), intermediate (IX and IVa2) and the major late unit (MLTU) regions flanked by left and right inverted terminal repeats (LITR and RITR, respectively), which contain the sequences required for the DNA replication.

In one embodiment of the invention, a fully serotype 3 oncolytic human adenoviral vector as recited in the claims comprises one or more regions selected from the group consisting of E1, E2, E3, E4, intermediate and late regions.

In one embodiment of the invention, an oncolytic human adenoviral vector as recited in the claims comprises the following regions: a left ITR, E1, pIX, pIVa2, E2, VA1, VA2, late region, E3 or partial E3, E4, and a right ITR.

The regions may be in any order in the vector, but in one embodiment of the invention, the regions are in a sequential order in the 5' to 3' direction. Open reading frames (ORFs) may be in the same DNA strand or in different DNA strands.

As used herein, the expression "adenovirus serotype 3 (Ad3)" refers to the genome or partial genome of human Ad3, which comprises one or several regions selected from the group consisting of E1, pIX, pIVa2, E2, VA1, VA2, late region, E3 or partial E3, and E4 of Ad3 origin.

As used herein, the expression "partial" region refers to a region, which lacks any part compared to a corresponding wild type region. "Partial E3" refers to the E3 region lacking gp19k.

As used herein, the expressions "VA1" and "VA2" refer to virus associated RNAs 1 and 2, which are transcribed by the adenovirus but are not translated. VA1 and VA2 have a role in combating cellular defence mechanisms.

The insertion of endogenous or exogenous elements may enhance effects of the vectors in target cells. The use of exogenous tissue or tumor-specific promoters is common in recombinant adenoviral vectors and they can also be utilized in the present invention. For example, viral replication can be restricted to target cells for example by promoters, which include but are not limited to hTERT, variants of hTERT, CEA, SLP, Cox-2, Midkine, E2F, variants of E2F, CXCR4, SCCA2 and TTS. In addition to the hTERT promoter control of the E1A region as recited in the claims, other genes, such as E1B or E4, can also be regulated by such a promoter in a vector according to the invention.

In the invention, an oncolytic human adenoviral vector as recited in the claims comprises the Ad3 E1A region under an hTERT promoter. Ad3-hTERT-E1A contains the human telomerase catalytic domain promoter up-stream of the E1A transcription site for tumor specific replication.

In recent decades research on telomeres and telomerases has progressed rapidly. Telomerase activation is a critical step in human carcinogenesis, and most human tumors feature activity of telomerase. This feature is closely linked with activity of hTERT promoter, which has been suggested to be active in most human tumors and therefore represents a useful tumor specific promoter. In one embodiment of the invention, an oncolytic human adenoviral vector as recited in the claims is capable of replicating only in cells with telomerase activity.

In one embodiment of the invention, an oncolytic adenoviral vector as recited in the claims comprises the Ad3 E3 region under a replication activated Ad3 E3 promoter. In one such specific embodiment of the present invention, the transgene is placed into a gp19k deleted E3 region, under the E3 promoter. This restricts the transgene expression to tumor cells that allow replication of the virus and subsequent activation of the E3 promoter. The E3 promoter may be any exogenous or endogenous promoter known in the art, preferably an endogenous promoter.

Exogenous insulators i.e. blocking elements against unspecific enhancers, the left ITR, the native E1A promoter or chromatin proteins may also be included in recombinant adenoviral vectors. Any additional components or modifications may optionally be used but are not obligatory in the vectors of the present invention.

The E3 region is nonessential for viral replication *in vitro,* but the E3 proteins have an important role in the regulation of the host immune response i.e. in the inhibition of both innate and specific immune responses. The vector of the invention may have deletion in the gp19k region in the E3. The gp19k gene product is known to bind and sequester major histocompatibility complex 1 (MHC1) molecules in the endoplasmic reticulum, and to prevent the recognition of infected cells by cytotoxic T-lymphocytes. Since many tumors are deficient in MHC1, deletion of gp19k increases the tumor selectivity of viruses (the virus is cleared faster than the wild type virus from normal cells but there is no difference in tumor cells).

In one embodiment of the invention, an oncolytic adenoviral vector as recited in the claims comprises one or more transgenes. In one such embodiment of the invention, the transgene is placed in E1 under the hTERT promoter and/or in E3 under the replication activated Ad3 E3 promoter. In one embodiment of the invention, an oncolytic human adenoviral vector as recited in the claims comprises the transgene in the place of the deleted gp19k in the E3 region.

In one embodiment of the invention, an oncolytic adenoviral vector as recited in the claims comprises an internal ribosomal entry site (IRES) between the transgene and the E1A region. IRES refers to a nucleotide sequence that enables the initiation of the translation in the middle of a messenger RNA sequence in the protein synthesis. In one such embodiment of the invention IRES is from encephalomyocarditis virus (EMCV), which is obtained from Clontech vector pIRES2-DsRed2. In one such embodiment of the invention, an oncolytic adenoviral vector comprises the hTERT promoter, transgene, IRES and E1A.

In one embodiment of the invention as recited in the claims, the transgene is selected from the group consisting of a granulocyte-macrophage colony-stimulating factor (GM-CSF), human sodium iodide symporter (hNIS), interferon alpha, interferon beta, interferon gamma, tumor necrosis factor alpha, CD40L, trastuzumab and other monoclonal antibodies.

GM-CSF participates in the immune response by acting through various mechanisms including the recruitment of natural killer (NK) cell and the stimulation of antigen presenting cells (APC). APC can then recruit, activate and target T-cells towards the tumor. The nucleotide sequence encoding GM-CSF may be from any animal, such as a human, ape, rat, mouse, hamster, dog or cat, but preferably GM-CSF is encoded by a human sequence. The nucleotide sequence encoding GM-CSF may be modified in order to improve the effects of GM-CSF, or unmodified, i.e. of a wild type. In a preferred embodiment of the invention, the nucleic acid sequence encoding GM-CSF is of a wild type.

In one embodiment of the invention, an oncolytic human adenoviral vector as recited in the claims comprises hNIS (SEQ ID NO. 15), which targets radioiodide to the target cell. The expression of the sodium iodide symporter (hNIS) as a transgene is a strategy for targeting systemically applicable radioisotopes, e.g., radioiodide to tumors. This approach allows tumor cells to be killed due to the oncolytic effect of the virus and due to radiation induced cell death, and the approach also takes advantage of synergy between radiation and oncolytic adenovirus replication. An additional useful aspect of hNIS is non-invasive imaging of transgene expression, which allows monitoring viral spread and persistence.

hNIS is an integral plasma membrane glycoprotein mainly expressed in thyroid follicular cells (Dohan O et al. 2003, Endocr Rev. 24: 48-77). The biological function of hNIS is to mediate active transport of iodine, which is a crucial component for thyroid hormone biosynthesis. This transporting ability of hNIS has been used for half a century in radioiodide therapy of thyroid carcinoma, where radioactive iodide molecules (¹³¹I) are used to internally radiate cancer cells of thyroid origin.

In the present invention, interferon alpha, interferon beta, interferon gamma, tumor necrosis factor, CD40L, trastuzumab and/or other monoclonal antibodies may also be utilized as transgenes in a vector or use as claimed for inducing the immune system of the patient against tumor cells, e.g., by activating natural killer cells, T-cells and/or macrophages.

The vector of the invention as claimed may also comprise other modifications than partial deletions of E3 and insertion of transgenes and/or promoters as mentioned above. Any capsid modification, i.e. modification of hexon, fibre and/or penton base proteins known in the art, which improves delivery of the virus to the tumor cell, may also be utilized in the present invention. As used herein "capsid" refers to the protein shell of the virus, which includes hexon, fiber and penton base proteins. Modifications may be genetic and/or physical modifications and include but are not limited to modifications for incorporating ligands, which recognize specific cellular receptors and/or block native receptor binding, for replacing the fiber or knob domain of an adenoviral vector with a knob of other adenovirus (chimerism) and for adding specific molecules (e.g. FGF2) to adenoviruses. Therefore, capsid modifications include but are not limited to incorporation of small peptide motif(s), peptide(s), chimerism(s) or mutation(s) into the fiber (e.g. into the knob, tail or shaft part), hexon and/or penton base.

Expression cassettes are used for expressing transgenes in a target, such as a cell, by utilizing vectors. As used herein, the term "expression cassette" refers to a DNA vector or a part thereof comprising nucleotide sequences, which encode cDNAs or genes, and nucleotide sequences, which control and/or regulate the expression of said cDNAs or genes. Similar or different expression cassettes may be inserted to one vector or to several different vectors. Ad3 vectors of the present invention as claimed may comprise either one or several expression cassettes. However, only one expression cassette is adequate. In one embodiment of the invention, the oncolytic human adenoviral vector as claimed comprises at least one expression cassette. In another embodiment of the invention, the oncolytic human adenoviral vector as claimed comprises only one expression cassette.

A cell comprising the adenoviral vector of the invention as claimed may be any cell, such as a eukaryotic cell, bacterial cell, animal cell, human cell, mouse cell etc. For example, the cell may be used for producing the adenoviral vector *in vitro* or *in vivo,* or the cell may be a target, such as a tumor cell, which has been infected with the adenoviral vector.

### Cancer

Any cancers or tumors, including both malignant and benign tumors as well as primary tumors and metastasis may be targets of gene therapies. In a specific embodiment of the invention, the cancer is any solid tumor. In one embodiment of the invention, the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

### Pharmaceutical composition

A pharmaceutical composition of the invention comprises at least one type of the vectors of the invention as claimed. Furthermore, the composition may comprise at least two, three or four different vectors of the invention as claimed. In addition to the vector of the invention as claimed, a pharmaceutical composition may a so comprise any other vectors, such as other adenoviral vectors, other therapeutically effective agents, any other agents, such as pharmaceutically acceptable carriers, buffers, excipients, adjuvants, antiseptics, filling, stabilising or thickening agents, and/or any components normally found in corresponding products.

The pharmaceutical composition may be in any form, such as solid, semisolid or liquid form, suitable for administration. A formulation can be selected from a group consisting of, but not limited to, solutions, emulsions, suspensions, tablets, pellets and capsules.

In one embodiment of the invention, the oncolytic adenoviral vector or pharmaceutical composition is provided as an *in situ* cancer vaccine. As used herein an "*in situ* cancer vaccine" refers to a cancer vaccine, which both kills tumor cells and also increases the immune response against tumor cells. Virus replication is a strong danger signal to the immune system (=needed for a TH1 type response), and thus acts as a powerful costimulatory phenomenon to the GM-CSF mediated maturation and activation of APCs, and the recruitment of NK cells. Tumor cell lysis also helps to present tumor fragments and epitopes to APCs and furthermore, costimulation is produced by inflammation. Thus, an epitope independent (i.e. not HLA restricted) response is produced in the context of each tumor and therefore takes place *in situ.* A tumor specific immune response is activated in the target cell as well as the surrounding cells, e.g. in the target tissue.

The effective dose of vectors depends on at least the subject in need of the treatment, the tumor type, the location of the tumor and the stage of the tumor. The dose may vary for example from about 10⁸ viral particles (VP) to about 10¹⁴ VP, preferably from about 5x10⁹ VP to about 10¹³ VP and more preferably from about 8x10⁹ VP to about 10¹² VP.

The pharmaceutical compositions may be produced by any conventional processes known in the art, for example by utilizing any one of the following: batch, fed-batch and perfusion culture modes, column-chromatography purification, CsCl gradient purification and perfusion modes with low-shear cell retention devices.

### Administration

The vector or pharmaceutical composition of the invention may be administered to any eukaryotic subject selected from a group consisting of plants, animals and human beings. In a preferred embodiment the vector for the use according to the invention or the use according to the invention in the manufacture of a medicament is for administration, to a human or an animal. An animal may be selected from a group consisting of pets, domestic animals and production animals.

Most adults have been exposed to the most widely used adenovirus serotype Ad5 and therefore, the immune system can rapidly produce neutralizing antibodies (NAb) against them. In fact, the prevalence of anti-Ad5 NAb may be up to 50%. It has been shown that NAb can be induced against most of the multiple immunogenic proteins of the adenoviral capsid. In one embodiment of the invention, the subject has high amounts of anti-Ad5 neutralizing antibodies. In one embodiment of the invention, the subject has previously been treated with Ad5.

Any conventional method may be used for administration of the vector or composition to a subject. The route of administration depends on the formulation or the form of the composition, the disease, the location of tumors, the patient, comorbidities and other factors. In one embodiment of the invention, the formulation may be for administration conducted through an intratumoral, intramuscular, intra-arterial, intravenous, intrapleural, intravesicular, intracavitary or peritoneal injection, or an oral administration.

Only one administration of oncolytic human adenoviral vectors of the invention may have therapeutic effects. However, in one embodiment of the invention, oncolytic adenoviral vectors or pharmaceutical compositions are formulated for administration several times during the treatment period. Oncolytic human adenoviral vectors or pharmaceutical compositions may be administered for example from 1 to 10 times in the first 2 weeks, 4 weeks, monthly or during the treatment period. In one embodiment of the invention, formulation is for administration three to seven times in the first 2 weeks, then at 4 weeks and then monthly. The length of the treatment period may vary, and may, for example, last from two to 12 months or more.

Nevertheless, in the context of advanced tumor masses, a single round of treatment may not eradicate the tumor. Therefore, re-administration of the virus is likely to be required for increasing efficacy. A key factor limiting systemic re-administration is the neutralizing antibody (NAb) response induced by the virus. In order to avoid neutralizing antibodies in a subject, the vectors of the invention may vary between treatments.

The vector of the invention for use in gene therapy is effective alone, but combination of adenoviral gene therapy with any other therapies, such as traditional therapy, may be more effective than either one alone. For example, each agent of the combination therapy may work independently in the tumor tissue, the adenoviral vectors may sensitize cells to chemotherapy or radiotherapy and/or chemotherapeutic agents may enhance the level of virus replication or effect the receptor status of the target cells. The agents of combination therapy may be administered simultaneously or sequentially.

In one embodiment of the invention, the vector for the use according to the invention or the use according to the invention in the manufacture of a medicament is for administration, with concurrent radiotherapy or radioiodide to a subject. In another embodiment of the invention, the vector for the use according to the invention or the use according to the invention in the manufacture of a medicament is for administration, with concurrent chemotherapy to a subject. As used herein "concurrent" refers to a therapy, which has been administered before, after or simultaneously with the gene therapy relying on the invention. The period for a concurrent therapy may vary from minutes to several weeks. Preferably the concurrent therapy lasts for some hours.

Agents suitable for combination therapy include but are not limited to All-trans retinoic acid, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Mechlorethamine, Mercaptopurine, Methotrexate, Mitoxantrone, Oxaliplatin, Paclitaxel, Pemetrexed, Temozolomide, Teniposide, Tioguanine, Valrubicin, Vinblastine, Vincristine, Vindesine and Vinorelbine.

Methods of producing adenoviral vectors include any conventional methods known in the art. Usually a transgene(s) is inserted into a shuttle plasmid and then homologous recombination is performed between the shuttle plasmid and a plasmid containing the remainder of the viral genome ("rescue plasmid"). The new genome can then be cut from the plasmid and transfected to virus production cells. Alternatively, the rescue plasmid can be co-transfected into producer cells for recombination in those cells.

In one embodiment of the present invention as claimed, a plasmid construct is used for placing a transgene(s) to the adenoviral vector.

In one embodiment of the invention as claimed, the adenoviral vector is produced in a cell line. Usually, eukaryotic cells are transfected with the adenoviral vector and thereafter virus-producing cells are selected, expanded and tested. The viral vectors of the present invention also need extensive purification before administration to animals or human beings.

The present invention is illustrated by the following examples, which are not intended to be limiting in any way.

### Examples

### Cell culture

**293** cells were purchased from Microbix (Toronto, Canada). 293-2v6-11 cells contain a ponasteron-inducible E4orf6 region (Mohammadi E.S. et al. 2004, Nucleic Acids Res 32: 2652-2659). 911-1c11 cells were kept in 1mg/ml G418 (Sirena, D. et al. 2005, Virology 343: 283-298). SKOV3.ip1 ovarian adenocarcinoma cell line was obtained from Dr. Price (M. D. Anderson Cancer Center, Houston, TX). Firefly luciferin-expressing ovarian adenocarcinoma cell line SKOV3-luc was kindly provided by Dr. Negrin (Stanford Medical School, Stanford, CA). PC-3MM2 highly metastatic hormone refractory subline of prostate carcinoma was a kind gift of Isaiah J. Findler (MD Anderson Cancer Center, Houston, TX). LNM35/EGFP, a highly lymphogenous metastatic subline of a human large cell carcinoma of the lung, was provided by Takashi Takahashi (The Honda Research Institute, Japan). The human umbilical vein endothelial cells known as the HUVEC were bought from Lonza (Basel, Switzerland). The following cell lines were obtained from ATCC (Manassas, VA): ACHN kidney cell carcinoma, A549 lung adenocarcinoma, FHS173WE human fibroblast cell line, HTC116 colorectal carcinoma, CAMA-1 breast adenocarcinoma, PANC-1 pancreatic epithelioid carcinoma. All cell lines were cultured under recommended conditions.

### Construction of Ad3-hTERT-E1

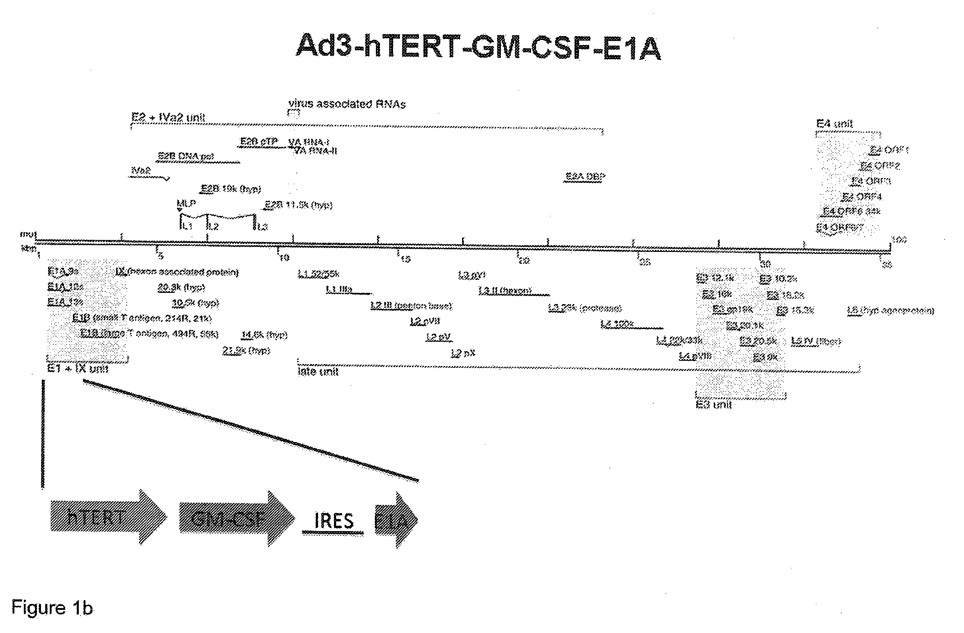

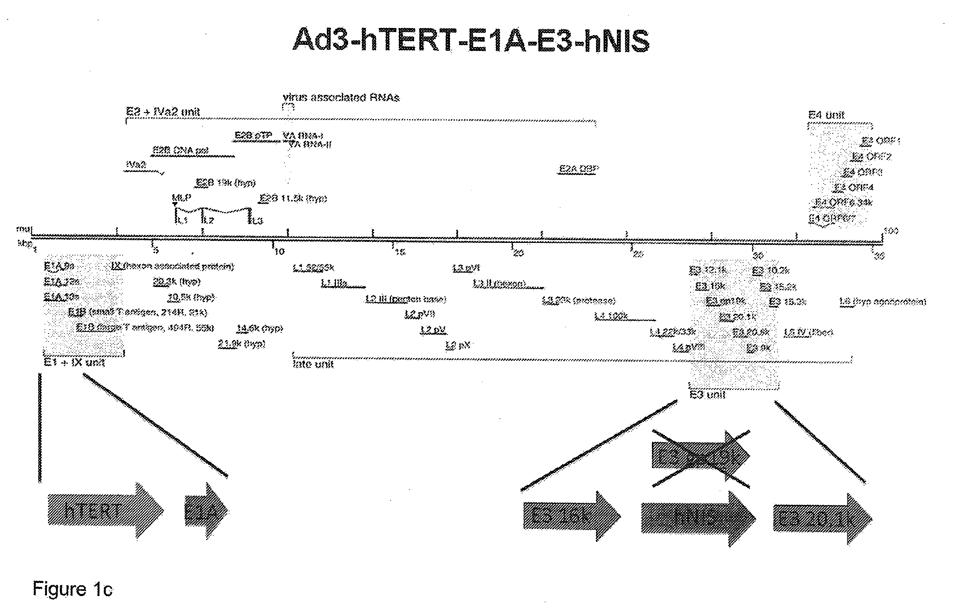

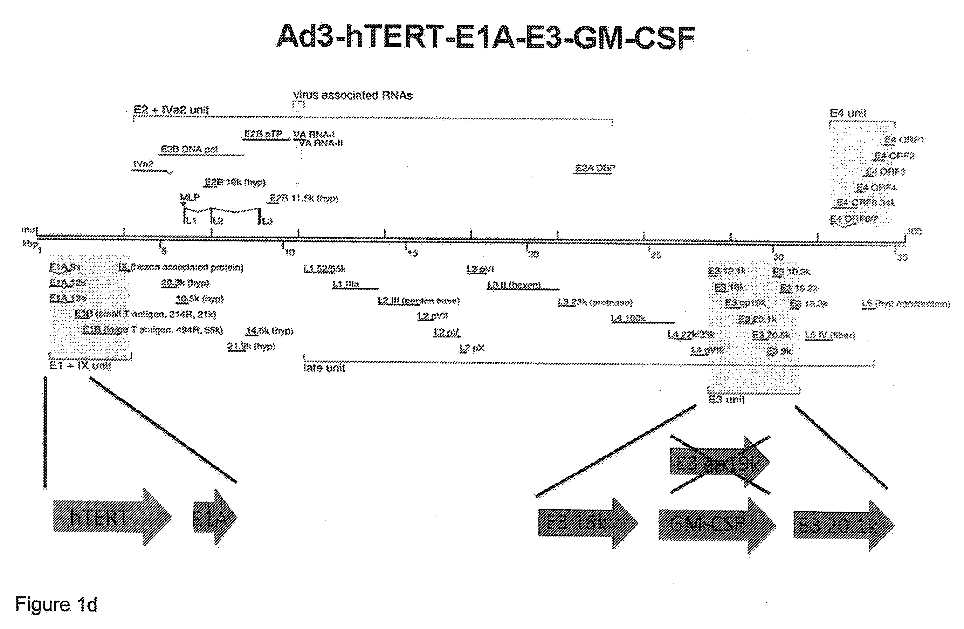

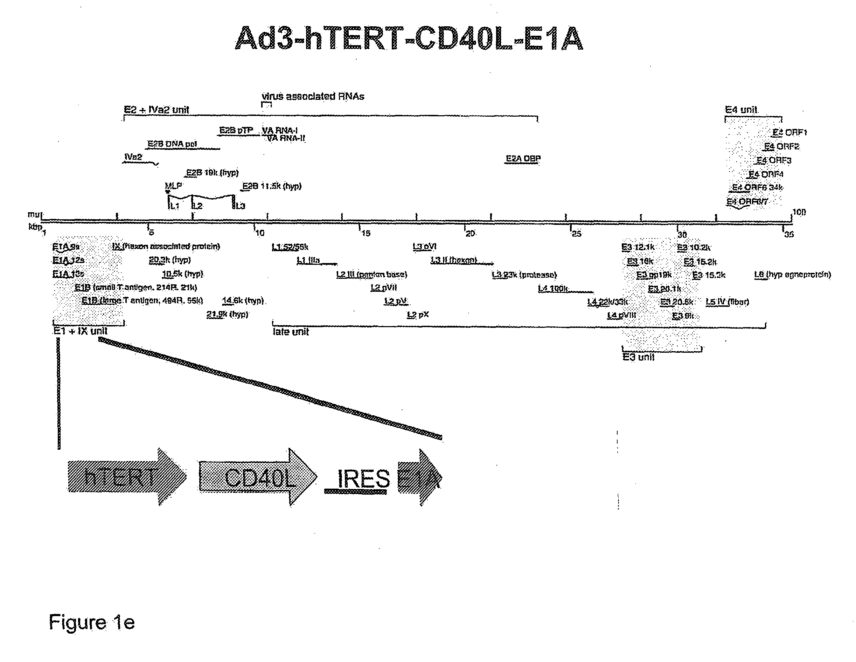

For cloning of Ad3-hTERT-E1 three different PCR were performed on Ad3-wt plasmid pKSB2Ad3wt (Sirena, D. et al. 2005, Virology 343: 283-298), two of them to exclude the original TATA-box from the Ad3wt sequence (Figures 1-3). The first PCR product included LITR with a Mlul digestion site until the TATA-box (forward primer: 5'-CAT GGT ACC CAA GTG TGT CGC TGT CGA GT-3' (SEQ ID NO: 1), reverse primer 5'-CAT GAT ATC AGC GAT CAG CTG ACA CCT AC-3' (SEQ ID NO: 2); adding Kpnl site in front and EcoRV site at the end), the second started directly after the TATA-box until the first half of the E1A region (forward primer: 5'-CAT GAT ATC GTG CCA GCG AGA AGA GTT TT-3' (SEQ ID NO: 3), reverse primer 5'-CAT TCT AGA GCG AGC ACA ATA GTT CTT TCA-3' (SEQ ID NO: 4); adding EcoRV site at front and Xbal at the end). The third PCR amplified 1600 bp of the end of adenovirus type 3 including RITR. The respectively digested first two PCR products around the TATA-box were cloned into Kpnl/Xbal digested pUC19 cloning vector resulting in pGBAd3E1, including a TATA-box excluded start of the Ad3 sequence. The hTERT promoter derived from pBT255 was digested with KpnI/XhoI, blunted, and cloned in front of the E1 region by a EcoRV digestion of pGBAd3E1 resulting in pGBAd-3hTERT-E1.

The third PCR product with the end of the Ad3 sequence was now cloned into NotI/blunted pShuttle, followed by digestion with HindIII, and subsequently cloned into HindIII digested pGBAd3-hTERT-E1 resulting in pSGBAd3-hTERT-E1 as shuttle vector with end and modified start of the adenovirus 3 genome. Finally pSGBAd3-hTERT-E1 was digested with XhoI/MscI, resulting in the plasmid being opened up in between the modified start and preserved end of the adenovirus 3 sequence, and homologous recombined with Mlul digested and thus backbone-free pKSB2Ad3wt, to reduce background due to same resistance gene and to achieve a plasmid with Ad3-hTERT-E1 sequence (pKGB-Ad3-hTERT-E1).

The recombinant Ad3-hTERT-E1A virus was rescued by transfecting 911-1c11 cells with Mlul-digested pKGB-Ad3-hTERT-E1. Subsequent amplification of a first large Ad3-hTERT-E1A virus stock was done in 293-2v6-11 cells, followed by 2^{nd} amplification in A549 cells. After amplification the viruses were purified on double cesium chloride gradients. The stocks were confirmed not to have serotype 5 contamination by PCR (for the standard method, see Kanerva, A. et al. 2003, Mol Ther 8: 449-458). Then the presence of the hTERT promoter in front of the E1A was confirmed (Fig. 3b). The primers used to get the 270bp band were 5'-GGT TAT GCC AGG GTG GAG TA-3' forward (SEQ ID NO: 5) and 5'-AAG GTG AAG GGG CAG GAC-3' reverse (SEQ ID NO: 6). For the 210bp band, the primers used were 5'-AGC CCC TCC CCT TCC TTT-3' forward (SEQ ID NO: 7) and 5'-CCC GGT CTC ACT GGA GAT AA-3' reverse (SEQ ID NO: 8). The PCR bands were then sequenced and the expected sequence was obtained (see SEQ ID NO: 9 and Figure 10).

Finally the Ad3-hTERT-E1A was observed by electron microscope (see Figure 9).

### Other adenoviruses

Please see Table 1 for adenoviruses relevant to this invention.

**Table 1. Adenoviruses**

| **Virus name** | **Back-bone sero-type** | **Fiber knob sero-type** | **E1A** | **Ratio (VP/pfu)** | **Reference** |
|---|---|---|---|---|---|
| **Ad5 wt** | 5 | 5 | Wild type | 19 | ATCC (Ad300 wt strain) |
| **Ad5/3-hTERT-E1A** | 5 | 3 | Controlled by hTERT promoter (mediates selectivity to telomerase active cells) | 10 | Bauerschmitz, G.J. et al. 2008, Cancer Res 68: 5533-5539 (Named here Ad5/3-hTERT-Δgp) |
| **Ad5/3-Δ24** | 5 | 3 | 24bp deletion (mediates selctivity to p16/Rb pathway mutant tumor cells) | 22 | Kanerva, A. et al. 2003, Mol Ther 8: 449-458. |
| **Ad5/3Iuc1** | 5 | 3 | Deleted (makes virus replication deficient) | 5 | Kanerva, A. et al. 2002, Clin Cancer Res 8: 275-280. |
| **Ad3 wt** | 3 | 3 | Wild type | 9 | Sirena, D. et al. 2005, Virology 343: 283-298. |
| **Ad3-hTERT-E1A** | 3 | 3 | Controlled by hTERT promoter (mediates selectivity to telomerase active cells) | 9 | This application |

### Progressive infectivity assay

The methodology established for Ad5 based viruses had to be optimized to account for the slower replication of Ad3 wild type and Ad3-hTERT-E1A. To estimate a functional titer, the usual 10-day cytopathic effect assay was not sufficient and therefore we developed a more dynamic progressive infectivity assay (Fig. 3c), where the cytopathic effect was allowed to develop until the titer plateaued. This represents the actual functional titer of the virus.

The cells were plated on 96-well plates, 10 000 cells/well and rows were infected the next day with decreasing 10-fold dilutions from 10⁻⁵ to 10⁻¹² of Ad3-hTERT-E1A, each dilution in ten duplicates. The infection was done in DMEM, 2% FBS. The plates were observed by microscope and the virus wells were compared to the mock wells. From the observations pfu/ml was calculated in the similar way as in TCID₅₀ (Adeasy manual, Agilent Technologies, Inc. 2008). The media (DMEM, 5% FBS) was changed every 4 to 7 days.

*In vitro*, the virus was slower in reaching the maximum titer and the functional infectivity assay required 35 days.

### In vitro cytotoxicity assays

The cells were plated at 10 000 cells/well on 96 well plates. The next day, the cells in triplicates or quadruplicates were infected with the viruses at 0.1 to 100 VP/well. The infection was done in 2% fetal bovine serum (FBS). The plates were regularly observed and the growth media was changed every 3-5 days. Six to twenty days later, as optimal for each line, the cell viability was analyzed with MTS assay (Cell Titer 96 AQueous One Solution Cell Proliferation Assay, Promega). MTS analyzes were performed on the following days: PC-3MM2 on day 8, A549 on day 17, HTC116 on day 6, SKOV3.ip1 on day 18, HUVEC on day 11, FSH174WE on day 13, LNM-35/EGFP on day 10, CAMA-1 on day 11, PANC-1 on day 20 and ACHN on day 11. The results are shown in Figures 4A-D.

Although both fully Ad3 viruses eventually killed all cancer cells in a cytotoxicity assay, they seemed slower than Ad5 based oncolytic agents, including a 5/3 serotype chimera. In non-cancer cells, Ad3-hTERT-E1A was less toxic than the control viruses, including wild type Ad3. Ad3-hTERT-E1A was not able to kill cells that lack the Ad3 receptor.

### Oncolytic potency of Ad3-hTERT-E1A on cancer cell lines in vitro

The ability of Ad3-hTERT-E1A to kill cancer cells *in vitro* was analyzed by infecting monolayers representing seven different tumor types (Figures 4A-D and Figures 7A-C). In all malignant cell lines Ad3-hTERT-E1A showed complete oncolysis, while the non-replicative Ad5/31ucl virus showed no cell killing (p<0.05). However, as anticipated based on experience from growing and titering the virus, Ad3-hTERT-E1A was somewhat slower than the serotype 5 viruses.

### Tumor selectivity of Ad3-hTERT-E1A

For analyzing the tumor selectivity, we infected HUVEC (human umbilical vein endothelial cells) and FSH173WE (fibroblasts) with the same viruses and performed cell killing assays (Fig. 5). No cell killing was seen at low virus concentrations and at higher doses Ad3-hTERT-E1A was less toxic than the control viruses Ad3wt, Ad5wt, Ad5/3-hTERT-E1A, Ad5/3-Δ24 (0.1, 1 and 10 VP/cell P<0.05). From the previous work with Ad5/3 chimeras, we knew that LNM-35/EGFP cannot be infected with Ad5/3 and thus may lack the Ad3 receptor (Särkioja, M. et al. 2006, Cancer 107: 1578-1588). Fittingly, no cell killing was seen with Ad3-hTERT-E1A or Ad3 wild type.

### Animal experiments

All animal experiments were approved by the Experimental Animal Committee of the University of Helsinki and the Provincial Government of Southern Finland. Mice where frequently monitored for their health status and euthanized as soon as signs for pain or distress was noticed. For the subcutaneus model female NMRI nude mice were used (Charles River, Germany). They were ordered at the age of 3-4 weeks and quarantined for 2 weeks. For the intraperitoneal model female fox chase SCID mice (Charles River, Germany) were used. They were ordered at the age of 6-7 weeks and quarantined for 4 months.

### Oncolytic potency in vivo

We tested the efficacy of Ad3-hTERT-E1A in mice bearing PC-3MM2 prostate cancer xenografts (Fig. 6A). Ad3-hTERT-E1A was able to reduce tumor growth significantly in comparison to PBS injections (P=0.0035). Interestingly, even though the virus was slower than positive controls *in vitro*, there was no difference in efficacy *in vivo*. Ad3-hTERT-E1A was at least as oncolytic as Ad5 and Ad5/3 based controls *in vivo*. In mice with A549 xenografts (Figure 6B), the Ad3-hTERT-E1A group had the smallest tumors from day 17 onwards, but because of rapid tumor growth in the PBS group, and termination of that group at day 17, the experiment lost much statistical power. At day 17 a borderline difference (P=0.051) could be seen comparing Ad3-hTERT-E1A and the PBS. At day 30 Ad3-hTERT-E1A had reduced tumor growth significantly (P=0.01) better than Ad5/3-hTERT-E1A, which is known to be a highly potent oncolytic adenovirus (Bauerschmitz, G. J. et al. 2008, Cancer Res 68: 5533-5539).

Subcutaneous tumors may not be optimal surrogates of human cancers and therefore we proceeded to utilize an orthotopic model featuring intraperitoneally disseminated carcinomatosis induced with luciferase expressing SKOV3Luc cells (Figures 6C, D). Ad3-hTERT-E1A significantly reduced the luciferase signal as compared to PBS treated mice (p<0.0001). There was no difference in efficacy between the different replication competent viruses. Ad3-hTERT-E1A extended the median survival of mice from 34 to 46 days (PBS *versus* Ad3-hTERT-E1A), which resulted in a significant difference in Kaplan-Meier survival analysis (p=0.01). One out of the 7 mice treated with Ad3-hTERT-E1A survived until the end of the experiment (day 120) and may have been cured as no evidence of tumor could be detected in luciferase imaging or autopsy and its behavior was normal.

### Subcutaneous animal experiment

Mice where injected subcutaneously to both flanks with 2 x 10⁶ PC-3MM2 or 5 x 10⁶A549 cells. After 13 and 11 days, respectively, the tumors had developed to measurable sizes and the mice were treated intratumoral by 10⁹VP in PBS or only PBS. Injection volume per tumor was 50 µl. The injection was repeated after one and two weeks. The tumors were measured frequently and the volume was calculated *V* = *L x H² x 0.52.* The measured volume before the first treatment was regarded as 100% and the tumor growth was compared to this. With the A549 group some of the tumors treated with PBS grew aggressively and the mice had to be euthanized after two and a half weeks.

### Intraperitoneal animal experiment

An orthotopic model of peritoneally disseminated ovarian cancer was developed by injecting 5 x 10⁶ SKOV3-luc cells intraperitoneally in 300 µl of pure DMEM into SCID mice. After three days the mice (n=7) were imagined noninvasively and treated intraperitoneally by injecting PBS or 10⁹VP in PBS per mouse. The mice were imaged on day 3, 7, 14, 21, 28, 35 and 42 using IVIS 100 (Xenogen, Alameda, CA, USA) to estimate the number of tumor cells is the mice (Fig. 8). For bioluminescence imaging, 150 mg/kg D-luciferin (Promega, Madison, WI, USA) was injected intraperitoneal and captured 10min later with 10s expousure time, If/stop, medium binning and open filter. During imaging the mice were in isoflurane gas anesthesia. The images were overlaid with Living Image 2.50 (Xenogen). Total flux (photons/s) was measured by drawing regions of interest (ROI) around the peritoneal area of the mice. Background was subtracted.

### Toxicology in mice.

In toxicity studies (Figures 18a-c), Ad3-hTERT-E1A was found less toxic than the Ad5 and Ad5/3 control viruses in an immune competent murine model. The histology of all major organs and basic blood values were analyzed. 72h after 8x10¹⁰ VP given intravenously, significant differences were seen in liver histology and in liver enzymes. With serotype 3 only minor liver inflammation close to nonportal veins was seen in some mice. At the same time point and dose serotype 5 and 5/3 groups displayed features of acute liver toxicity and elevated liver enzymes. Other organs and blood values showed no signs of toxicity. Graph of blood samples and liver histology pictures are attached (Fig 18c). Here is the full histopathological report: PBS: 5/5 Normal. No mitoses. No inflammation. Ad3wt: Mostly normal. Mitoses in 5/5 samples. Minor inflammation near (nonportal)veins in the parenchyme 5/5. Ad3-hTERT-E1A: Mostly normal. No mitoses. Borderline inflammation near (nonportal) veins in the parenchyme were seen in 3/5 samples. Ad5wt: 5/5: Many apoptotic hepatocytes throughout the parenchyme, endothelitis and damage in central veins (at the portal area same can not be seen), no pericentral hepatocyte destruction, steatosis, nekrosis, no lymfocytes in the portal area, acute liver damage, damage througout the parenchyme. Ad5/3-hTERT-deltagp19k: 5/5: Almost normal, some point necrosis (not as much as in Ad5wt), some apoptotic cells, mitosis of hepatocytes, (lobular) inflammation in the parenchyme, inflammation (as with Ad3) around the veins. Ad5/3-Δ24: 5/5: Acute fulminant liver failure, 50% of tissue in necrosis, damage stronger near the portal area where all cells are dead, possible fatty generation, some mitoses.

### Virus kinetics in human patients.

So far 8 patients with advanced solid tumors, refractory to standard therapies, have been treated with Ad3-hTERT-E1A. The safety has been good with doses of 5x10¹⁰ up to 5x10¹¹ virus particles and dose escalation continues. Overall, all patients have experienced grade 1-2 adverse events, typically fever, nausea and tiredness. No grade 3 or higher adverse events have been seen so far. Complete analyses, including efficacy and immunological data are being processed.

From the first two patients blood samples were taken, as shown in Figure 19. The blood cells were separated using Percoll gradients and the DNA was extracted. The samples were analyzed by qPCR. With patient 1 the virus seems to be cleared from the platelets and peripheral blood mononuclear cells (PBMC) in an hour. With patient 2 much of the virus seems to go to the PBMCs and plasma before rapid clearance from blood. With both patients no virus was detected from the red blood cells (RBC). Results suggest that treatment of cancer patients with Ad3-hTERT-E1A might be safe. Patient 1 gave two virus free urine samples suggesting that the virus is not secreted to the urine.

### Statistical analysis

Nonparametric Man-Whitney test (SPSS 15.0 for Windows) was used to compare two independent samples for all the *in vitro* and some of the *in vivo* data. The analysis of tumor size was performed using a repeated measures model with PROC MIXED (SAS Ver. 9.1). Models were run with the tumor size measurements in the natural metric and log transformed. The effects of the treatment group, time in days and the interaction of the treatment group and time were evaluated by F tests. Curvature in the models was tested for by a quadratic term for time. The a priori planned comparisons of specific differences in predicted treatment means averaged over time and at the last timepoint were computed by t-statistics. For the A549 experiment we compared the groups at 17 and 30 days and the PC-3MM2 at 30 days and time-averaged. Survival was evaluated using the Kaplan-Meier survival plot with log rank regression (SPSS 15.0 for Windows). For all analyses a two-sided P value of <0.05 was deemed statistically significant.

### Viral constructs comprising HNIS and/or GMCSF and/or CD40L

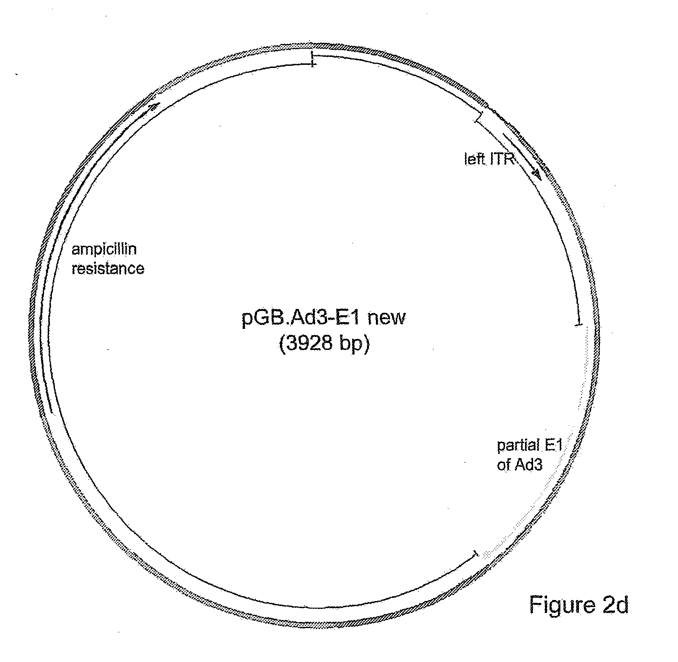

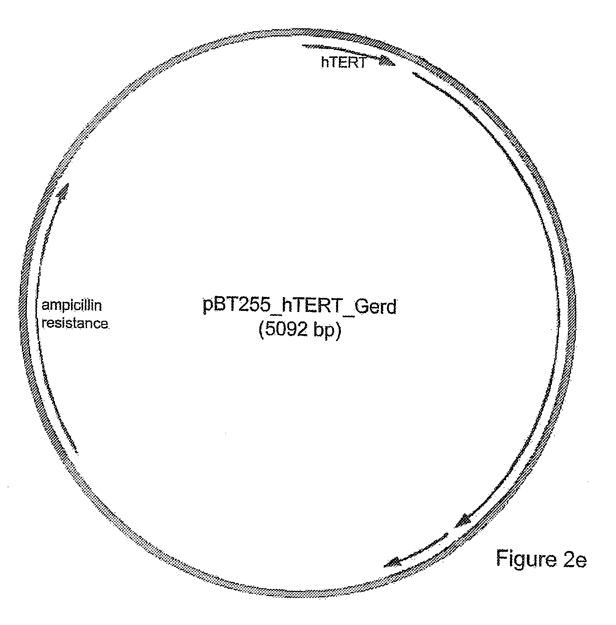

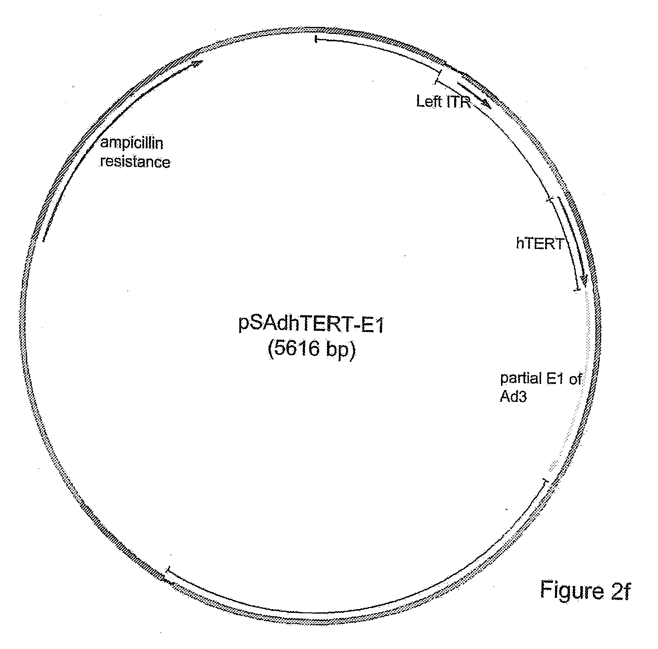

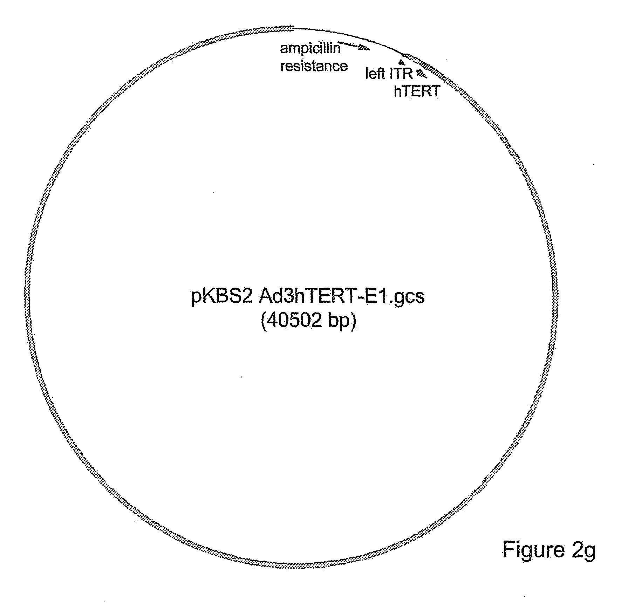

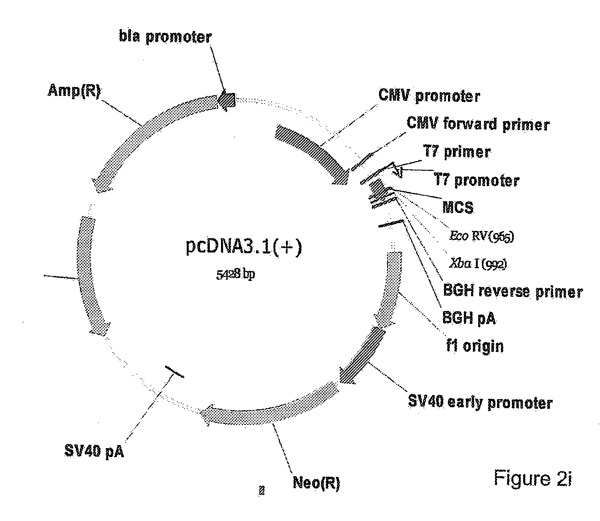

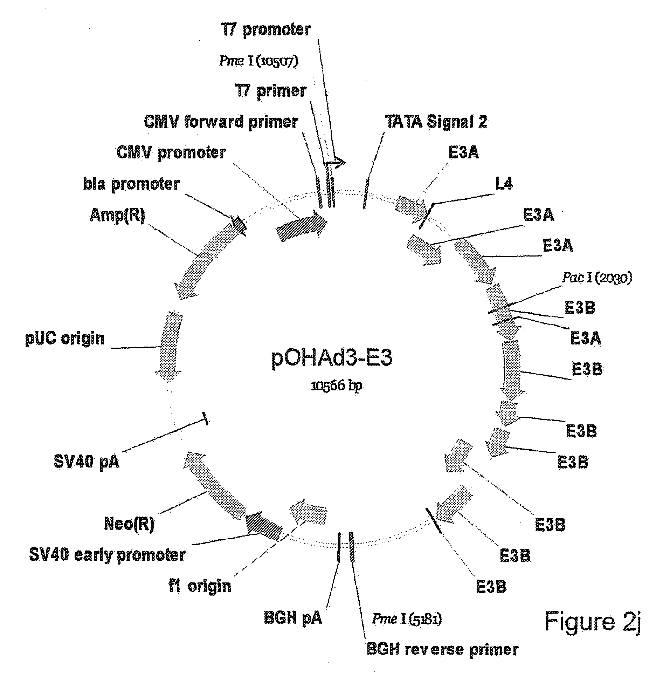

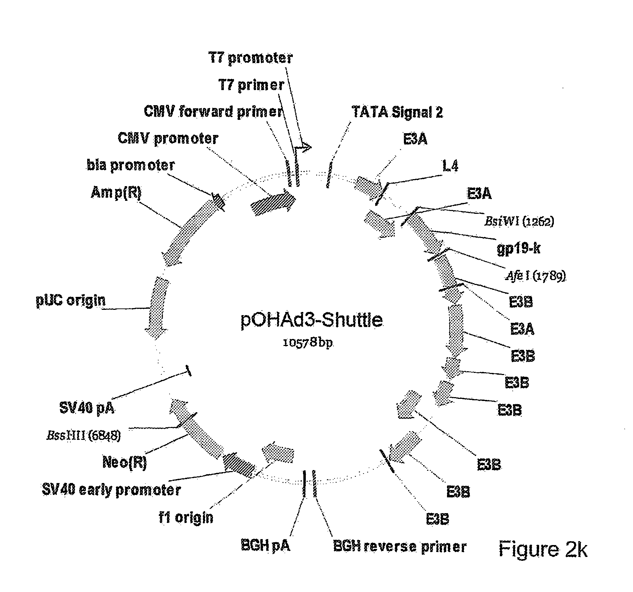

HNIS and/or GMCSF transgene is (are) added to the human Ad3 based vector of the invention by utilizing methods known in the art or as described in the above examples or in the examples below, wherein a non-Ad5 adenoviral vector of the present invention, for example Ad3-hTERT-E1A, can be used instead of the Ad5/3-Δ24 backbone (see also Figures 1 and 2):

### 1. HNIS transgene

### MATERIALS AND METHODS

### Cell culture

Andorgen independent prostate cancer cell lines 22Rv1, PC-3, DU-145 and lung adenocarcinoma cell line A549 were purchased from ATCC (Manassas, VA). PC-3MM2 cells are a metastatic hormone refractory sub line of PC-3 (courtesy of Isaiah J. Fidler, MD Anderson Cancer Center, Houston, TX). Human embryonic kidney epithelial 293 cells were purchased from Microbix (Toronto, Canada). Cell line 911 was obtained from Dr Alex J. van der Eb (University of Leiden, The Netherlands). All cell lines were cultured in the recommended conditions.

### Viral constructs

To create Ad5/3-Δ24-hNIS, EcoRI-digested and blunted HNIS fragment (from pcDNA3-hNIS, courtesy of Steve Russell, Mayo Clinic, Rochester, MN) was inserted into BsiWI-Mfel-digested and blunted pTHSN (Kanerva A. et al. Gene Ther 12: 87-94) to obtain pTHSN-hNIS. To make the control virus Ad5/3-Δ24-Δgp19K, BsiWI-Mfel-digested and blunted pTHSN was self-ligated resulting in pTHSN-Δgp19K. In order to obtain pAd5/3-Δ24-hNIS and pAd5/3-Δ24Δgp19K, Pmel-linearized pShuttle-Δ24 (Suzuki, K. et al. 2002, Clin Cancer Res 8: 3348-59) and pAd5/3- E1-hNIS or pAd5/3-E1-Δgp19K were electroporated into BJ5183 cells.

Adenoviral plasmids were linearized and transfected into 911 cells with Superfect (Qiagen, Valencia, CA) following the manufacturer's instructions. Adenovirus colonies were picked and viruses were propagated in A549 cells and purified using standard techniques. Ad5/3-Δ24 and Ad5/3luc1 viruses were constructed previously (Kanerva, A. et al. 2003, Mol Ther 8: 449-58; Kanerva, A, et al. 2002, Clin Cancer Res 8: 275-80). Viral particles (VP) were determined with spectrophotometry and plaque forming units (pfu) with TCID50 assay. Titers were Ad5/3-Δ24- hNIS: 1.1 x 10¹² VP/ml; 9.0 x 10¹⁰pfu/ml, Ad5/3-Δ24-Δgp19K: 1.5 x 10¹² VP/ml; 2.8 x 10¹¹ pfu/ml, Ad5/3-Δ24: 1.7 x 10¹² VP/ml and Ad5/3luc1: 6.9 x 10¹¹ VP/ml.

### RT-PCR

Prostate cancer cells were infected with Ad5/3-Δ24-hNIS and Ad5/3-Δ24-Δgp19K (10 VP/cell) for 2 h at +37°C. Cells were harvested 24 and 48 h after infection. Total RNA was isolated using the RNeasy Kit (Qiagen) and treated with DNase before RT-PCR. 350 ng of total RNA was used for each reaction. Amplification (35 cycles, annealing at +54°C) was carried out with the OneStep RTPCR Kit (Qiagen) using hNIS-specific primers (Forward 5'-CTT CTG AAC TCG GTC CTC AC-3' (SEQ ID NO: 10); Reverse 5'-TCC AGA ATG TAT AGC GGC TC-3' (SEQ ID NO: 11) and β-actin-specific primers (Forward 5'- CGA GGC CCA GAG CAA GAC A -3' (SEQ ID NO: 12); Reverse 5'-CAC AGC TTC TCC TTA ATG TCA CG -3' (SEQ ID NO: 13). PCR product size for HNIS and β-action was 453 bp and 482 bp, respectively.

### Iodide uptake

Prostate cancer cells were infected with Ad5/3-Δ24-hNIS and Ad5/3-Δ24-Δgp19K (10 VP/cell) for 2 h at +37°C. Cells were washed with 1 x PBS 24 and 48 h post infections and incubated with 7.4 kBq of sodium iodide [¹²⁵I]Nal (MAP Medical Technologies Oy, Tikkakoski, Finland) for 20 min at room temperature. Cells were washed twice with 1 x PBS followed by lysis using 300 µl of Cell Culture Lysis Reagent for each sample (Promega, Madison, WI). Radioactivity was quantified with a well counter connected with a multi-channel analyzer Atomlab 950 (Biodex Medical System, Shirley, NY).

### Cell killing assays

Prostate cancer cells were infected with Ad5/3-Δ24-hNIS, Ad5/3-Δ24-Δgp19K, Ad5/3-Δ24 and Ad5/3luc1 using 0.01, 0.1, 1, 10 and 100 VP/cell. Growth medium was replaced every other day. Cell viability was determined 6 days post infection (p.i.) for PC-3MM2, 7 days p.i. for DU-145 and PC-3 and 9 days p.i. for 22Rv1 cells.

-9 days post infection with CellTiter 96 AQueous One Solution cell proliferation assay [also called the 3-(4,5-dimethylthiazol-2-yl)-5-(3-carboxy-methoxyphenyl)-2-(4-sulfophenyl)-2H-tetrazolium [MTS] assay; Promega, Madison, WI).

### Tissue culture infectious dose (TCID50) assay

*Cell line samples:* 5 x 10⁴ prostate cancer cells/well were plated in triplicates in 1 ml of 5% GM on 24-well plates, incubated overnight and infected with Ad5/3-Δ24-hNIS (5 vp/cell) for 2 h at +37°C. At 8, 24, 48 and 72 h post infection cells and growth media were collected, frozen at -80°C and freeze-thawn three times. For TCID50, the cells were spun down and the supernatant was added to 293 cells.

*Tissue samples:* Organs collected from mice treated with Ad5/3-Δ24-hNIS and ¹³¹I were collected, weighed, manually homogenized and diluted into 800 µl of DMEM. They were freeze-thawed at -80°C three times. For TCID50, the tissue samples were spun down and the supernatant was added to 293 cells.

For both experiments, 10⁴4 293 cells were plated in 100 µl of 5% DMEM into 96-well plates, incubated overnight, and infected with the supernantants of aboce experiments. After 10 days, the development of CPE was assessed to estimate the titer.

### In vivo studies

Male NMRI/nude mice were purchased from Taconic (Ejby, Denmark). Tumors were established in eight-week old mice by subcutaneous injection of 5 x 10⁶ PC-3MM2 cells under medetomidineketamine- 0.9% saline (1:2:7) anesthesia. In the imaging experiment, 3 mice received intratumorally saline (upper left tumor), 7 x 10⁸ VP Ad5/3-Δ24-Δgp19K (upper right tumor) and 7 x 10⁸ VP Ad5/3-Δ24-hNIS (lower right and left tumors) 11 and 12 days after tumor cell inoculation. A day after the last viral injection mice received intravenously 1.85 MBq of sodium iodide [¹²³I]Nal (MAP Medical Technologies Oy, Tikkakoski, Finland) followed by imaging with gamma camera Toshiba 7200A/UI (Toshiba, Tokyo, Japan) 0.5 h, 1 h, 1.5 h, 2h, 4h and 13h after iodide exposure. Animal regulation did not allow analysis of later time points. The gamma energy peak 159 keV was recorded using 20% window, and 150 kilocounts were collected per image. The used matrix size was 256 x 256 x 16, this made the pixel size 1.08 mm x 1.08 mm. At 13 h, various organs (heart, lung, liver, spleen, kidney, thyroid, stomach, muscle, blood and bone) and tumors were collected, weighed and analysed with a well counter connected with a multi-channel analyzer Atomlab 950 (Biodex Medical System, Shirley, NY). Results are expressed as percentage from initial iodide dose per gram of tissue (ID%/g).

For the therapeutic experiment, tumor bearing mice were randomized into 4 groups (6 mice/group, 2 tumors/mouse): mock, 131I, Ad5/3-Δ24-hNIS and Ad5/3-Δ24-hNIS + ¹³¹I. 6 and 7 days after tumor cell inoculation mice received intratumorally either growth medium or 7 x 108 VP of Ad5/3-Δ24-hNIS per tumor. A day after the last viral injection, mice received intraperitoneally 50 MBq of carrier-free sodium iodide ([¹³¹I]Nal, MAP Medical Technologies Oy, Tikkakoski, Finland). Tumor size was measured every other day and the volume calculated using the formula (length x width2 x 0.5). When mice were killed due to the tumor size, the organs were collected, weighed and their radioactivity determined with a gamma counter (Wizard 3, PerkinElmer, Turku, Finland) for 180 seconds. The results are expressed as percentage from initial iodide dose per gram of tissue (ID%/g). The whole body emitted radioactivity was measured at days 9, 10, 11, 13, 14, 15, and 17. On days 9-11, measurements were performed on a dose calibrator (CRC-120, Capintec, Inc., Ramsey, NJ), and after day 11 on a whole-body measurement apparatus consisting of a lead measurement chamber and an UniSpec tube-base multichannel analyzer with sodium iodide scintillation crystal (Canberra Industries, Inc., Meridien, CT). The mice were weighed at day 14, and the same weight was used in calculations throughout the experiment.

In all experiments, mice were killed when the tumor size reached 15 mm in any diameter, as required by animal regulations. The therapeutic *in vivo* experiment had to be stopped on day 17 due to animal husbandry constraints. All the animal experiments were approved by the national committee for animal experimentation in Finland (State Provincial Office of Southern Finland, Hämeenlinna, Finland).

### Statistical analysis

Analysis of the iodide uptake was performed by one-way analysis of variance (ANOVA) with Bonferroni's post-hoc test. The analysis of the tumor volume over time between experimental groups was performed using a repeated measures model with PROC MIXED (SAS Ver. 9.1, Cary, NC). The tumor volume measurements were log transformed for normality. The a priori planned comparisons of specific differences in predicted treatment means averaged over time and at each time point by t-statistics and Tukey-Kramer model was used for adjustments. For all analyses a two-sided p value of <0.05 was deemed statistically significant.

### Results

### HNIS specific mRNA is detected in Ad5/3-Δ24-hNIS infected prostate cancer cells

A new virus containing the Ad3 knob in the Ad5 fiber shaft, a 24 bp deletion in the *E1A* region and HNIS inserted in the *E3* was constructed. To assess the expression of the transgene from the virus, hormone refractory prostate cancer cell lines PC-3MM2, 22Rv1, PC-3 and DU-145 were infected with Ad5/3-Δ24-hNlS or Ad5/3-Δ24-Δgp19K (a positive control without a transgene) and analysed with RT-PCR 24 and 48h after infection. All Ad5/3-Δ24-hNIS infected cell lines featured a 454 bp sized, HNIS specific amplification product, suggesting that HNIS is expressed from the virus genome. In contrast, Ad5/3-Δ24-Δgp19K infected cells or cells *per se* did not yield to an amplification product.

### Ad5/3-Δ24-hNIS can mediate efficient iodide uptake into prostate cancer cells

In order to assess the functionality of the HNIS expressed from the virus, prostate cancer cells were infected with Ad5/3-Δ24-hNIS or Ad5/3-Δ24-Δgp19K followed by exposure to ¹²⁵I and quantification of the iodide accumulation (Fig. 11). When compared to non-infected or control virus treated cells, Ad5/3-Δ24-hNIS resulted in a significantly higher iodide uptake in all examined cell lines. In comparison to the mock infection, Ad5/3-Δ24-hNIS caused up to 2.5 (*p*<0.001) and 3 (*p*<0.001) times higher iodide accumulation in PC-3MM2 and 22Rv1 cells, respectively. In PC-3 and DU-145 cells a more modest, but still significantly enhanced accumulation was seen when compared to mock treated cells (up to 1.6 (*p*<0.05) and 1.3 (*p*<0.001) times higher levels, respectively). Ad5/3-Δ24-Δgp19K treatment did not result in iodide accumulation, suggesting that iodide uptake was mediated by HNIS expressed from Ad5/3-Δ24-hNIS rather than from viral infection or replication *per se.*

### Ad5/3-Δ24-hNIS replicates in and causes oncolysis of prostate cancer cells

Prostate cancer cell lines were infected with Ad5/3-Δ24-hNIS, Ad5/3-Δ24-Δgp19K, Ad5/3-Δ24 and Ad5/3luc1 followed by determination of cell viability 6 (PC3-MM2), 7 (PC-3 and DU-145) or 9 (22Rv1) days post infection (Fig. 12). Ad5/3-Δ24-hNIS showed efficient cell killing in all examined prostate cancer cell lines. The most rapid and efficient oncolysis was seen in PC-3MM2 cells, where almost complete cell killing was achieved 6 days after infection using 1 VP/cell of Ad5/3-Δ24-hNIS. In addition, oncolysis was similar to that seen with Ad5/3-Δ24-Δgp19K. In other prostate cancer cell lines, the oncolytic potency of Ad5/3-Δ24-hNIS was slightly less than Ad5/3-Δ24-Δgp19K. In fact, Ad5/3-Δ24-Δgp19K which has the partial deletion in E3, showed the highest oncolytic potency in all examined cell lines. It is possible that the larger genome size of Ad5/3-Δ24-hNIS may impact the speed of oncolysis as compared to Ad5/3-Δ24-Δgp19K. The HNIS transgene is ~2.2 kbp, which together with the 1 kbp Δgp19K keeps the genome size under 105%, which has been suggested important for retaining virus functionality. The replicativity of Ad5/3-Δ24-hNIS was assessed in PC-3 and 22Rv1 cells where the TCID50 assay showed the increase in infectious particles in function of time.

### Ad5/3-Δ24-hNIS can mediate radioactive iodide uptake into prostate cancer tumors

Subcutaneous PC-3MM2 tumors were established in nude/NMRI male mice. The lower tumors were infected intratumorally on two consecutive days with 7 x 10⁸ VP/tumor of Ad5/3-Δ24-hNIS and the upper right tumor with the same dose of Ad5/3-Δ24-Δgp19k (Figs. 13A and B). The upper left tumor was mock treated. A day after the last viral injection, 1.85 MBq of ¹²³I per mouse was administered intravenously and mice were imaged with a gamma camera from 0.5 to 13 h after the ¹²³I injection. Some iodide accumulation into Ad5/3-Δ24-hNIS treated tumors was seen already 0.5 h after the ¹²³I injection. Two hours after the iodide exposure, a strong iodide accumulation was seen throughout Ad5/3-Δ24-hNIS treated tumors, whereas tumors injected with a control virus or saline did not uptake iodide (Fig. 13A). After 2h, the iodide accumulation reached a plateau and remained relatively constant up to the last imaging time point of the experiment 13 h after the iodide injection (Fig. 13B). In addition, iodide was heavily accumulated into thyroid and stomach due to the endogenous NIS expression of these organs. A high stomach uptake is typically seen in mice but not in humans. In patients, it would be possible to protect the normal thyroid from radioiodide, but this was not done in this mouse study. Since clearance of the radioactive iodide occurs through the urine, iodide accumulation in the bladder was seen.

To quantify ¹²³I biodistribution *in vivo*, organs and tumors were collected after imaging and their radioactive content was determined (Fig. 13C). Because of the endogenous HNIS expression, the thyroid and stomach captured about 30% of the injected dose. Nevertheless, the Ad5/3-Δ24-hNIS treated tumors accumulated more than 6% of the total iodide dose. This correlated with large amounts of infectious virus present in tumors, while only trace amounts were seen in the thyroid and stomach. Since tumors were collected more than 10 days after the virus injection and TCID50 measures infectious virus, high TCID50 values probably reflect virus replication. Adenoviruses lose their capsid when they enter cells and thus the input virus cannot be detected with TCID50; it requires production of new virions. The iodide uptake remained low in other organs including mock or Ad5/3-Δ24-Δgp19K treated tumors (<1% of the initial dose).

### Ad5/3-Δ24-hNIS with ¹³¹I inhibits tumor growth in vivo

To assess the efficacy of Ad5/3-Δ24-hNIS *in vivo*, prostate tumor bearing mice were treated with ¹³¹I or Ad5/3-Δ24-hNIS alone, or with their combination (Fig. 14). When tumors were treated with Ad5/3-Δ24-hNIS without radioiodide, tumor growth was significantly slower than in the mock or ¹³¹I alone groups (p<0.05 for both). When the mice received both Ad5/3-Δ24-hNIS and 131I, tumor sizes were significantly smaller than in any other group (all *p* <0.001). An adjusted pairwise analysis indicated a significant difference between Ad5/3-Δ24-hNIS + ¹³¹I *versus* virus alone, ¹³¹I alone or mock already on day 2 (all p <0.05). The adjusted pairwise *p*-values between Ad5/3-Δ24-HNIS + ¹³¹I *versus* virus alone, ¹³¹I alone or mock on days 4, 6 and 8 were also all <0.001. Due to animal husbandry constraints, it was predefined that the experiment would be ended on day 17, although some of the mice were still in good condition.

Whole body emitted radioactivity was measured starting from one day after the iodide injection. Radioactivity declined rapidly during the next 3 days as expected by clearance through kidneys.

When tumor size reached 15 mm in any diameter, mice were killed and organs were collected for radioactivity measurements. Radioactivity declined over time and most radioactivity was seen in the thyroid gland and stomach, and briefly in the heart probably due to propagation of ¹³¹I by circulation. In other organs the radioactivity remained low. Importantly, the virus injection did not affect iodide biodistribution and therefore the safety profile of the approach resembles that of radioiodide therapy for thyroid cancer.

### II. GM-CSF transgene

### Cloning of three D24-GM-CSF type viruses

- PCR out hGM-CSF,
- create Sunl/Munl sites => 445 bp (pORF-GM-CSF as a template)
- Sunl/Munl digestion of PCR product and pTHSN
- Sticky-end ligation =>
- *Pme*I linearized pShuttle-D24 + pTG3602 => pAd5-D24
- Ad5-D24-GM-CSF
   Homol recomb: *Srf*I linearized pAd5-D24 + *Fsp*I linearized
   pTHSN-GM-CSF => pAd5-D24-GM-CSF
   Pacl linearization & transfection => Ad5-D24-GM-CSF

All phases of the cloning were confirmed with PCR and multiple restriction digestions. The shuttle plasmid pTHSN-GMCSF was sequenced. Absence of wild type E1 was confirmed with PCR. The E1 region, transgene and fiber were checked in the final virus with sequencing and PCR, which was then taken to the clean lab for production. To this end, viral DNA was extracted by over night (ON) incubation with an appropriate buffer solution and after PCR and sequencing was performed to analyze the integrity of the fiber as well as the GMCSF cDNA. All phases of the virus production, including transfection, were done on A549 cells to avoid risk of wild type recombination as described before (Kanerva, A. et al. 2003, Mol Ther 8, 449-58; Baeurschmitz, G.J. et al. 2006, Mol Ther 14, 164-74). GM-CSF is under the E3 promoter, which results in replication associate transgene expression, which starts about 8h after infection. E3 is intact except for deletion of *6.7Klgp19K.* Ad5/3-D24-GM-CSF and Ad5-RGDD24-GM-CSF were constructed identically, except a rescue plasmid featuring either a knob from serotype 3, or RGD-4C in the Ad5 fiber HI-loop were used.

### In vitro analysis of D24-GM-CSF type viruses

*The in vitro* efficacy of D24-GM-CSF type viruses was studied in lung cancer cells (A549), breast cancer stem cell derived explant cells (JIMT-1) and breast cancer cells (MDA-MB-436) utilizing MTS cell killing assays. The MTS assay is currently the standard method to assess cell viability in cancer gene therapy publications. Ad5Luc1 is a replication deficient virus and acts as a negative control. Ad5wt is a wild type Ad5 virus (strain Ad300wt) and was used as a positive control. Ad5-d24-E3 harbors an isogenic 24bp deletion in E1 but is intact in E3. VP indicates virus particles.

In summary, Ad5-D24-GMCSF had oncolytic activity similar to positive controls in vitro, and therefore transgene production did not compromise the oncolytic potency of the virus (Figures 15a-c). Similar data was shown for Ad5/3-D24-GM-CSF and Ad5-RGD-D24-GM-CSF (Figure 15d).

To test whether Ad5D24-GMCSF was able to express the transgene, A549 cell line was infected with 1000 VP/cell and media were collected over time. The concentration of GMCSF (Figure 16a) in the media was measured by FACSARRAY. In addition to that, we also analyzed whether the virus-expressed GMCSF retained its biological function. To this end, TF1 cell line, whose growth and survival is strictly dependent on human GMCSF, was treated with media collected from a A549 cell line previously infected with Ad5D24-GMCSF. The TF1 viability was assessed over time by the MTS assay. The result of this experiment was that the virus expressed-GMCSF was able to stimulate growth of such cell line, and no difference was found with the same cell line treated with human recombinant GMCSF (Sigma) (Figure 16b).

### In vivo analysis of D24-GM-CSF type viruses in animals

The *in vivo* efficacy of Ad5-D24-GM-CSF was tested in immune competent Syrian hamsters, which are semipermissive for human adenovirus replication (mice are non-permissive) (Ying B. et al. 2009, Cancer Gene Ther doi:10.1038/cgt.2009.6.). 7x10⁶ HapT1 pancreatic cancer cells were injected subcutaneously and 1x10⁹ virus particles (VP) of Ad5-D24-GM-CSF or Ad5D24E3 (which does not express GM-CSF) were injected intratumorally on day 0, 2 and 4. The mock group was injected with the same volume of growth media at the same indicated time points. Figure 17b shows that intratumoral injections of Ad5-D24-GMCSF resulted in high levels of hGM-CSF in the serum of Syrian hamsters. Human GM-CSF is known to be active in Syrian hamsters (Cho, Exp Toxicol Pathol 2006 vol. 57 (4) pp. 321-8). Interestingly, all animals were tumor-free by day sixteen except for mock group (Figure 17a). Tumor scars were still analyzed for two additional weeks to assess whether reappearance of the tumor might have occurred. However, on day 32 there was still no sign of tumor in these animals so that the first part of the experiment was terminated and animals of the Mock group were euthanized. The remaining treated animals were at this point challenged with the same tumor in the right side of the upper body by subcutaneous injection of 7x10⁶ HapT1 cells, while on the left side were challenged with a different tumor (1x10⁶ of HaK tumor) for which the animals were naïve. Tumor growth was measured over time and is reported in Figures 17c-d. Interestingly, the animals that were previously treated with Ad5D24GMCSF completely rejected the HapT1 tumor challenge, while Hak tumors grew normally, whereas in the animals that were previously treated with Ad5D24E3 HapT1 and HaK tumors grew independently (Figures 17c-d).

In summary, the data indicates that Ad5-D24-GM-CSF has an antitumor activity in immune competent tumor bearing animals, and it is able to elicit tumor-specific immunity to the degree of rejecting subsequent challenge of the same tumor.

### III. CD40L transgene

Using methods similar to those described above Ad3-hTERT-CD40L-EIA was constructed and analyzed for biodistribution in mice (Figures 20a-b). Murine biodistribution at 6h after 5x10¹⁰ VP intravenously (Figure 20a) suggested that much of the serotype 3 virus stays still in the blood and blood rich organs, such as spleen, lung and the liver. All major organs were analyzed by qPCR (Figure 20b). Entry to other organs was found attenuated. In this assay blood clots β-actin was used for the serum values. Due to the lack of β-actin in blood compartments, blood values are best compared with absolute values as done in Fig 20a. The results suggest that Ad3 virus does not associate with RBCs or serum but is abundantly present in blood clots and plasma. Thus WBCs and platelets are possible Ad3 virus carriers in mice.

## Claims

1. An oncolytic human adenoviral vector comprising a fully serotype 3 backbone and a tumor specific human telomerase catalytic domain hTERT promoter controlling the adenovirus serotype 3 Ad3 E1A region.

2. An oncolytic human adenoviral vector according to claim 1, comprising a replication activated Ad3 E3 promoter controlling the Ad3 E3 region.

3. An oncolytic human adenoviral vector according to claim 1 or 2 comprising one or more transgenes, such as granulocyte-macrophage colony-stimulating factor (GM-CSF), human sodium iodide symporter (hNIS), interferon alpha, interferon beta, interferon gamma, tumor necrosis factor, CD40L, trastuzumab and other monoclonal antibodies.

4. An oncolytic human adenoviral vector according to claim 3, wherein the transgene is placed in E1 under the hTERT promoter and/or in E3 under the replication activated Ad3 E3 promoter.

5. An oncolytic human adenoviral vector according to any one of claims 1-4 comprising internal ribosomal entry site (IRES) between the transgene and the E1A region.

6. An oncolytic human adenoviral vector according to any one of claims 1-5 comprising the hTERT promoter, transgene, IRES and E1A.

7. An oncolytic human adenoviral vector according to any one of the previous claims comprising at least one expression cassette.

8. A cell comprising the adenoviral vector according to any one of claims 1-7.

9. A pharmaceutical composition comprising the adenoviral vector according to any one of claims 1-7.

10. An oncolytic human adenoviral vector or a pharmaceutical composition according to any one of the previous claims as an *in situ* cancer vaccine.

11. An adenoviral vector according to any one of claims 1-7 for use in the treatment of cancer in a subject.

12. Use of an adenoviral vector according to any one of claims 1-7 in the manufacture of a medicament for treating cancer in a subject.

13. A use according to claim 12 or an adenoviral vector for use according to claim 11, wherein the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

14. A use according to claim 12 or 13 or an adenoviral vector for use according to claim 11 or 13, wherein the subject is a human or an animal.

15. A use according to any one of claims 12-14 or an adenoviral vector for use according to any one of claims 11, 13 or 14 wherein the adenoviral vector or pharmaceutical composition is administered through an intratumoral, intramuscular, intra-arterial, intravenous, intrapleural, intravesicular, intracavitary or peritoneal injection, or an oral administration, preferably several times during the treatment period.

16. A use according to any one of claims 12-15 or an adenoviral vector for use according to any one of claims 11 or 13-15, wherein the adenoviral vector or pharmaceutical composition is administered concurrently with radiotherapy or radioiodide to a subject, or concurrent chemotherapy to a subject.

17. A use according to any one of claims 12-16 or an adenoviral vector for use according to any one of claims 11 or 13-16 wherein the subject to be treated has high amounts of anti-Ad5 neutralizing antibodies and/or has previously been treated with Ad5.

18. A method of producing an adenoviral vector according to any one of claims 1-7 comprising:
i) providing a DNA vector comprising at least partial Ad3 DNA and one or several tumor or tissue specific promoters and/or optionally one or several transgenes, and
ii) inserting the remainder of the Ad3 genome and optionally one or several tumor or tissue specific promoters and/or optionally one or several transgenes to the vector;
whereby an oncolytic human adenoviral vector comprising a fully serotype 3 backbone and a tumor-specific hTERT promoter controlling the Ad3 E1A region is produced.

## Patentansprüche

1. Onkolytischer humaner adenoviraler Vektor umfassend ein vollständiges Serotyp-3- Rückgrat und einen tumorspezifischen humanen katalytischen Telomerase-Bereichs-hTERT-Promoter, der die Adenovirus-Serotyp-3-Ad3E1A-Region steuert.

2. Onkolytischer humaner adenoviraler Vektor nach Anspruch 1, umfassend einen replikationsaktivierten Ad3E3-Promoter, der die Ad3E3-Region steuert.

3. Onkolytischer humaner adenoviraler Vektor nach Anspruch 1 oder 2, umfassend eines oder mehrere Transgene, wie zum Beispiel Granulozyt-Makrophagen koloniestimulierenden Faktor (GM-CSF), humanen Natrium-Jodid-Symporter (hNIS), Alpha-Interferon, Beta-Interferon, Gamma-Interferon, Tumornekrosefaktor, CD40L, Trastuzumab und andere monoklonale Antikörper.

4. Onkolytischer humaner adenoviraler Vektor nach Anspruch 3, wobei das Transgen in E1 unter dem hTERT-Promoter und/oder in E3 unter dem replikationsaktivierten Ad3E3-Promoter angeordnet wird.

5. Onkolytischer humaner adenoviraler Vektor nach einem der Ansprüche 1 bis 4, umfassend die interne ribosomale Eintrittsstelle (internal ribosomal entry site, IRES) zwischen dem Transgen und der E1A-Region.

6. Onkolytischer humaner adenoviraler Vektor nach einem der Ansprüche 1 bis 5, umfassend den hTERT-Promoter, Transgen, IRES und E1A.

7. Onkolytischer humaner adenoviraler Vektor nach einem der vorangehenden Ansprüche, umfassend wenigstens eine Expessionskassette.

8. Zelle umfassend den adenoviralen Vektor gemäß einem der Ansprüche 1 bis 7.

9. Pharmazeutische Zusammensetzung umfassend den adenoviralen Vektor gemäß einem der Ansprüche 1 bis 7.

10. Onkolytischer humaner adenoviraler Vektor oder eine pharmazeutische Zusammensetzung nach einem der vorangehenden Ansprüche als *in situ-*Krebsimpfstoff.

11. Adenoviraler Vektor nach einem der Ansprüche 1 bis 7 zur Verwendung bei der Behandlung von Krebs bei einem Patienten.

12. Verwendung eines adenoviralen Vektors nach einem der Ansprüche 1 bis 7 bei der Herstellung eines Medikamentes zur Behandlung von Krebs bei einem Patienten.

13. Verwendung nach Anspruch 12 oder ein adenoviraler Vektor zur Verwendung nach Anspruch 11, wobei der Krebs ausgewählt ist aus einer Gruppe bestehend aus Krebs des Nasen-Rachenraumes, Synovialkrebs, Hepatozellularkrebs, Nierenkrebs, Krebs des Bindegewebes, Melanomen, Lungenkrebs, Darmkrebs, Dickdarmkrebs, Mastdarmkrebs, Kolorektalkrebs, Hirnkrebs, Kehlkopfkrebs, Mundhöhlenkrebs, Leberkrebs, Knochenkrebs, Bauchspeicheldrüsenkrebs, Choriokarzinom, Gastrinom, Phäochromozytom, Prolaktinom, T-Zellen-Leukämie/Lymphom, Neurom, von Hippel-Lindau-Erkrankung, Zollinger-Ellison-Syndrom, Nebennierenkrebs, Analkrebs, Gallenwegskrebs, Blasenkrebs, Harnleiterkrebs, Hirnkrebs, Oligodendrogliom, Neuroblastom, Meningiom, Rückenmarktumor, Knochenkrebs, Osteochondrom, Chondrosarkom, Ewing-Sarkom, Krebs unbekannten Ursprungsortes, Karzinoid, Karzinoid des Magen-Darm-Traktes, Fibrosarkom, Brustkrebs, Paget-Karzinom, Gebärmutterhalskrebs, Kolorektalkrebs, Mastdarmkrebs, Speiseröhrenkrebs, Gallenblasenkrebs, Kopfkrebs, Augenkrebs, Nackenkrebs, Nierenkrebs, Wilm's Tumor, Leberkrebs, Kaposisarkom, Prostatakrebs, Lungenkrebs, Hodenkrebs, Hodgkinsche Krankheit, Non-Hodgkin-Lymphom, Mundkrebs, Hautkrebs, Mesotheliom, multiples Myelom, Eierstockkrebs, endokriner Bauchspeicheldrüsenkrebs, Glukagonom, Bauchspeicheldrüsenkrebs, Nebenschilddrüsenkrebs, Peniskrebs, Hypophysenkrebs, Weichgewebesarkom, Retinoblastom, Dünndarmkrebs, Magenkrebs, Thymuskrebs, Schilddrüsenkrebs, Trophoblastkrebs, Blasenmole, Gebärmutterkrebs, Endometriumkrebs, Vaginalkrebs, Vulvakrebs, akustisches Neurom, Mycosis fungoides, Insulinom, Karzinoidsyndrom, Somatostatinom, Zahnfleischkrebs, Herzkrebs, Lippenkrebs, Hirnhautkrebs, Mundkrebs, Nervenkrebs, Gaumenkrebs, Ohrspeicheldrüsenkrebs, Peritoneumkrebs, Pharynxkrebs, Pleurakrebs, Speicheldrüsenkrebs, Zungenkrebs und Mandelkrebs.

14. Verwendung nach Anspruch 12 oder 13 oder ein adenoviraler Vektor zur Verwendung nach Anspruch 11 oder 13, wobei der Patient ein Mensch oder ein Tier ist.

15. Verwendung nach einem der Ansprüche 12 bis 14 oder ein adenoviraler Vektor zur Verwendung nach einem der Ansprüche 11 , 13 oder 14, wobei der adenovirale Vektor oder die pharmazeutische Zusammensetzung durch eine intratumorale, intramuskuläre, intraarterielle, intravenöse, intrapleurale, intravesikale, intrakavitäre oder peritoneale Injektion oder eine orale Verabreichung verabreicht werden, bevorzugt mehrmals während des Behandlungszeitraumes.

16. Verwendung nach einem der Ansprüche 12 bis 15 oder ein adenoviraler Vektor zur Verwendung nach einem der Ansprüche 11 oder 13 bis 15, wobei der adenovirale Vektor oder die pharmazeutische Zusammensetzung einem Patienten gleichzeitig mit Radiotherapie oder Radiojodid verabreicht werden oder gleichzeitig zur Chemotherapie eines Patienten.

17. Verwendung nach einem der Ansprüche 12 bis 16 oder ein adenoviraler Vektor zur Verwendung nach einem der Ansprüche 11 oder 13 bis 16, wobei der zu behandelnde Patient hohe Mengen an anti-Ad5-neutralisierenden Antikörpern hat, und/oder vorher mit Ad5 behandelt wurde.

18. Verfahren zur Herstellung eines adenoviralen Vektors nach einem der Ansprüche 1 bis 7, umfassend:
i) Bereitstellung eines DNA-Vektors, der wenigstens einen Teil der Ad3DNA umfasst und einen oder mehrere tumor- oder gewebespezifische/n Promoter/en und/oder optional ein oder mehrere Transgen/e; und
ii) Einbringen des Restes des Ad3-Genoms und optional eines oder mehrerer tumor- oder gewebespezifischen/r Promoters/en und/oder optional eines oder mehrerer Transgens/e zum Vektor;
wobei ein onkolytischer humaner adenoviraler Vektor umfassend ein vollständiges Serotyp-3-Rückgrat und einen tumorspezifischen hTERT-Promoter, der die Ad3 E1A-Region steuert, erzeugt wird.

## Revendications

1. Vecteur adénoviral humain oncolytique comprenant un squelette totalement de sérotype 3 et un promoteur hTERT de domaine catalytique de télomérase humaine à spécificité tumorale contrôlant la région E1A d'adénovirus de sérotype 3 Ad3.

2. Vecteur adénoviral humain oncolytique selon la revendication 1, comprenant le promoteur E3 d'Ad3 activé par réplication contrôlant la région E3 d'Ad3.

3. Vecteur adénoviral humain oncolytique selon la revendication 1 ou 2, comprenant un ou plusieurs transgènes, tels que le facteur de stimulation des colonies de granulocytes et de macrophages (GM-CSF), le symporteur sodium/iodure humain (hNIS), l'interféron alpha, l'interféron bêta, l'interféron gamma, le facteur de la nécrose tumorale, le CD40L, le trastuzumab et d'autres anticorps monoclonaux.

4. Vecteur adénoviral humain oncolytique selon la revendication 3, dans lequel le transgène est placé dans E1 sous le promoteur hTERT et/ou dans E3 sous le promoteur E3 d'Ad3 activé par réplication.

5. Vecteur adénoviral humain oncolytique selon l'une quelconque des revendications 1 à 4, comprenant un site d'entrée interne des ribosomes (IRES) entre le transgène et la région E1A.

6. Vecteur adénoviral humain oncolytique selon l'une quelconque des revendications 1 à 5 comprenant le promoteur hTERT, le transgène, l'IRES et l'E1A.

7. Vecteur adénoviral humain oncolytique selon l'une quelconque des revendications précédentes, comprenant au moins une cassette d'expression.

8. Cellule comprenant le vecteur adénoviral selon l'une quelconque des revendications 1 à 7.

9. Composition pharmaceutique comprenant le vecteur adénoviral selon l'une quelconque des revendications 1 à 7.

10. Vecteur adénoviral humain oncolytique ou composition pharmaceutique selon l'une quelconque des revendications précédentes en tant que vaccin anticancéreux *in situ.*

11. Vecteur adénoviral selon l'une quelconque des revendications 1 à 7 pour une utilisation dans le traitement du cancer chez un sujet.

12. Utilisation d'un vecteur adénoviral selon l'une quelconque des revendications 1 à 7 dans la fabrication d'un médicament pour le traitement du cancer chez un sujet.

13. Utilisation selon la revendication 12 ou vecteur adénoviral pour une utilisation selon la revendication 11, dans lequel le cancer est choisi dans un groupe constitué de cancer rhinopharyngé, cancer synovial, cancer hépatocellulaire, cancer rénal, cancer des tissus conjonctifs, mélanome, cancer du poumon, cancer de l'intestin, cancer du côlon, cancer rectal, cancer colorectal, cancer du cerveau, cancer de la gorge, cancer oral, cancer du foie, cancer des os, cancer pancréatique, choriocarcinome, gastrinome, phéochromocytome, prolactinome, leucémie/lymphome à cellules T, névrome, maladie de von Hippel-Lindau, syndrome de Zollinger-Ellison, cancer surrénalien, cancer anal, cancer des canaux biliaires, cancer de la vessie, cancer de l'uretère, cancer du cerveau, oligodendrogliome, neuroblastome, méningiome, tumeur de la moelle épinière, cancer des os, ostéochondrome, chondrosarcome, sarcome de Ewing, cancer de site primaire inconnu, carcinoïde, carcinoïde du tube digestif, fibrosarcome, cancer du sein, maladie de Paget, cancer du col de l'utérus, cancer colorectal, cancer rectal, cancer de l'oesophage, cancer de la vésicule biliaire, cancer de la tête, cancer de l'oeil, cancer du cou, cancer du rein, tumeur de Wilms, cancer du foie, sarcome de Kaposi, cancer de la prostate, cancer du poumon, cancer testiculaire, maladie de Hodgkin, lymphome non hodgkinien, cancer oral, cancer de la peau, mésothéliome, myélome multiple, cancer ovarien, cancer pancréatique endocrinien, glucagonome, cancer pancréatique, cancer parathyroïdien, cancer du pénis, cancer de l'hypophyse, sarcome des tissus mous, rétinoblastome, cancer de l'intestin grêle, cancer de l'estomac, cancer du thymus, cancer de la thyroïde, cancer trophoblastique, môle hydatiforme, cancer utérin, cancer endométrial, cancer du vagin, cancer de la vulve, névrome acoustique, mycosis fungoïde, insulinome, syndrome carcinoïde, somatostatinome, cancer des gencives, cancer du coeur, cancer de la lèvre, cancer des méninges, cancer de la bouche, cancer des nerfs, cancer du palais, cancer des glandes parotides, cancer du péritoine, cancer du pharynx, cancer de la plèvre, cancer des glandes salivaires, cancer de la langue et cancer des amygdales.

14. Utilisation selon la revendication 12 ou 13 ou vecteur adénoviral pour une utilisation selon la revendication 11 ou 13, dans lequel le sujet est un être humain ou un animal.

15. Utilisation selon l'une quelconque des revendications 12 à 14 ou vecteur adénoviral pour une utilisation selon l'une quelconque des revendications 11, 13 ou 14, dans lequel le vecteur adénoviral ou la composition pharmaceutique est administré par une injection intratumorale, intramusculaire, intra-artérielle, intraveineuse, intrapleurale, intravésiculaire, intracavitaire ou péritonéale ou une administration orale, de préférence plusieurs fois durant la période de traitement.

16. Utilisation selon l'une quelconque des revendications 12 à 15 ou vecteur adénoviral pour une utilisation selon l'une quelconque des revendications 11 ou 13 à 15, dans lequel le vecteur adénoviral ou la composition pharmaceutique est administré simultanément à une radiothérapie ou du radio-iodure à un sujet, ou une chimiothérapie simultanée à un sujet.

17. Utilisation selon l'une quelconque des revendications 12 à 16 ou vecteur adénoviral pour une utilisation selon l'une quelconque des revendications 11 ou 13 à 16, dans lequel le sujet à traiter présente de grandes quantités d'anticorps neutralisants anti-Ad5 et/ou a été traité auparavant avec un Ad5.

18. Procédé de production d'un vecteur adénoviral selon l'une quelconque des revendications 1 à 7 comprenant :
i) la fourniture d'un vecteur d'ADN comprenant au moins de l'ADN partiel d'Ad3 et un ou plusieurs promoteurs à spécificité tumorale ou tissulaire et/ou éventuellement un ou plusieurs transgènes, et
ii) l'insertion du reste du génome de l'Ad3 et éventuellement un ou plusieurs promoteurs à spécificité tumorale ou tissulaire et/ou éventuellement un ou plusieurs transgènes dans le vecteur ;
moyennant quoi il est produit un vecteur adénoviral humain oncolytique comprenant un squelette totalement de sérotype 3 et un promoteur hTERT à spécificité tumorale contrôlant la région E1A d'Ad3.
